(19) Europäisches Patentamt
European Patent Office
Office européen des brevets

(11) Veröffentlichungsnummer: **0 003 833**
**B2**

(12) # NEUE EUROPÄISCHE PATENTSCHRIFT

(45) Veröffentlichungstag der neuen Patentschrift: **19.12.90**

(51) Int. Cl.[5]: **C 07 K 9/00,** A 61 K 39/00, A 61 K 39/39

(21) Anmeldenummer: **79100513.5**

(22) Anmeldetag: **21.02.79**

(54) **Antigenderivate, Verfahren zu deren Herstellung, diese enthaltende pharmazeutische Präparate.**

(30) Priorität: **24.02.78 CH 2035/78**
**07.04.78 CH 3777/78**
**18.05.78 CH 5394/78**

(43) Veröffentlichungstag der Anmeldung: **05.09.79 Patentblatt 79/18**

(45) Bekanntmachung des Hinweises auf die Patenterteilung: **20.07.83 Patenblatt 83/29**

(45) Bekanntmachung des Hinweises auf die Entscheidung über den Einspruch: **19.12.90 Patentblatt 90/51**

(84) Benannte Vertragsstaaten: **BE CH DE FR GB IT LU NL SE**

(56) Entgegenhaltungen:
**DE-A-1 915 970**
**DE-A-2 450 355**
**DE-A-2 655 500**
**FR-A-2 288 527**

(73) Patentinhaber: **CIBA-GEIGY AG**
**Klybeckstrasse 141**
**CH-4002 Basel (CH)**

(72) Erfinder: **Baschang, Gerhard, Dr.**
**Bückenweg 7**
**CH-4126 Bettingen (CH)**
Erfinder: **Dietrich, Felix M., Prof. Dr.**
**Marignanostrasse 64**
**CH-4059 Basel (CH)**
Erfinder: **Gisler, Roland, Dr.**
**Ob d. Hügliacker 15**
**CH-4102 Binningen (CH)**
Erfinder: **Hartmann, Albert, Dr.**
**Steingasse 21A**
**D-7889 Grenzach (DE)**
Erfinder: **Stanek, Jaroslav, Dr.**
**Florastrasse 6**
**CH-4127 Birsfelden (CH)**
Erfinder: **Tarcsay, Lajos, Dr.**
**Muttenzerstrasse 25**
**D-7889 Grenzach-Wyhlen (DE)**

(74) Vertreter: **Zumstein, Fritz, Dr. et al**
**Bräuhausstrasse 4**
**D-8000 München 2 (DE)**

EP 0 003 833 B2

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
Israel J. Med. Sci., 1977, 1054-1055
Z. Immunforschung 149 (1975), 349-353
Immunology 35 (1978), 573-579
Journ. of Immunology 116 (1976), 1635-1639
Proc. Nat. Acad. Sci., USA 73 (1976), 2472-2475
Cellular Immunology 21 (1976), 243-249
Colloques INSERM 72 (1977), 249-262
C.R. Acad. Sci. Paris 285 (1977), Série D, 467-470
Journ. of Immunology 119 (1977), 2073-2077
Proc. Nat. Acad. Sci., USA 72 (1975), 4105-4109
Amer. Soc. Microbiol. 14 (1976), 18-27
Physiological Effects of Immunity against Reproductive Hormones (Cambridge 1976), 249-277
Lehrbuch der pharmazeutischen Technologie (Berlin, 1975), S. 721-726
Z. Immunforschung 149 (1975), 341-348
Lexikon der Biochemie (Weinheim 1976), 373

**Beschreibung**

Die Erfindung betrifft neue Antigenderivate, ferner Verfahren zu deren Herstellung, sowie pharmazeutische Präparate, die diese neuen Antigenderivate enthalten, wie auch deren Verwendung als Impfstoff.

Die Erfindung betrifft insbesondere als Antigen wirksame Verbindungen der Formel III

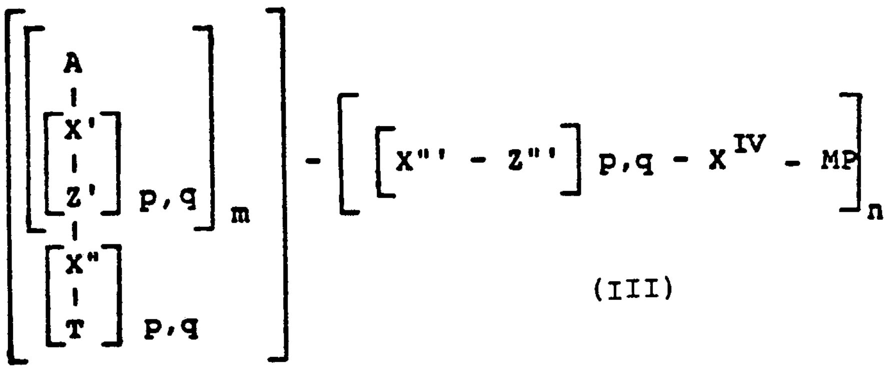

(III)

worin A den Rest eines Antigens, T den Rest eines Trägers, MP den Rest eines Muramylpeptids der Formel II,

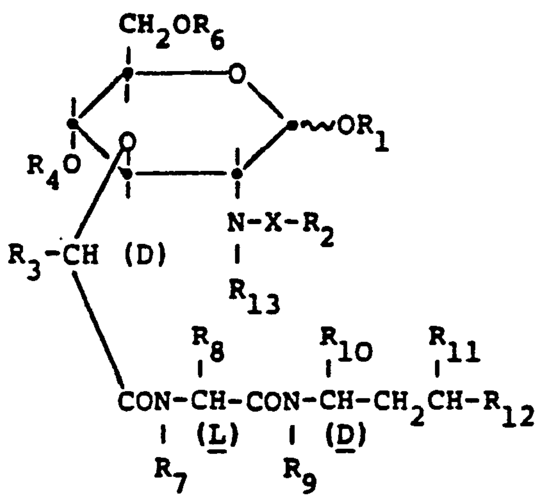

(II)

worin X eine Carbonyl-, Carbonyloxy- oder Sulfonylgruppe, $R_1$ Wasserstoff, Alkyl, gegebenenfalls substituiertes Benzyl oder Acyl, $R_2$ gegebenenfalls substituiertes Alkyl oder carbocyclisches Aryl, $R_4$ und $R_6$ unabhängig voneinander Wasserstoff, Alkyl, gegebenenfalls substituiertes Benzyl oder Acyl, $R_3$ Wasserstoff oder Alkyl, $R_7$ und $R_{13}$ Wasserstoff oder Niederalkyl, $R_8$ Wasserstoff, Niederalkyl, freies, verestertes oder veräthertes Hydroxyniederalkyl, freies, verestertes oder veräthertes Mercapto-niederalkyl, freies oder acyliertes Aminoniederalkyl, Cycloalkyl mit 4 oder 6 Kohlenstoffatomen, Cycloalkyl-niederalkyl, dessen Cycloalkylrest 5 oder 6 Kohlenstoffatome enthält, gegebenenfalls substituiertes Aryl oder Aralkyl, stickstoffhaltiges Heterocyclyl- oder Heterocyclyl-niederalkyl, $R_7$ und $R_8$ zusammen auch Alkylen mit 3 oder 4 Kohlenstoffatomen, $R_9$ Wasserstoff oder Niederalkyl, die Reste $R_{10}$, $R_{11}$ und $R_{12}$ unabhängig voneinander einen gegebenenfalls veresterten oder amidierten Carboxyrest und $R_{11}$ auch Waserstoff bedeuten, oder $R_1$, $R_4$ und $R_6$ Triniederalkylsilyl, insbesondere Trimethylsilyl, sind, wobei das Präfix "Nieder" stets einen Rest bis und mit 7 C-Atomen kennzeichnet, Z' und Z''' bivalente Brückenglieder, X', X'', X''' und $X^{IV}$ unabhängig voneinander die kovalenten Verknüpfungselemente Carbonyloxy

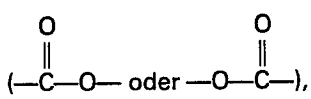

Carbonylimino 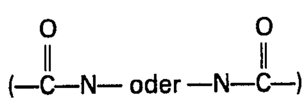 oder Carbonylthio

$$(-\overset{O}{\overset{\|}{C}}-S- \text{ oder } -S-\overset{O}{\overset{\|}{C}}-),$$

p = 0, q = 1 und m und n ganze Zahlen grösser als 0 bedeuten, mit der Massgabe, dass in den Verbindungen der Formel III mindestens eine in der Peptidchemie übliche Verknüpfung vorhanden ist, die synthetisch hergestellt worden sein muss und dass die als Ausgangsstoffe verwendeten Komponenten Muramylpeptid, Träger und Antigen die zur Verknüpfung miteinander oder mit dem Brückenglied geeigneten funktionellen Gruppen aufweisen.

Zum Zeitpunkt der Erfindung war es aus de Deutschen Offenlegungsschrift 1 915 970, S. 8, bekannt, dass man Membrane bakteriellen Ursprungs kovalent mit Albuminmolekülen als *Träger* kuppeln kann. Die erhaltenen Verbindungen bewirken die Bildung antibakterieller Antikörper. Der Erfindungsgedanke der vorliegenden Erfindung unterscheidet sich von dem diesem Phänomen zugrundeliegenden Prinzip grundlegend.

H. Langbeheim et al. haben in Israel J. Med. Sci. *13*, 1054—1055 (1977) in einem kurzen Artikel mit dem Titel "Adjuvant effect of peptidoglycan in the immune response to a synthetic fragment of the MS-2 bacteriophage" andeutungsweise ein Peptidoglykan-Antigen-Träger-Konjugat erwähnt, womit anscheinend bei Verabreichung *in Oel* ein höherer Adjuvanzeffekt im Hinblick auf die humorale Immunantwort erzielt wurde als mit dem Gemisch, bestehend aus dem Peptidoglykan und dem Antigen-Träger-Konjugat. Das obengenannte Peptidoglykan ist ein im dreidimensionalen Raum polymerisiertes Makromolekül, das einem Muramylpeptid weder strukturell noch wirkungsmässig gleicht.

Die obengenanten Literaturstellen legen die Lösung der erfindungsgemässen Aufgabe nicht nahe. Der vorliegenden Erfindung liegt die Aufgabe zugrunde, neue Verbindungen bereitzustellen, welche in Abwesenheit von Mineralöl, d.h. unter klinisch akzeptablen Verabreichungsbedingungen, in vivo und in sowohl wirkungsdauermässig als auch quantitativ verbessertem Ausmass eine zellvermittelte Immunität zu induzieren vermögen, wobei die genannte Induktion der zellvermittelten Immunität sich auch gegen lösliche Antigene erstrecken soll.

Die erfindungsgemässe Aufgabe wird durch die obegenannten Verbindungen der Formel III gelöst.

Dabei wird unter Antigen ein organischer Stoff verstanden, der vom physiologischen Medium, d.h. dem menschlichen oder tierischen Organismus, als immunlogisch fremd erkannt wird oder unter geeigneten Voraussetzungen als fremd erkannt werden kann.

zu den Antigenen gehören in erster Linie sämtliche, die spezifische Immunisierung eines lebenden Organismus gegen infektöse Krankheitserreger oder unerwünschte Reaktionen, wie allergische Sensibilisierung oder Abstossung von transplantiertem Fremdgewebe, verursachenden Stoffe. Insbesondere sind darin Antigene als Inhaltsstoffe von Vakzinen einbezogen.

Als Vakzine kommen einerseits solche in Betracht, die im Rahmen klassischer Impfverfahren zum spezifischen immunologischen Schutz gegen Infektionskrankheiten eingesetzt werden können. Als Antigene, die in solchen Vakzinen enthalten sind, kommen die abgeschwächten lebenden oder abgetöteten, modifizierten oder abgebauten Erreger der Infektionskrankheiten, die von diesen Erregern gebildeten Toxoide oder natürliche bzw. synthetisch hergestellte Teilkomponenten von Erregern und Toxoiden in Frag. Als Klassen von Erregern sind namentlich zu nennen: Viren, Chlamydien, Rickettsien, Bakterien, Protozoen und metazoische Parasiten. Bevorzugte Antigene sind solche, die als Bestandteile von Vakzinen gegeben zur Behandlung von z.B. Influenza A und B, Parainfluenza 1—3, durch respiratorisches Syncytial-Virus, Rhinoviren oder Adenoviren hervorgerufene respiratiorische Erkranungen, Cytomegalie, Röteln, Masern, Mumps, Pertussis, Poliomyelitis, Herpes simplex 1 und 2, Varizellen und Herpes zoster, Rotavirus-Erkranungen, Hepatitis A, B und andere Tollwut, Maul- und Klauenseuche, Trachom, Karies, durch Meningokokken A, B und C verursachte Meningitiden, Erkrankungen durch Pneumokokken, H. influenzae, Streptokokken (insbesondere rheumatisches Fieber), Pseudomonas und Proteus, Typhus, Paratyphus und andere durch Enterobateriaceen hervorgerufene Durchfallkrankheiten, Gonorrhoe, Syphillis, Malaria, Trypanosomiasen (Schlafkrankheiten und Chagas-Krankheit), Leishmaniosen, Filariosen, Schistosomiasen, Ankylostomiasen und andere Wurmerkrankungen, geeignet sind.

Andererseits sind neuartige Vakzine zu nennen, die nicht gegen Erreger von Infektionskrankheiten, sondern entweder gegen körpereigene Bestandteile, d.h. normale und aberrante Autoantigene, oder gegen sensibilisierende Umwelstantigene, d.h. Allergene gerichtet sind.

In einem Fall wird angestrebt, durch Immunisierung gegen normale oder abartige Autoantigene die funktion körpereigener Moleküle (z.B. von Hormonen, von anderen Mediatoren und von humoralen und zellständigen Rezeptoren) auszuschalten bzw. die Ausbreitung oder Persistenz abartiger, namentlich neoplastischer Zell-Linien aufzuheben. Bevorzugte Antigene als Bestandteile solcher Vakzine sind z.B.: Teilsequenzen des humanen Choriongonadotropins oder Bestandteile von Spermatozoen zur Immunisierung gegen Mediatoren der Fertilität und damit zur immunologischen Ausschaltung von Reproduktionsfunktionen; Mediatoren entzündlicher Prozesse, insbesondere in gereinigter Form, darunter die von Lymphozyten abgesonderten Lymphokine, insbesondere MIF (macrophage migration inhibitory factory) zur immunologischen Unterdrückung von Entzündungskrankheiten; immunologisch spezifische Antigenrezeptoren von Lymphozyten und von Antikörpern (fraktionierte antigenspezifische Lymphozyten, von diesen Lymphozyten extrahierte oder sezernierte Antigenrezeptoren, fraktionierte antigenspezifische

Antikörper bzw. Antikörperfragmente) zur Antiidiotypenimmunisierung (Immunisierung gegen die für die Antigenrezeptor-Strukturen charakteristischen Autoantigene) mit em Ziel der Ausschaltung spezifischer Immunreaktionen zur Elimination krankmachender Immunprozesse wie Autoimmunität (z.B. gegen Synovialantigene und Immunglobuline bei der primär chronischen Polyarthritis, gegen Myelinkomponenten bei degenerativen Erkrankungen des Zentralnervensystems, gegen TSH-Rezeptoren bei autoimmuner Thyreoiditis, gegen Acetylcholin-Rezeptoren der quergestreiften Muskulatur bei der Myasthenia gravis, gegen Inselzellkomponenten beim juvenilen Diabetes etc.) oder Allergie (z.B. gegen Gräserpollen, Stäube oder Medikamente bei allergischem Asthma, allergischer Rhinitis bzw. Arzneimittelüberempfindlichkeit) oder zur Vermeidung an sich normaler, aber unerwünschter Immunreaktionen (z.B. Induktion von Immuntoleranz zur Verhinderung der Abstossung transplantierter fremder Organe und Gewebe); autologe oder mit ihnen kreuzreagierende homologe bzw. heterologe Tumorzellen, Tumorzellfragmente oder -membrankomponenten einschliesslich Onkornavirus-kodierter Glykoproteine, wie GP 70, zur tumorspezifischen Immunisierung im Rahmen der Prophylaxe und Therapie von Krebskrankheiten.

Im anderen Fall wird versucht, durch Immunisierung gegen Allergene an Stelle der pathologisch relevanten IgE-Antikörperantwort vorwiegend bzw. in ausreichendem Masse IgG- und IgA-Antikörper gegen die sensibilisierenden Umweltantigene zu indusizeren und dadurch in der Zirkulation uind in den Sekreten Allergene durch spezifische Antikörper abzufangen, bevor sie mit den an Mastzellen gebundenen IgE-Antikörper reagieren und damit die Freisetzung allergischer Mediatoren auslöen können. Bei dieser antigenspezifischen Desensibilisierung sind die Allergene selbst, d.h. z.B. Gräserpollen, Stäube oder Medikamente, Antigenbestandteile der Vakzine.

Antigene können, insbesondere wenn sie niedermolekular sind, durchaus auch und mit Vorteil, an einen hochmolekularen Träger kovalent gebunden sein. Als Träger seien insbesondere Polymere der Milchsäure und deren Derivate, die am Kettenende eine freie Carboxylgruppe tragen, wie ihre Ester mit Glykolsäuren, Polymilschsäureamide oder Milchsäurepolyesteramide wie sie z.B. in der Hydroxy- oder Mercaptogruppen, z.B. Niederalkoxy oder Niederalkylmercaptogruppen, oder Halogenatome oder freie oderfunktionell abgewandelte Carboxyl-, wie Carbo-niederalkoxy- oder Carbamoylgruppen in Frage. Dabei kann der substituierte Alkylrest, wie Niederalkylrest einen, zwei oder mehrere gleiche oder verschiedene Substituenten, insbesondere freie Hydroxygruppen oder Halogenatome tragen.

Carbocyclische Arylreste sind insbesondere monocyclische, sowie bicyclische Arylreste, in erster Linie Phenyl, aber auch Naphthyl. Sie können gegebenenfalls, z.B. durch Niederalkylgruppen, freie, verätherte oder verestertes Hydroxy, z.B. Niederalkoxy oder Niederalkylendioxy oder Halogenatome, und/oder Trifluoromethylgruppen, mono-, di- oder polysubstituiert sein.

Aralkyl ist insbesondere Aryl-niederalkyl, worin Aryl die oben gegebene Bedeutung hat. In erster Linie steht Arylniederalkyl für Benzyl oder Phenyläthyl, worin der Phenylkern mono-, di- oder polysubstituiert sein kann.

Gegebenenfalls substituierte Benzylreste sind insbesondere solche Benzylreste, die im aromatischen Kern gegebenenfalls, z.B. durch Niederalkyl, freie, verätherte oder veresterte Hydroxy- oder Mercaptogruppen, z.b. Niederalkoxy oder Niederalkylendioxy, sowie Niederalkylmercapto- oder Trifluormethylgruppen und/oder Halogenatome, mono-, di- oder polysubstituiert sind.

Stickstoffhaltiges Heterocyclyl ist insbesondere der Rest einer 5- oder 6-gliedrigen, ein oder 2-Stickstoffatome im Ring enthaltenden heterocyclischen Verbindung. Er kann ungesättigt oder auch gesättigt sein, und z.B. einen ankondensierten Phenylrest enthalten. Als solche seien z.B. der Pyrrol-, Indan-, Pyridyl- oder Imidazolring genannt.

Eine gegebenenfalls veresterte oder amidierte Carboxylgruppe ist in erster Linie die Carboxylgruppe selbst, oder eine mit einem Niederalkanol veresterte Carboxylgruppe oder auch die Carbamoylgruppe, die am Stickstoffatom unsubstituiert ist oder mit Alkyl, insbesondere Niederalkyl, Aryl, in erster Linie Phenyl, oder Aralkyl, wie Benzyl, mono- oder di-substituiert ist. Die Carbamoylgruppe kann aber auch einen Alkylen-, wie den Tetra- oder Pentamethylenrest tragen. Die Carbamoylgruppe $R_{10}$ kann am Stickstoff auch durch die Carbamoyl-methylgruppe substituiert sein.

Acyl ist insbesondere ein Acylrest einer organischen Säure, insbesondere einer organischen Carbonsäure. So ist Acyl insbesondere Alkanoyl, vor allem mit 2—18 Kohlenstoffatomen, in erster Linie jedoch Niederalkanoyl, oder auch Aroyl, wie Naphthoyl-1, Naphthoyl-2 und insbesondere Benzoyl oder durch Halogen, Niederalkyl, Niederalkoxy, Trifluormethyl, Hydroxy oder Niederalkanoyloxy substituiertes Benzoyl oder Naphthoyl, oder auch ein Acylrest einer organischen Sulfonsäure, z.B. einer Alkansulfonsäure, insbesondere einer Niederalkansulfonsäure, oder einer Arylsulfonsäure, insbesondere einer gegebenenfalls Niederalkyl- oder Halogen-substituierten Phenylsulfonsäure, wie der Benzolsulfonsäure oder p-Toluolsulfonsäure, sowie Carbamoyl, z.B. unsubstituiertes Carbamoyl, Niederalkylcarbamoyl oder Arylcarbamoyl, wie Methyl- oder Phenyl-carbamoyl.

Verestertes oder veräthertes Hydroxy ist insbesondere Niederalkoxy oder Niederacyloxy, wie Niederalkanoyloxy.

Verestertes oder veräthertes Mercapto ist insbesondere Niederalkylmercapto- oder Niederacyl-, wie Niederalkanoylmercapto.

Acyliertes Amino ist insbesondere Niederalkanoylamino oder Carbamoylamino.

Die im Zusammenhang mit der vorliegenden Beschreibung und den Patentansprüchen mit "Nieder"

bezeichneten Reste und Verbindungen enthalten bis und mit 7 und in erster Linie bis und mit 4 Kohlenstoffatome.

Vorstehend, wie nachfolgend können die Allgemeinbegriffe folgende Bedeutung haben:

Niederalkyl ist z.B. n-Prpyl, n-Butyl, Isobutyl, sek.-Butyl oder tert.-Butyl, ferner n-Pentyl, n-Hexyl, Isohexyl oder n-Heptyl und in erster Linie Methyl oder Äthyl. In Aryl-, Cycloalkyl- oder Heterocyclylniederalkyl ist der Niederalkylrest insbesondere Methyl oder Äthyl, wobei der Aryl-, Cycloalkyl- oder Heterocyclyl-rest die obgenannte Bedeutung besitzt.

Niederalkoxy ist z.B. n-Propoxy, n-Butoxy, Isobutoxy, sek.-Butoxy oder tert.-Butoxy und in erster Linie Methoxy oder Äthoxy.

Niederalkylmercapto ist z.B. n-Propyl, n-Butyl-Isobutyl, sek.-Butyl oder tert.-Butylmercapto und in erster Linie Methylmercatpo oder Äthylmercapto.

Niederalkylendioxy ist insbesondere Methylendioxy, Äthylen- oder Propylendioxy.

Halogen steht für Fluor oder Brom, vorzugsweise jedoch für Chlor.

Niederalkanoyl ist insbesondere Propionyl oder Butyryl, in erster Linie jedoch Acetyl.

Die neuen Verbindungen der vorliegenden Erfindung können in Form von Gemischen von Isomeren oder von reinen Isomeren vorliegen.

Es ist bekannt, dass Muramylpeptide gute Adjuvantien sind, die in geeigneter Mischung mit Antigenen deren Immunogenität zu steigern vermögen. Es hat sich allerdings gezeigt, dass sie nur englische Patentschrift Nr. 932,382 beschrieben sind, ferner Alginsäure, Polygalakturonsäure, Pektin, Carboxymethylcellulose oder Agarose genannt. Weiterhin kommen als Träger in Frage basische, neutrale oder saure Polyaminosäuren, die nicht selbst immunogen sind, wie Polyasparaginsäure, Polyglutaminsäure, Polylysin oder Polyornithin. Als Träger kommen im übrigen auch beliebige andere Antigene im Sinne der vorstehend gegebenen Definition bzw. aufgeführten Beispiele in Betracht, insofern als eine Immunreaktion gegen diese in Kauf genommen werden kan oder sogar erwünscht ist. So kann z.B. ein HCG-Peptid an Tetanustoxid, als Träger, kovalent gebunden sein. Die obengenannten Muramylpeptide der allgemeinen Formel II sind synthetisch herstellbar.

Die verschiedenen Teile der neuen Verbindungen sind kovalent miteinander verbunden, d.h. das Antigen ist mit mindestens einer seiner funktionellen Gruppen über eine in der Peptidchemie übliche Verknüpung mit dem Rest des Muramyl-peptids, direkt oder über ein Brückenglied (Spacer) verbunden.

Als Brückenglieder (Spacer) dienen insbesondere bivalente Reste von aliphatischen Verbindungen, z.B. von solchen, die mindestens zwei Aminogruppen, mindestens eine Aminogruppe und eine Carboxy- oder Thiocarboxygruppe, oder mindestens zwei Carboxy- oder Thiocarboxygruppen besitzen, wie aliphatische Diamine, Amino-thiocarbonsäuren, neutrale, basische oder saure aliphatische Aminosäuren, Di- oder Oligopeptide.

Als Brückenglieder seien in erster Linie genannt α,Ω-Diamino-alkane, insbesondere α,Ω-Diamino-niederalkane, wie Aethylendiamin, Propylendiamin, Tetramethylendiamin, Alkyl-dicarbonsäuren, wie Bernsteinsäure oder Glutarsäure, α,β oder γ-Aminoalkancarbonsäuren, vorzugsweise α-Amino-niederalkancarbonsäuren, insbesondere die natürlichen α-Amino-niederalkancarbonsäuren, wie Glycin, β-Alanin, L-Alanin, α-Amino-isobuttersäure, Valin oder Leucin.

Alkyl ist vorzugsweise geradkettiges oder verzweigtes, in beliebiger Stellung gebundenes Alkyl mit bis zu 18 Kohlenstoffatomen, in erster Linie jedoch Niederalkyl.

Als Substituenten der gegebenenfalls substituierten Alkylgruppe kommen in erster Linie freie oder funktionell abgewandelte Hydroxy- oder Mercaptogruppen, wie verätherte oder veresterte eine kurzfristige Wirksamkeit entfalten, da sie relativ rasch aus dem menschlichen oder tierischen Organismus ausgeschieden werde. Vor allem sind sie nur unter bestimmten, für klinische Zwekke nicht geeigneten Bedingungen, nämlich in Mischung mit Antigenen in einer Emulsion mit Mineralöl in der Lage, in vivo eine zellvermittelte Immunität gegen lösliche Antigene zu induzieren. Die neuen Verbindungen der vorliegenden Erfindung bringen nun eine ausgeprägte Steigerung der Immunantwort auf das Antigen, insbesondere auch eine zellvermittelte Immunität unter klinisch akzeptablen Verabreichungsbedingungen, wie dies an Hand der nachstehend beschriebenen Versuchsanordnungen gezeigt werden kann.

1. Potenzierung der zellvermittelten Immunität in vivo: Steigerung der Spättyp-Überempfindlichkeit gegen bovines Serumalbumin (BSA) und gegen Schaferythrocyten (SRBC) beim Meerschweinschen.

Pirbright Meerschweinchen werden am Tag 0 mit 1 BSA oder mit 1 mg SRBC-'Ghosts' (SRBCG) in komplettem Freund'schem Adjuvans durch Injektion von je 0,1 ml eines Antigen-Adjuvans-Gemisches in die beiden Hinterpfoten immunisiert. 3 Wochen später werden Hautreaktionen durch intrakutane Injektion von 100 µg BVSA bzw. 100 µg SRBCG in 0,1 ml gepufferter physiologischer Salzlösung ausgelöst und aufgrund des 24 Stunden danach anhand der Erythemfläche und der Hautdickenzunahme berechtenen Reaktionsvolumens quantifiziert. Die nach 24 Stunden (Spättypreaktion) beobachtete antigenspezifische Zunahme des Reaktionsvolumens gilt als ein Mass für zellvermittelte Immunität. BSA und SRBCG sind zu schwache Immunogene, um für sic allein oder in einer Wasser-Öl-Emulsion mit inkomplettem Freund'scheum Adjuvans (10 Teile BSDA-Lösung resp. SRBCG Suspension in 0,9% NaCl gemischt mit 8,5 Teilen Bayol F (Warenzeichen, Paraffinöl, Kohlenwasserstoff-Fraktion des Erdöls) und 1,5 Teilen Arlacel A (Mannitan-Monooleat) eine Spättypreaktion zu induzieren, sondern müssen für eine effektive Immunisierung in komplettem Akjuvans, dem Mycobakterien zugesetzt (5 mg abgetöte und lyophilisierte M. butyricum pro 10 ml Bayol F®/Arlacel A®) sind, appliziert werden.

An Stelle der Mycobakterien wurden die neuen Verbindungen enthaltend als Antigen BSA (1 mg BSA, 60 µg MDP pro Tier) oder als Antigen SRBCG (1 mg SRBCG, 25 µg MDP pro Tier) entweder als Antigen-Öl-Gemisch oder in Carboxymethylcellulose (CMC) suspendiert appliziert. Die neuen Verbindungen induzieren, in Abwesenheit von Mycobakterien in der beschriebenen Versuchsanordnung Spättypreaktionen.

Eine signifikante Potenzierung der Spättypreaktivität gegen BSA und gegen SRBCG kann auch dadurch erreicht werden, dass die neuen Verbindungen nicht in inkomplettes Freund'sches Adjuvans inkorporiert, sondern in CMC suspendiert appliziert werden. Als besonders wirksam erwies sich in diesem Falle die intramuskuläre Applikation. Unter diesen Umständen war eine gleiche Menge von freiem Muramylpeptid, die dem CMC-Gemisch beigemengt wurde, wesentlich weniger aktiv als die neue Wirksubstanz. Damit wird gezeight, dass die neuen Verbindungen unter klinisch akzeptablen Verabreichungsbedingungen, d.h. bei Applikation mit körperfreundlichen Begleitstoffen, zellvermittelte Immunität sogar gegenüber einem löslichen Proteinantigen zu induzieren vermögen.

2. Potenzierung der zellvermittelten Immunität in vivo: Steigerung der Spättyp-Überempfindlichkeit gegen BSA und gegen SRBCG bei der Maus.

Männliche MAG-Mäuse werden am Tag 0 mit abgestuften Dosen von nicht mit Muramylpeptid konjugierten Antigenen (BSA-Agarose oder SRBCG) oder von mit Muramylpeptid konjugierten Antigenen (BSA-Agarose oder SRBCG) immunisiert. Die BSA-Agarose-Präparate werden in einer Dosierung von 0,1 bis 100 µg (entspricht bei den neuen Verbindungen mit Muramylpeptiden einer Wirksubstanzdosis von 0,0029—6 µg pro Tier) in einem Volumen von 0,2 ml gepufferter physiologischer Kochsalzlösung subcutan appliziert. Die SRBCG-Präparate werden in einer Dosierung von 0,01—3 mg (entspricht bei den neuen Verbindungen mit Muramylpeptiden einer Wirksubstanzdosis von 0,25—75 µg pro Tier) in einem Volumen von 0,5 ml in gepufferter physiologischer Salzlösung intraperitoneal oder 0,05 ml intradermal resp. in 3 Fusspfoten verteilt appliziert.

4 bis 20 Tage später werden Spättypreaktionen durch Injektion von 100 µg BSA resp. $10^8$ SRBC in 20 µl gepufferter physiologischer Salzlösung in die linke hintere Fusspfote ausgelöst und aufgrund des 24 und 48 Stunden danach anhand der Fusspfotenschwellung ermittelten Reaktionsvolumens quantifiziert. Die beobachtete antigenspezifische Zunahme des Fusspfotenvolumens gilt als Mass für zellvermittelte Immunität.

Ab Tag 14 nach Immunisierung mit BSA-Agarose-MDP-Verbindungen treten auch schon bei sehr niedriger Dosierung (0,1 µg; entspricht 0,006 µg Wirksubstanz) ausgeprägte Spättypreaktionen auf. Im Gegensatz dazu ist freie BSA-Agarose nicht in der Lage, für Spättyp-Reaktionen zu sensibilisieren. Ebenso sind SRBGCG-MDP-Verbindungen — vor allem nach intradermaler Applikation — fähig, Spättypreaktivität zu induzieren, die signifikant ausgeprägter ist, als diejenige, die man nach Sensibilisierung mit SRBCG erhalten kann, das nicht mit Muramylpeptid verbunden ist.

Damit wird ebenfalls gezeigt, dass die neuen Verbindungen zelluläre Immunität erheblich zu steigern vermögen.

3. Potenzierung der humoralen Immunität in vivo: Steigerung der Antikörperproduktion gegen BSA bei der Maus.

NMRI Mäuse werden durch intraperitoneale Injektion von 0,1 bis 100 µg BSA verbunden mit Muramylpeptiden (0,0014 bis 6,0 µg Wirksubstanz) am Tag 0 immunisiert. 10, 17 und 28 Tage später werden Serumproben entnommen und auf ihren Gehalt an anti-BSA Antikörpern mit einer passiven Haemagglutinationstechnik untersucht. In der verwendeten Dosis ist freies BSA für die Empfängertiere subimmunogen, d.h. es vermag keine oder nur eine ganz geringfügige Produktion von Antikörpern auszulösen. Die Verbindung von BSA mit einem Muramylpeptid ermöglich einen 2—3-fachen Anstieg der Antikörpertiter im Serum (Titer-Summe der $\log_2$ Titerdifferenzen an drei Blutungstagen). Bemerkenswert ist ausserdem, dass die neuen Verbindungen, die zudem noch an Agarose als Träger gekuppelt wurden, nach intraperitonealer oder subcutaner Verabreichung noch stärker immunogen sind.

Anhand der geschilderten Versuchen wird gezeigt, dass die erfindungsgemässen neuen Verbindungen auch die humorale Immunität erheblich zu steigern vermögen.

Die neuen Antigenderivate sind neuartige oder verbesserte bekannte Impfstoffe und dienen zu neuartigen Impfverfahren oder zur Vereinfachung gebräuchlicher Impfverfahren (indem z.B. die Zahl der Impfungen, die zur Aufrechterhaltung des Schutzes über längere Zeitspannen notwendig ist, gesenkt werden kann).

Die Erfindung betrifft insbesondere Antigenderivate, die den Rest eines Muramylpeptids der Formel II kovalent gebunden enthalten, worin $R_1$, $R_3$, $R_4$, $R_6$ und $R_6$ und $R_7$ Wasserstoff, X Carbonyl und $R_2$ gegebenenfalls durch Hydroxy oder Niederalkoxy substituiertes Niederalkyl oder gegebenenfalls durch Hydroxy, Niederalkoxy, Niederalkyl oder Halogen substituiertes Phenyl bedeuten, und $R_8$, $R_9$, $R_{10}$, $R_{11}$, $R_{12}$ und $R_{13}$ die oben angegebene Bedeutung besitzen.

Die Erfindung betrifft insbesondere auch Antigenderivate, die den Rest eines Muramylpeptids der formel II kovalent gebunden enthalten, worin $R_1$, $R_4$, $R_6$ und $R_7$ Wasserstoff, X Carbonyl, $R_2$ gegebenenfalls durch Hydroxy oder Niederalkoxy substituiertes Niederalkyl oder gegebenenfalls durch Hydroxy, Niederalkoxy, Niederalkyl oder Halogen substituiertes Phenyl und $R_3$ Methyl bedeuten und $R_8$, $R_9$, $R_{10}$, $R_{11}$,

$R_{12}$ und $R_{13}$ die oben angegebene Bedeutung besitzen.

Die Erfindung betrifft in erster Linie Antigenderivate, die den Rest eines Muramylpeptids der Formel II kovalent gebunden enthalten, worin $R_1$, $R_4$, $R_6$, $R_7$, $R_9$ und $R_{13}$ Wasserstoff, X Carbonyl, $R_2$ gegebenenfalls durch Hydroxy oder Methoxy substituiertes Niederalkyl oder gegebenenfalls durch Hydoxy, Methoxy, Methyl, Äthyl oder Halogen substituiertes Phenyl, $R_3$ Wasserstoff oder Methyl, $R_8$ Niederalkyl, Niederalkylmercapto-niederalkyl, Hydroxyniederalkyl, Benzyl, p-Hydroxybenzyl oder Phenyl und $R_{10}$, $R_{11}$ und $R_{12}$ Carboxyl, Niederalkoxycarbonyl, oder Carbamoyl und $R_{12}$ auch Wasserstoff bedeuten.

Die Erfindung betrifft vor allem Antigenderivate, die den Rest eines Muramylpeptides der Formel II kovalent gebunden enthalten, worin $R_1$, $R_4$, $R_6$ und $R_{13}$ Wasserstoff, X Carbonyl, $R_2$ gegebenenfalls durch Hydroxy oder Methoxy substituiertes Niederalkyl oder gegebenenfalls durch Hydroxy, Methoxy, Methyl, Äthyl oder Halogen substituiertes Phenyl, $R_3$ und $R_9$ Wasserstoff oder Methyl, $R_8$ Methyl, Äthyl, n-Propyl, i-Propyl, 2-Methylpropyl, Methylmercaptomethyl, Hydroxymethyl, Hydroxyäthyl, Phenyl, Benzyl oder p-Hydroxybenzyl und $R_{10}$, $R_{11}$ und $R_{12}$ Carboxy, Niederalkoxycarbonyl oder Carbamoyl und $R_{11}$ auch Wasserstoff bedeuten.

Die Erfindung betrifft auch Antigenderivate, die den Rest eines Muramylpeptids der Formel II kovalent gebunden enthalten, worin $R_1$, $R_4$, $R_6$ und $R_{13}$ Wasserstoff, X Carbonyl, $R_7$ und $R_8$ zusammen Propylen oder Butylen bedeuten und $R_2$, $R_3$, $R_9$, $R_{10}$, $R_{11}$ und $R_{12}$ die oben genannte Bedeutung besitzen.

Brückenglieder in den obigen Definitionen sind insbesondere α,Ω-Diamino-niederalkane, Niederalkyldicarbonsäuren und natürliche α-Amino-niederalkancarbonsäuren.

Insbesondere betrifft die Erfindung die neuen, in den Beispielen beschriebenen Antigenderivate.

Die neuen Verbindungen können nach an sich bekannten Methoden hergestellt werden.

So lassen sich sich erhalten, wenn man ein gegebenenfalls mit Brückengliedern verknüpftes Antigen mit gegebenenfalls mit Brückengliedern verknüpften Muramylpeptiden, wobei einer der beiden Teile mindestens eine freie Amino-, Hydroxy- oder Mercaptogruppe und der andere mindestens eine Carbonsäuregruppe aufweist, miteinander kondensiert, und, wenn erwünscht, die erhaltene Verbindung an einen gegebenenfalls mit Brükkengliedern verbundenen Träger kondensiert.

Die Kondensationen erfolgen dabei z.B. in der Weise, dass man die eine Verbindung in Form einer aktivierten Carbonsäure mit der andern Verbindung als freie Amino-, Hydroxy- oder Mercaptoverbindung umsetzt. Die aktivierte Carbhoxylgruppe kann beispielsweise Säureanhydrid, vorzugsweise ein Säureazid, ein Säureamid, wie ein Imidazolid, Isoxazolid oder ein aktivierter Ester sein. Als aktivierte Ester seien insbesondere genannt: Cyanmethylester, Carboxymethylester, p-Nitrophenylthioester, Methoxyäthylthioester, Acetylaminoäthylthioester, p-Nitrophenylester, 2-4-5-Trichlorophenylester, N-Hydroxysuccinimidester, N-Hydroxyphthalimidester, 8-Hydroxychinolinester, N-Hydroxypiperidinester. Aktive Ester können auch gegebenenfalls mit einem Carbondiimid unter Zusatz von N-Hydroxysuccinimid oder einem unsubstituierten oder z.B. durch Halogen, Methyl oder Methoxy substituierten 1-Hydroxybenzotriazol oder 3-Hydroxy-4-oxo-3,4-dihydrobenzo[d]-1,2,3-triazin erhalten werden.

Die bei dieser Kondensation verwendeten Abgangsgruppen müssen ungiftig oder aber gut entfernbar sein, um zu vermeiden, dass die meist hochmolekularen Verbindungen durch Adsorption giftige Teilstücke zurückbehalten.

Man bevorzugt deshalb aktive Ester solche mit N-Hydroxysuccinimid oder deren C-Substitutionsprodukten, wie N-Hydroxy-methyl- oder -dimethylsuccinimid, oder die Umsetzung mit Carbodiimid, wie Carbodiimid selbst oder 1-Äthyl-3-(3-dimethylaminopropyl)-carbodiimid.

Die obigen Reaktionen werden in üblicher Weise in An- und Abwesenheit von Verdünnungs- Kondensations- und/oder katalytischen Mitteln, falls notwendig, bei erniedrigter oder erhöhter Temperatur durchgeführst. Vorzugsweise arbeitet man, um die Antigene nicht zu zerstören, in wässrigem Milieu und bei einem pH-Bereich von 6—9, in erster Linie von 7—8.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Verfahrensschritte durchführt oder bei denen man Ausgangsstoffe unter den Verfahrensbedingungen herstellt, oder bei denen eine Reaktionskomponente gegebenenfalls in Form ihrer Derivate, wie ihre Salze und/oder in Form von Isomerengemischen oder reinen Isomeren einsetzt.

Zweckmässig verwendet man für die Durchführung der erfindungsgemässen Kondensation solche Ausgangsstoffe, die zu den eingangs besonders erwähnten Gruppen von Endstoffen und vor allem zu den speziell beschriebenen oder hervorgehobenen Endstoffen führen.

Die verwendeten Ausgangsstoffe sind bekannt, oder, falls neu, lassen sich nach an sich bekannten Methoden herstellen.

Die vorliegende Erfindung betrifft ebenfalls pharmazeutische Präparate, welche die neuen Antigenderivate enthalten. Bei den erfindungsgemässen pharmazeutischen Präparaten handelt es sich um solche zur enteralen, wie oralen oder rektalen, sowie parenteralen Verabreichung an Warmblüter, welche den pharmakologischen Wirkstoff allein oder zusammen mit einem pharmazeutisch anwendbaren Trägermaterial enthalten.

Die neuen pharmazeutischen Präparate enthalten von etwa 10% bis etwa 95%, vorzugsweise von etwa 20% bis etwa 90% des Wirkstoffes. Erfindungsgemässe pharmazeutische Präparate in Dosiseinheitsform, wie Dragées, Tabletten, Kapseln, Suppositorien oder Ampullen.

Die pharmazeutischen Präparate der vorliegenden Erfindung werden in an sich bekannter Weise, z.B.

mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hifsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose, und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess- Reglier- und Schmiermittel, z.B. Kieselsäure, Talk, Stearinsäure oder Salz davon, wie Magnesium-oder Calciumstearat, und/oder Polyäthylenglykol. Dragée-Kerne werden mit geeigneten, gegebenenfalls Magensaft-resisten Überzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcelulosephthalat, verwendet. Den Tabletten oder Dragée-Überzügen können Farbstoffe oder Pigmente, z.B. zur Identifizierung oder zur Kennzichnung verschiedener Wirkstoffen beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Zu parenteralen Verabreichung eignen sich in erster Linie wässrige Lösungen eines Wirkstoffes in wasserlöslicher Form, z.B. eines wasserlöslichen Salzes, ferner Suspensionen des Wirkstoffs, wie entsprechende ölige Injektionssupensionen, wobei man geeignete lipophile Lösungsmittel oder Vehikel, wie fette Öle, z.B. Sesamöl, oder synthetische Fettsäureester, z.B. Äthyloleat oder Triglyceride, verwendet, oder wässrige Injektionssuspensionen, welche viskositätserhöhende Stoffe, z.B. Natrium-Carboxymethylcellulose, Sorbit und/oder Dextran und gegebenenfalls Stabilisatoren enthalten.

Eine bevorzugte Applikationsart besteht in einer Lösung oder Suspension der neuen Antigenderivate enthaltend, vorzugsweise bis zu 10 Gew.-Prozen, Carboxymethylcellulose.

Die Erfindung umfasst ebenfalls die Verwendung der neuen Antigenderivate, also pharmakologisch wirksame Stoffe, insbesondere als Immunisierungsmittel, vorzugsweise in Form von pharmazeutischen Präparaten. Die Dosierung des Wirkstoffs hängt vom Warmblüter-Spezies, dem Körpergewicht und Alter und von individuellen Zustand, sowie von der Applikationsweise ab.

Dabei werden die neuen Impfstoffe in Anlehnung an die bei bekannten Impfverfahren erkannten und bekannten Dosierungen in Gewichts- oder internationalen Einheiten appliziert, z.B. gibt man Lymphoblasten enthaltende Antigenderivate in einer Menge die pro Injektion $10^6$—$10^{10}$ Zellorganismen enthalten, in Abständen von 2—8 Wochen 1—6 mal. Dabei sollen vorzugsweise pro mg Protein 5—200 mikro- Muramylpeptide enthalten sein.

Vorzugsweise verwendet man zur Immunisierung mit löslichen Verbindungen die entsprechende Menge Antigenderivat in einer Falz-Lösung (BSS) bestehend aus 0,14 g Calziumchlorid, 8,0 g Kochsalz, 0,2 g $7H_2O$, 0,2 g Magnesiumsulfat. Magnesiumchlorid. $6H_2O$, 0,6 g Kaliumdihydrogenphosphat 0,24 g Dinatriumhydrogenphosphat. $2H_2O$ per 1 Liter Wasser. Falls eine Depotwirkung angestrebt wird $7H_2O$, 0,2 g (z.B. bei lokaler, intradermaler oder intrammolekularer Applikation) kann dem so gelösten Antigenderivat noch Carboxymethylzellulose (Endkonzentration vorzugsweise 5%) zugegeben werden.

Nicht-lösliche makromolekulare Antigenderivate appliziert man vorzugsweise als Suspension in BSS und Carboxymethylzellulose als Stabilisator (Endkonzentration vorzugsweise 5%). Dabei wird zur Herstellung einer stabilisen Suspension das auf Eis gekühlte Gemisch bevorzugt konzentriert mit Ultraschall behandelt.

Antigenderivate mit Zellen werden in erster Linie in einem für den jeweiligen Zelltyp besonders geeigneten Gewebekulturmedium (z.B. für Lymphozyten EAGLE'S high amino acid medium) [vgl. Click et al. Cell. Immunol. vol. 3, p. 264—276 (1972)] appliziert.

Die zum Teil neuen als Ausgangsstoffe dienenden Muramylpeptide der Formel II können erhalten werden, wenn man in an sich bekannter Weise eine Verbindung der Formel

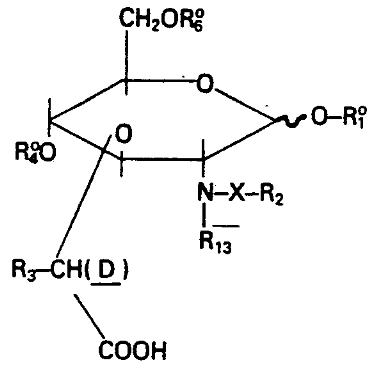

(IV)

worin X, $R_2$, $R_3$ und $R_{13}$ die obgenannte Bedeutung besitzen und $R_1^o$, $R_4^o$ und $R_6^o$ für die Reste $R_1$, $R_4$ bzw. $R_6$ oder für eine leicht abspaltbare Schutzgruppe stehen, oder ein Derivat davon mit einer Verbindung der Formel

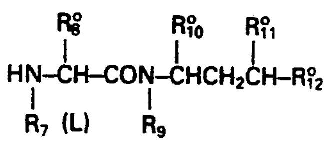

(V)

worin $R_8^o$, $R_{10}^o$ und $R_{12}^o$ die Bedeutung von $R_8$, $R_{10}$, $R_{11}$ und $R_{12}$ besitzen, mit der Massgabe, dass in diesen Resten vorhandene Carboxy- und, wenn erwünscht, freie Hydroxylgruppen durch leicht abspaltbare Schutzgruppen geschützt sind, kondensiert und gegebenenfalls vorhandene Schutzgruppen abspaltet.

Die Kondensation erfolgt dabei z.B. in der Weise, dass man die Verbindung IV in Form der aktivierten Carbonsäure mit der Aminoverbindung V umsetzt oder dass man die Säure IV mit der Verbindung V, deren Aminogruppe in aktivierter Form vorliegt, umsetzt. Die aktivierte Carboxylgruppe kann beispielsweise ein Säureanhydrid, vorzugsweise ein gemischtes Säureanhydrid wie ein Säureazid, ein Säureamid, wie ein Imidazolid, Isoxazolid oder ein aktivierter Ester sein. Als aktivierte Ester seien insbesondere genannt: Cyanmethylester, Carboxymethylester, p-Nitrophenylthioester, p-Nitrophenylester, 2,4,5-Trichlorphenylester, Pentachlorphenylester, N-Hydroxysuccininimidester, N-Hydroxyphthalimidester, 8-Hydroxychinolinester, 2-Hydroxy-1,2-di-hydro-1-carboäthoxy-chinolin-ester, N-Hydroxypiperidinester oder Enolester, die mit N-Äthyl-5-phenyl-isoxazolium-3'-sulfonat gewonnen werden. Aktivierte Ester können auch gegebenenfalls mit einem Carbodiimid unter Zusatz von N-Hydroxysuccinimid oder einem unsubstituierten oder z.B. durch Halogen, Methyl oder Methoxy substituierten 1-Hydroxybenzotriazol, 3-Hydroxy-4-oxo-3,4-dihydro-benzo[d]-1,2,3-triazin erhalten werden.

Die Aminogruppe ist beispielsweise durch Reaktion mit einem Phosphitamid aktiviert.

Unter den Methoden der Reaktion mit aktivierten Estern sind insbesondere diejenigen mit N-Äthyl-5-phenyl-isoxazolium-3'-sulfonat (Woodward Reagens K) oder 2-Äthoxy-1,2-dihydro-1-carboäthoxy-chinolin oder Carbodiimid zu erwähnen.

Leicht abspaltbare Schutzgruppen sind solche, die aus der Peptid- bzw. Zuckerchemie bekannt sind. Für Carboxygruppen sollen insbesondere tertiär-Butyl, Benzyl oder Benzhydryl und für Hydroxygruppen insbesondere Acylreste, z.B. Niederalkanoylreste wie Acetyl, Aroylreste, wie Benzoyl und vor allem von der Kohlensäure sich ableitende Reste wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl oder Tetrahydropyranyl oder gegebenenfalls substituierte Alkylidenreste, die die Sauerstoffatome in 4- und 6-Stellung verbinden, genannt werden. Solche Alkylidenreste sind insbesondere ein Niederalkyliden-, in erster Linie der Äthyliden-, Isopropyliden- oder Propylidenrest oder auch ein gegebenenfalls substituierter, vorzugsweise in p-Stellung substituierter Benzylidenrest.

Diese Schutzgruppen können in an sich bekannter Weise abgespalten werden. So kann man sie hydrogenolytisch z.B. mit Wasserstoff in Gegenwart eines Edelmetall-, wie Palladium- oder Platinkatalysators oder durch saure Hydrolyse entfernen.

Die verwendeten Ausgangsstoffe sind bekannt, oder lassen sich in man sich bekannter Weise herstellen.

Eine andere Verfahrensweise zur Herstellung dieser Ausgangsstoffe besteht darin, dass man in an sich bekannter Weise eine Verbindung der Formel VI

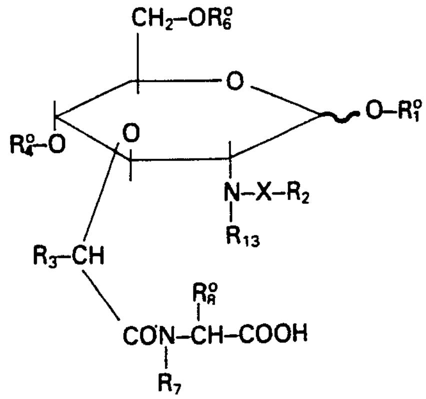

(VI)

worin $R_{10}^{o}$, $R_2$, $R_3$, $R_4^{o}$, $R_6^{o}$, $R_7$ und $R_8^{o}$ die obgenannten Bedeutungen haben, mit einer Verbindung der Formel

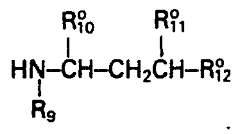

(VII)

worin $R_1^{o}$, $R_{11}^{o}$ und $R_{12}^{o}$ die obgenannte Bedeutung haben, mit der Massgabe, dass in den Resten $R_8^{o}$, $R_{10}^{o}$ und $R_{12}^{o}$ vorhandene Carboxyl- und, wenn erwünscht, freie Hydroxygruppen durch leicht abspaltbare Schutzgruppen geschützt sind, kondenziert und gegebenenfalls vorhandene Schutzgruppen abspaltet.

Die Kondensation erfolgt dabei z.B. in der Weise, dass man die Verbindung VI in Form der aktivierten Carbonsäure mit der Aminoverbindung VII umsetzt, oder dass man die Säure VI mit der Verbindung VII, deren Aminogruppen in aktivierter Form vorliegt, umsetzt. Die aktivierte Carboxylgruppe kann beispielsweise ein Säureanhydrid, vorzugsweise ein gemischtes Säureanhydrid, ein Säureamid oder ein aktivierter Ester sein. Als solche kommen insbesondere die oben genannten Säureanhydride, Amide oder Ester in Frage. Die Aminogruppe ist beispielsweise durch Reaktion mit einem Phosphitamid aktiviert.

Auch die leicht abspaltbaren Schutzgruppen entsprechen den den bereits oben genannten. Sie können in an sich bekannter Weise abgespalten werden; z.B. hydrogenolytisch, beispielsweise mit Wasserstoff in Gegenwart eines Edelmetall -wie Palladium- oder Platin-katalysators oder durch saure Hydrolyse.

Die Ausgangsstoffe lassen sich in an sich bekannter Weise erhalten. So kann man z.B. entsprechende in 3-Stellung unsubstituierte Zucker mit einer Halogen-$R_3$-acetamido-$R_8^{o}$-essigsäure umsetzten, oder eine Verbindung der Formel IV mit einer Amino-$R_8^{o}$-essigsäure, deren Carboxylgruppe geschützt ist in der oben gezeigten Weise umsetzen, und die Schutzgruppe abspalten.

Eine weitere Verfahrensmethode zur Einführung der in 3-Stellung des Zuckerrestes sitzenden Seitenkette besteht darin, dass man eine Verbindung

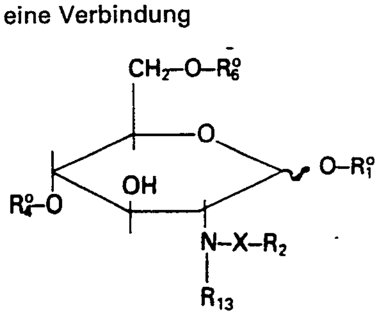

(VIII)

worin X, $R_2$, $R_1^{o}$, $R_4^{o}$, $R_6^{o}$ und $R_{13}$ die obgenannten Bedeutungen haben, und gegebenenfalls darin vorhandene Hydroxygruppen mit einer leicht abspaltbaren Schutzgruppe geschützt sind, mit einer Verbindung der Formel

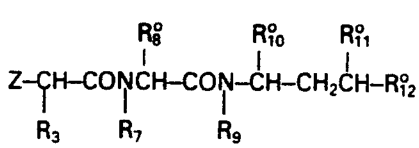

(IX)

umsetzt, worin Z eine reaktionsfähig veresterte Hydroxygruppe darstellt und $R_8^0$, $R_{10}^0$, $R_{11}^0$, $R_{12}^0$ die oben angegebene Bedeutung haben, und gegebenenfalls vorhandene Schutzgruppen abspaltet.

Eine reaktionsfähig veresterte Hydroxygruppe ist insbesondere eine mit einer starken anorganischen oder organischen Säure veresterte Hydroxygruppe, in erster Linie eine solche, die mit Halogenwasserstoffsäuren, wie Chlor-, Brom- oder Jodwasserstoffsäure, verestert ist.

Die leicht abspaltbaren Schutzgruppen entsprechen den bereits oben genannten. Sie können in an sich bekannter Weise abgespalten werden, z.B. hydrogenolytisch beispielsweise mit Wasserstoff in Gegenwart eines Edelmetall- wie Palladium- oder Platin-Katalysators oder durch saure Hydrolyse.

Die als Ausgangsstoffe zu verwendenden Muramylpeptide der Formel II, worin X eine Carbonylgruppe, $R_1$, $R_4$ und $R_6$ Triniederalkylsilyl, in erster Linie Trimethylsilyl, $R_2$ gegebenenfalls substituiertes Alkyl oder carbocyclisches Aryl, $R_3$ Wasserstoff oder Alkyl, $R_7$ und $R_{13}$ Wasserstoff oder Niederalkyl, $R_8$ Wasserstoff, Niederalkyl, freies, verestertes oder veräthertes Hydroxyniederalkyl, freies, verestertes oder veräthertes Mercapto-niederalkyl, freies oder acyliertes Aminoniederalkyl, Cycloalkyl mit 5 oder 6 Kohlenstoffatomen, Cycloalkyl-niederalkyl, dessen Cycloalkylrest 5 oder 6 Kohlenstoffatome enthält, gegebenenfalls substituiertes Aryl oder Aralkyl, stickstoffhaltiges Heterocyclyl- oder Heterocyclyl-niederalkyl, $R_7$ und $R_8$ zusammen auch Alkylen mit 3 oder 4 Kohlenstoffatomen, $R_9$ Wasserstoff oder Niederalkyl und die Reste $R_{10}$, $R_{11}$ und $R_{12}$ unabhängig voneinander einen gegebenenfalls veresterten oder amidierten Carboxyrest und $R_{11}$ auch Wasserstoff bedeuten, können erhalten werden, wenn man in an sich bekannter Weise eine Verbindung der Formel

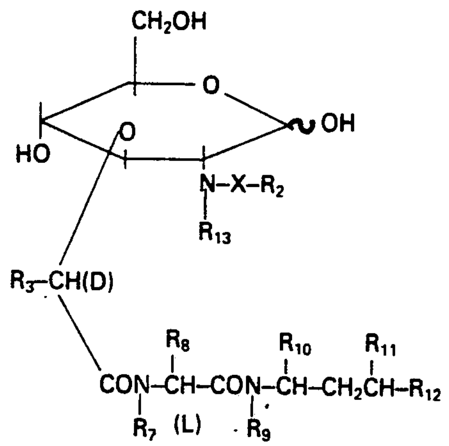

(X)

mit einem reaktionsfähigen Ester einer Tri-niederalkylsilylverbindung, umsetzt.

Als reaktionsfähige Ester einer Tri-niederalkylsilyl-verbindung seien insbesondere genannt: Triniederalkyl-silyl-halogenide, besonders -Chloride oder -Bromide, Bis-niederalkyl-silyl-acetamid oder -sulfamid.

Die Reaktion wird vorzugsweise in einem Lösungsmittel, das keine reaktionsfähigen Hydroxy- oder Aminogruppe enthält, wie Dimethylformamid, Dioxan, Tetrahydrofuran, Dimethoxyäthan oder Chloroform vorgenommen.

Die nachfolgenden Beispiele illustrieren die obenbeschriebene Erfindung; sie sollen jedoch diese in ihrem Umfang in keiner Weise einschränken, Temperaturen werden in Celsiusgraden angegeben.

Beispiel 1

Zu einer Lösung von 1 g Rinderserumalbumin in 100 ml einer 0,1 M Natriumhydrogencarbonat-0,5 M Kochsalzlösung werden unter Rühren 500 mg 2-Acetamido-3-O-{[*L*-1-)*D*-1-carbamoyl-3-succinimidooxycarbonyl-propyl)-carbamoyläthyl]-carbamoyl-methyl}-2-deoxy-D-glucose zugegeben. Die Lösung wird 4 Stunden bei Raumtemperatur gerührt und anschliessend sterilfiltriert (MF-Milipore [Warenzeichen], 0,45 μm Sterilfilter). Das konjugierte Rinderserumalbumin wird im Dialfiltrationsverfahen durch einen Amikon UM-10 (Warenzeichen) Filter (Ultrafiltrationsmembran mit einer nominalen Tenngrenze von 10 000 Dalton) von niedermolekularen Reaktionsprodukten bzw. von Salzen abgetrennt und gefriergetrocknet.

Die quantitative Bestimmung des an das Rinderserum Albumin gebundenen Muramylpeptids erfolgt mit der Morgan-Elson Reaktion nach der Modifikation von J. M. Ghuysen et al. [in "Methods in Enzymology" *8*, (1966) 629]. Durchschnittlich werden 60 μg des Muramylpeptids in 1 mg Rinderserumalbumin-Verbindung gefunden.

Der Succinimmidoester, der als Ausgangsstoff verwendet wird, lässt sich z.B. wie folgt herstellen:

1 mMol 2-Acetamido-3-O-{[*L*-1-(*D*-1-carbamoyl-3-carboxy-propyl)-carbamoyl-äthyl]-carbamoyl-methyl}-2-deoxy-*D*-glucose, 1 mMol Dicyclohexylcarbodiimid und 1, 1 mMol N-Hydroxysuccinimid werden in 3 ml absolutem Dimethylformamid gelöst und im geschlossenen Gefäss unter Wasserausschluss 20 Stunden gerührt. Der ausgefallene Dicyclohexylharnstoff wird abgetrennt, das Lösungsmittel im

Hochvakuum abgedampft und der Rückstand mit Äther behandelt, dann abgesaugt und getrocknet. Der so erhaltene Succinimidooxyester kann unter Feuchtigkeitsausschluss, z.B. in Stickstoffampulle, aufbewahrt werden.

Beispiel 2

Kondensation des im Beispiel 1 erhaltenen Antigenderivats an aktivierte Agarose; dabei verwendet man ein kommerziell erhältliches Agarosederivat von BIO-RAD, dass Affi-Gel 10 (Warenzeichen). Es enthält auf einem Agarosegerüst, Äther-Seitenketten ("spacer arms") von N-Alkyl-succinamiden, die mit N-Hydroxysuccinimid verestert sind.

Man löst 120 mg des nach Beispiel 1 erhaltenen Rinderserumalbumin-derivats in 12,5 ml 0,1 M Phosphatpuffer, pH 7,3 bei 4°C. 500 mg Affi-Gel 10® werden dann in der Lösung durch Shütteln suspendiert und 4 Stunden bei 4°C weitergeschüttelt. Die noch verbliebenen aktiven Estergruppen werden durch 30-minütige Behandlung mit einer 1 M Äthanolamin.HCl-0,1 M Phosphatpuffer-Lösung (pH 7,3) umgesetzt. Danach wird das Gel in eine Säule gefüllt und zuerst mit 200 ml 0,1 M Phosphatpuffer-1 M Kochsalz-Lösung (pH 7,3) danach mit 20 ml physiologischer Kochsalzlösung gewaschen.

Die Bestimmung von an das Affi-Gel 10® ankondensierten Rinderserumalbumin-Muramylpeptid-Verbindungen erfolgt durch die quantitative Analyse von repräsentativen Rinderserumalbumin-Aminosäuren in Totalhydrolysaten von definierten Gel-Aliquoten. Durchschnittlich werden 9—10 mg des Rinderserumalbumin-Muramylpeptid-Derivats an 1 ml gequollenes Affi-Gel 10® gebunden.

Beispiel 3

Schaferythrocytenmembranen werden aus frischem Schafsblut nach der Methode von Dodge, J. I. et al. [Arch. Biochem. Biophys. 100, (1963) p. 114—180] gewonnen. Zu einer Suspension von 500 mg Schaferythrocytenmembranen in 50 ml 0,1 M Natriumhydrogencarbonat-0,1 M Kochsalzlösung werden unter Rühren 400 mg 2 - Acetamido - 3 - O - {[*L* - 1 - (*D* - 1 - carbamoyl - 3 - succinimidooxycarbonyl - propyl) - carbamoyl - äthyl] - carbamoyl - methyl} - 2 - deoxy - D - glucose zugegeben, und das Gemisch 4 Stunden bei Raumtemperatur gerührt. Danach werden das Erythrocytenmembran-Konjugat durch eine einstündige Ultrazentrifugation bei 90 000 g und 4°C sedimentiert. Das Sediment wird dreimal — jeweils durch Resuspendieren und Ultrazentrifugieren — mit Phosphat-gepufferter Kochsalzlösung und einmal mit destilliertem Wasser gewaschen. Das gewaschene Erythrocytenmembran-Konjugat wird in 100 ml destilliertem Wasser suspendiert und gefriergetrocknet.

Die quantitative Bestimmung von an die Schaferythrocyten gebundenem Muramyldipeptid erfolgt mit dem Morgan-Elson Reaktion und ergibt 25 µg Muramyldipeptid pro 1 mg Erythrocytenmembran.

Beispiel 4

100 mg Gruppe C Polysaccharid von Neisseria meningitidis und 110 mg (0,2 mMol) 2 - Acetamido - 3 - O - {*L* - 1 - *D* - 1 - carbamoyl - 3 - N - aminoäthyl - carbamoyl - propyl) - carbamoyl - äthyl] - carbamoyl - methyl} - 2 - deoxy - D - gluclose - HCl werden in 10 ml destilliertem Wasser gelöst, der pH-Wert der Lösung wird mit verdünnter Salzsäure auf 5 eingestellt.

19,2 mg 1-Äthyl-3-(3-dimethylaminopropyl)-carbodiimid.HCl werden unter Rühren zu der Lösung gegeben. Die Mischung wird 1 Stunde bei Raumtemperatur gerührt, der pH-Wert wird — unter Zugabe von verdünnter Natronlauge — bei 5 gehalten, danach werden 5 ml 2 M Natriumacetat-Pufferlösung, pH 5, zugegeben und 30 Minuten weitergerührt. Anschliessend wird der pH-Wert mit Natronlauge auf 7 eingestellt. Die Lösung wird sterilfiltriert und bei 4°C gegen destilliertes Wasser dialysiert und anschliessend gefriergetrocknet.

Die quantitative Bestimmung von an das C-Polysaccharid gekupeltem Desmethylmuramyldipeptid erfolgt wie in Beispiel 1 beschrieben mit der Morgan-Elson Reaktion und ergibt 80 µg Desmethylmuramyldipeptid pro 1 mg Polysaccharid.

Das verwendete 2 - Acetamino - 3 - O - {[*L* - 1 - (D - 1 - carbamoyl - 3 - N - aminoäthyl - carbamoyl - propyl) - carbamoyl - äthyl] - methyl} - 2 - desoxy - D - glucosehydrochlorid lässt sich z.B. wie folgt herstellen:

1 mMol 2 - Acetoamino - 3 - O - {[*L* - 1 - (D - 1 - carbamoyl - 3 - carboxy - propyl) - carbamoyl - äthyl] - carbamoylmethyl} - 2 - desoxy - D - glucose und 2 mMol N-Äthyl-N'-(3-dimethyl-aminopropyl)-carbodimid.HCl werden in 10 ml Wasser gelöst und das pH mit Salzsäure auf pH 5,0 eingestellt; danach wird eine Lösung von 5 mMol Äthylendiamindihydrochlorid in 10 ml Wasser zugegeben. Der Ansatz wird bei pH 5,0 und Raumtemperatur 6 Stunden gerührt. Das Gemisch wird auf eine schwach saure Kationenaustauschersäule-Amberlite CG 50 II (Warenzeichen), 2,5 × 45 cm, aufgetragen und mit einem linearen Gradienten von 0,05 M Pyridinacetat, pH 6 (300ml) zu 0,5 M Pyridinacetat, pH 3,7 (300 ml) eluiert. Die, das erhaltene 2 - Acetamino - 3 - O - {[L - 1 - (D - 1 - carbamoyl - 3 - N - aminoäthyl - carbamoyl - propyl) - carbamoyl - äthyl] - carbamoyl - methyl} - 2 - desoxy - D - glucose - acetat enthaltenden Fraktionen — die Fraktionen werden mit Ninhydrin bzw. Hochspannungselektrophorese getestet und charaktersiert — werden gefriergetrocknet. Die Umwandlung in die Hydrochloridform geschieht folgendermassen: das Lyophilisat löst man in 6 ml 0,2 N HCl und chromatographiert an einer Bio-Gel P2-Säule (Warenzeichen, Molekularsieb aus Polyacrylamid, Trennbereich 100—1800 Dalton), 2,5 × 90 cm, mit Wasser als Elutionsmittel. Die 2 - Acetamino - 3 - O{[L - 1 - (D - 1 - carbamoyl - 3 - N -

aminoäthyl - carbamoylpropyl) - carbamoyl - äthyl] - carbamoyl - methyl} - 2 - desoxy - D - glucose - hydrochloridhaltigen Fraktionen werden gefriergetrocknet. Das Präparat ist einheitlich in der Hochspannungselektroporese. Bei der Bestimmung der Konstituenten in Totalhydrolysaten wird das molekulare Verhältnis 1 Muraminsäure: 1 L-Alanin: 1 D-Glutaminsäure: 1 Äthylendiamin gefunden.

In analoger Weise erhält man ausgehend von entsprechenden Muramyldipeptiden die entsprechenden β'-Aminoäthylamid. HCl-Verbindungen, nämlich:

2-Acetamino-3-O-{*D*-1-[*L*-1-(*D*-carbamoyl-3-N-aminoäthyl-carbamoyl-propyl)-carbamoyl-äthyl]-carbamoyl-äthyl}-2-desoxy-D-glucose.HCl,

2-Benzoylamino-3-O-{*D*-1-[*L*-1-(*D*-1-carbamoyl-3-N-aminoäthyl-carbamoyl-propyl)-carbamoyl-äthyl]-carbamoyl-äthyl}-2-desoxy-D-glucose.HCl,

2-Benzoylamino-3-O-{*L*-1-(*D*-1-carbamoyl-N-aminoäthyl-carbamoyl-propyl)-carbamoyl-äthyl]carbamoyl-methyl}-2-desoxy-D-glucose.HCl,

2-Acetamino-3-O-{*L*-1-(*D*-1-N-carbamoyl-methyl-carbamoyl-3-N-aminoäthyl-carbamoyl-propyl)-carbamoyl-äthyl]carbamoyl-methyl}-2-desoxy-D-glucose.HCl,

2-Benzoylamino-3-O-{*L*-1-(*D*-1-carbamoyl-3-N-aminoäthyl-carbamoyl-propyl)-carbamoylpropyl]-carbamoyl-methyl}-2-desoxy-D-glucose.HCl,

2-Propionylamino-3-O-{[*L*-1-(*D*-1-carbamoyl-3-N-aminoäthyl-carbamoyl-propyl)-carbamoyl-äthyl]-carbamoyl-methyl}-2-desoxy-D-glucose.HCl,

2-Acetylamino-3-O-{[*L*-1-(*D*-1-carbamoyl-3-N-aminoäthyl-carbamoyl-propyl)-carbamoylpropyl]-carbamoyl-methyl}-2-desoxy-D-glucose.HCl,

2-Acetylamino-3-O-{[*L*-1-(*D*-1-carbamoyl-3-N-aminoäthyl-carbamoyl-propyl)-carbamoyl-2-hydroxyäthyl]-carbamoyl-methyl}-2-desoxy-D-glucose.HCl,

2-Propionylamino-3-O-{[*L*-1-(*D*-1(oder 3)-carboxy-3(oder 1)-N-aminoäthyl-carbamoyl-propyl)carbamoyl-äthyl]-carbamoyl-methyl}-2-desoxy-D-glucose.HCl,

2-Benzoylamino-3-O-{[*L*-1-(*D*-1-N-carbamoyl-methyl-carbamoyl-3-N-aminoäthyl-carbamoyl-propyl)-carbamoyl-äthyl]-carbamoyl-methyl}-2-desoxy-D-glucose.HCl,

2-Benzoylamino-3-O-{[*L*-1-(*D*-1-carboxy-3-N-aminoäthyl-carbamoyl-propyl)-carbamoyl-äthyl]carbamoyl-methyl}-2-desoxy-D-glucose.HCl,

2-Acetamino-3-O-{*L*-1-(*D*-1-carbamoyl-3-N-aminoäthyl-carbamoyl-propyl)-carbamoyl-2'-methyl-propyl]-carbamoyl-methyl}-2-desoxy-D-glucose.HCl,

2-Acetamino-3-O-{*D*-1-[*L*-1-(*D*-1-carbamoyl)-3-(1-N-aminoäthyl-carbamoyl)-äthyl-carbamoylpropyl)-carbamoyl-äthyl]-carbamoyl-äthyl}-2-desoxy-D-glucose.HCl,

2-Acetamino-3-O-{[*L*-1-(*D*-1-carboxy-3-N-aminoathyl-carbamoyl-propyl)-carbamoyl-2'-methyl-propyl]-carbamoyl-methyl}-2-desoxy-D-glucose.HCl,

2-Acetamino-2-O-{[*L*-1-(*D*-1-carbamoyl-3-N-aminoäthyl-carbamoyl-propyl)-carbamoyl-N.N-tetramethylen]-carbamoyl-methyl}-2-desoxy-D-glucose.HCl,

2-Acetamino-3-O-{[*L*-1-(*D*-1-carbamoyl-3-N-aminoäthyl-carbamoyl-propyl)-carbamoyl-äthyl]-N-methyl-carbamoyl-methyl}-2-desoxy-D-glucose.HCl oder

2-Benzoylamino-3-O-{[*L*-1-(*D*-1-carbamoyl-3-N-aminoäthyl-carbamoyl-propyl)-carbamoyl-2'-methyl-propyl]-carbamoyl-methyl}-2-desoxy-D-glucose.HCl.

Diese Verbindungen werden wie oben beschrieben mit Gruppe C Polysaccharid von Neisseria meningitidis kondensiert.

## Beispiel 5

Unmittelbar nach der Gewinnung werden Merozoiten des Malariaerregers Plasmodium knowlesi — die gesamte Ausbeute aus dem Blut eines infizierten Rhesusaffen [Methode der Gewinnung von Merozoiten: siehe G. H. Mitchel et al. (1975), Immunology, 29, 397] — in einer Lösung von 100 mg (0,17 mMol) 2 - Acetamino - 3 - O - {[*L* - 1 - (*D* - 1 - carbamoyl - 3 - succinimidooxy - carbonyl - propyl) - carbamoyl - äthyl] - carbamoyl - methyl} - 2 - desoxy - D - glucose in 15 ml physiologischer Pufferlösung, pH 7.2, suspendiert. Die Suspension wird eine Stunde bei 37°C inkubiert. Danach weren die Konjugate durch Zentrifugation sedimentiert. Das Sediment wird durch resuspendieren in physiologischer Pufferlösung und erneutes Zentrifugieren gewaschen. Die gewaschenen Merozoiten-Konjugate werden nach G. H. Mitchel (Ref. siehe oben) in 10%-igem autologem Rhesusaffenserum suspendiert und gefriergetrocknet.

Die quantitative Bestimmung von an die Meroziten gekuppeltem Muramyldipeptid erfolgt mit der Morgan-Elson Reaktion und ergibt 40—60 µg Muramyldipeptid pro 1 mg Merozoiten.

## Beispiel 6

In einer Lösung von 200 mg (0,34 mMol) 2-Acetamino-3-O-{[*L*-1-(D-1-carbamoyl-3-succinimidooxy-carbonyl-propyl)-carbamoyl-äthyl]-carbamoyl-methyl}-2-deoxy-D-glucose in 20 ml Phosphat-gepufferter physiologischer Kochsalzlösung, pH 7,2, suspendiert man $10^9$ T-Lymphoblasten von CBA/J Mäusen. (Die T-Lymphoblasten werden in einer "mixed lymphocyte culture" gegen C57BL/6 Maus Stimulatorzellen nach L. C. Anderson et al., (1977), The Journal of Experimental Medicine, 146, 1124, gewonnen). Die Suspension wird 90 Minuten bei Raumtemperatur inkubiert. Danach werden die Lymphoblasten-Konjugate durch Zentrifugation sedimentiert und durch Resuspendieren in Phosphat-gepufferter physiologischer

Kochsalzlösung und erneutes Zentrifugieren gewaschen.

Die quantitative Bestimmung von an die T-Lymphoblasten gekuppeltem Muramyldipeptid erfolgt mit der Morgan-Elson Reaktion (siehe Beispiel 1) und ergibt 60—70 µg Muramyldipeptid pro $10^7$ T-Lymphoblasten.

Beispiel 7

In analoger Weise zu den vorstehenden Beispielen erhält man Rinderserumalbumin gekuppelt mit

2-Acetamido-3-O-{D-1[*L*-1-(D-1-carbamoyl-3-carboxy-propyl)-carbamoyl-äthyl]-carbamoyl-äthyl}-2-deoxy-D-glucose,

2-Benzoylamino-3-O-{*D*-1-[*L*-1-(*D*-1-carbamoyl-3-carboxy-propyl)-carbamoyl-äthyl]-carbamoyl-äthyl]-2-deoxy-α,β-D-glucose,

2-Benzoylamino-3-O-{[*L*-1-(*D*-1-carbamoyl-3-carboxy-propyl)-carbamoyl-äthyl]-carbamoyl-methyl}-2-deoxy-α,β-D-glucose,

2-Acetamido-3-O-{[*L*-1-(*D*-1-carbamoyl-methyl-carbamoyl-3-carboxypropyl)-carbamoyl-äthyl]-carbamoyl-methyl}-2-deoxy-D-glucose,

2-Benzamido-2-deoxy-3-O-{[L-1-(D-1-carbamoyl-3-carboxy-propyl)-carbamoyl-propyl]-carbamoyl-methyl}-D-glucopyranose,

3-O-{[L-1-(D-1-carbamoyl-3-carboxy-propyl)carbamoyl-äthyl]-carbamoyl-methyl}-2-deoxy-2-propionamido-D-glucose,

2-Acetamido-3-O-{[*L*-1-(*D*-1-carbamoyl-3-carboxy-propyl)-carbamoyl-propyl]-carbamoyl-methyl}-2-deoxy-D-glucose,

2-Acetamino-3-O-{[*L*-1-(*D*-1-carbamoyl-3-carboxy-propyl)-carbamoyl-2-hydroxyäthyl]-carbamoyl-methyl}-2-desoxy-D-glucose,

2-Propionylamino-3-O-{[L-1-(*D*-1,3-dicarboxy-propyl)-carbamoyl-äthyl]-carbamoyl-methyl}-2-desoxy-D-glucose,

2-Benzoylamino-3-O-{[*L*-1-(*D*-1-N-carbamoyl-methyl-carbamoyl-3-carboxy-propyl)-carbamoyl-äthyl]-carbamoyl-methyl}-2-desoxy-D-glucose,

2-Acetamino-3-O-{[D-1-(*L*-1-carbamoyl-3-carboxy-propyl)-carbamoyl-2′-methyl-propyl]-carbamoyl-äthyl}-2-desoxy-D-glucose,

2-Benzoylamino-3-O-{[*L*-1-(D-1,3-dicarboxy-propyl)-carbamoyl-äthyl]-carbamoyl-methyl}-2-desoxy-D-glucose,

2-Acetamino-3-O-{[*L*-1-(*D*-1-carbamoyl-3-carboxy-propyl)-carbamoyl-2′-methyl-propyl]-carbamoyl-methyl}-2-desoxy-D-glucose,

2-Acetamino-3-O-{*D*-1-[*L*-1-(*D*-1-carbamoyl-3-(L-1-carboxy-äthyl)-carbamoyl-propyl)-carbamoyl-äthyl]-carbamoyl-äthyl}-2-desoxy-D-glucose,

2-Acetamino-3-O-{[L-1-(D-1,3-dicarboxypropyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylene]-carbamoyl-methyl}-2-D-glucose,

2-Acetamino-3-O-{[L-1-D-1-carbamoyl-3-carboxy-propyl)-carbamoyl-methyl]-N-methyl-carbamoyl-methyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose.

Beispiel 8

In analoger Weise zu Beispiel 3 erhält man Schaferythrocytenmembranen gekuppelt mit

2-Acetamino-3-O-{*D*-1-[*L*-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{[*L*-1-(D-1,3-dicarboxypropyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyläthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoylmethyl}-2-desoxy-D-glucose,

2-Acetamino-3-O-{[L-1-(D-1-carbamoyl-3-carboxy-propyl)-carbamoylpropyl]-carbamoylmethyl}-2-desoxy-D-glucose,

2-Acetamino-3-O-{[*L*-1-(D-1-carbamoyl-3-carboxypropyl)-carbamoyl-2-hydroxyäthyl]-carbamoyl-methyl}-2-desoxy-D-glucose,

2-Propionylamino-3-O-{[L-1-(*D*-1,3-dicarboxypropyl)-carbamoyläthyl]-carbamoylmethyl}-2-desoxy-D-glucose,

2-Benzoylamino-3-O-{[*L*-1-(*D*-1-N-carbamoylmethyl-carbamoyl-3-carboxy-propyl)-carbamoyläthyl]-carbamoylmethyl}-2-desoxy-D-glucose,

2-Acetamino-3-O-{[*L*-1-(*D*-1-carbamoyl-3-carboxy-propyl)-carbamoyl-2′-methyl-propyl]-carbamoylmethyl}-2-desoxy-D-glucose,

2-Acetamino-3-O-{*D*-1-[*L*-1-(*D*-1-carbamoyl-3-(L-1-carboxy-äthyl)-carbamoyl-propyl)-carbamoyläthyl]-carbamoyläthyl}-2-desoxy-D-glucose,

2-Acetamino-3-O-{[L-1-(D-1,3-dicarboxy-propyl)-carbamoyl-2′-methyl-propyl]-carbamoyl-methyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoylmethyl}-2-desoxy-D-glucose,

2-Acetamino-3-O-{[L-1-(D-1-carbamoyl-3-carboxy-propyl)-carbamoyl-äthyl]-N-methyl-carbamoylmethyl}-2-desoxy-D-glucose,

2-Acetamino-3-O-{D-1-(*L*-1-carbamoyl-3-carboxy-propyl)-carbamoyl-2′-methyl-propyl]-carbamoyläthyl}-2-desoxy-D-glucose.

Beispiel 9

In analoger Weise zu Beispiel 5 erhält man Merozoiten des Malariaerregers Plasmodium knowlesi gekuppelt mit

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{[L-1-(D-1,3-dicarboxypropyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyläthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoylmethyl}-2-desoxy-D-glucose,

2-Acetylamino-3-O-{[L-1-(D-1-carbamoyl-3-carboxy-propyl)-carbamoylpropyl]-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoylmethyl}-2-desoxy-D-glucose,

2-Propionylamino-3-O-{[L-1-(*D*-1,3-dicarboxypropyl)-carbamoyläthyl]-carbamoylmethyl}-2-desoxy-D-glucose,

2-Benzoylamino-3-O-{[*L*-1-(*D*-1-N-carbamoylmethyl-carbamoyl-3-carboxy-propyl)-carbamoyläthyl]-carbamoylmethyl}-2-desoxy-D-glucose,

2-Acetamino-3-O-{[*L*-1-(*D*-1-carbamoyl-3-carboxy-propyl)-carbamoyl-2′-methyl-propyl]-carbamoylmethyl}-2-desoxy-D-glucose,

2-Acetamino-3-O-{*D*-1-[*L*-1-(*D*-1-carbamoyl-3-(L-1-carboxy-äthyl)-carbamoyl-propyl)-carbamoyläthyl]-carbamoyläthyl}-2-desoxy-D-glucose,

2-Acetamino-3-O-{[L-1-(D-1,3-dicarboxypropyl)-carbamoyl-2′-methyl-propyl]-carbamoylmethyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoylmethyl}-2-desoxy-D-glucose,

2-Acetamino-3-O-{[L-1-(D-1-carbamoyl-3-carboxy-propyl)-carbamoyl-äthyl]-N-methyl-carbamoylmethyl}-2-desoxy-D-glucose,

2-Acetamino-3-O-{D-1-[*L*-1-carbamoyl-3-carboxy-propyl)-carbamoyl-2′-methyl-propyl]-carbamoyläthyl}-2-desoxy-D-glucose.

Beispiel 10

In einer Lösung von 100 mg 2-Acetamino-3-O-{D-1-[*L*-1-(*D*-1-carbamoyl-3-succinimidooxycarbonyl-propyl)-carbamoyl-äthyl]-carbamoyl-äthyl}-2-desoxy-D-glucose in 10 ml Phosphat-gepufferter physiologischer Kochsalzlösung, pH 7,2, suspendiert man $10^8$ Mammacarzinomzellen vom Hund. [Die Tumorzellen werden nach H. H. Sedlack, H. Messmann und F. R. Seiler, Behring Inst. Mitt., No. 55, 349—355 (1974) gewonnen]. Die Suspension wird 90 Minuten bei Raumtemperatur inkubiert. Danach werden die Tumorzellen-Muramyldipeptid-Konjugate durch Zentrifugation sedimentiert und durch Resuspendieren in Phosphatgepufferter physiologischer Kochsalzlösung und erneutes Zentrifugat gewaschen.

Die quantitative Bestimmung von an die Tumorzellen gekuppeltem Muramyldipeptid erfolgt mit der Morgan-Elson Reaktion (siehe Beispiel 1) und ergibt 60—80µ Muramyldipeptid pro $10^7$-Mammacarcinomzellen.

Beispiel 11

In analoger Weise zu Beispiel 10 erhält man Mammacarzinomzellen vom Hund gekuppelt mit

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{[*L*-1-(D-1,3-dicarboxypropyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyläthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoylmethyl}-2-desoxy-D-glucose,

2-Acetylamino-3-O-{[L-1-(D-1-carbamoyl-3-carboxy-propyl)-carbamoylpropyl]-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoylmethyl}-2-desoxy-D-glucose,

2-Propionylamino-3-O-{[L-1-(*D*-1,3-dicarboxypropyl)-carbamoyläthyl]-carbamoylmethyl}-2-desoxy-D-glucose,

2-Benzoylamino-3-O-{[*L*-1-(*D*-1-N-carbamoylmethyl-carbamoyl-3-carboxy-propyl)-carbamoyläthyl]-carbamoylmethyl}-2-desoxy-D-glucose,

2-Acetamino-3-O-{[*L*-1-(*D*-1-carbamoyl-3-carboxy-propyl)-carbamoyl-2′-methyl-propyl]-carbamoylmethyl}-2-desoxy-D-glucose,

2-Acetamino-3-O-{[*D*-1-[*L*-1-(*D*-1-carbamoyl-3-(L-1-carboxyäthyl)-carbamoyl-propyl)-carbamoyläthyl]-carbamoyläthyl}-2-desoxy-D-glucose,

2-Acetamino-3-O-{[L-1-(D-1,3-dicarboxy-propyl)-carbamoyl-2′-methyl-propyl]-carbamoyl-methyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoylmethyl}-2-desoxy-D-glucose,

2-Acetamino-3-O-{[L-1-(D-1-carbamoyl-3-carboxy-propyl)-carbamoyl-äthyl]-N-methyl-carbamoylmethyl}-2-desoxy-D-glucose,

2-Acetamino-3-O-{D-1-[*L*-1-carbamoyl-3-carboxy-propyl)-carbamoyl-2′-methyl-propyl-carbamoyläthyl}-2-desoxy-D-glucose.

Beispiel 12

Zu 10 ml einer Suspension von Maul- und Klauenseuche-Kultur Vakzine Behringwerke in Phosphatgepufferter physiologischer Kochsalzlösung, werden 100 mg 2-Acetamino-3-O-{[*L*-1-(*D*-1-carbamoyl-3-succinimidooxy-carbonyl-propyl)-carbamoyl-äthyl]-carbamoyl-methyl}-2-desoxy-D-glucose zugegeben. Die Suspension wird 4 Stunden bei 4°C geschüttelt. Danach werden das Virus-Muramyldipeptid-Konjugat sterilfiltriert, durch Dialyse gegen Wasser von niedermolekularen Reaktionsprodukten abgetrennt und gefriergetrocknet. Die quantitative Bestimmung von an die Viren gekuppeltem Muramyldipeptid erfolgt mit der Morgan-Elson Reaktion (siehe Beispiel 1).

Beispiel 13

In analoger Weise zu Beispiel 12 erhält man Maul- und Klauensäuche Vakzine gekuppelt mit

2-Acetamino-3-O-{*D*-1-[*L*-1-(*D*-1-carbamoyl-3-carboxy-propyl)-carbamoyl-äthyl]-carbamoyläthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{[*L*-1-(D-1,3-dicarboxypropyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyläthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoyl-methyl}-2-desoxy-D-glucose,
2-Acetylamino-3-O-{[L-1-(D-1-carbamoyl-3-carboxy-propyl)-carbamoylpropyl]-carbamoyl-methyl}-2-desoxy-D-glucose,
2-Acetamino-3-O-{[*L*-1-(D-1-carbamoyl-3-carboxypropyl)-carbamoyl-2-hydroxyäthyl]-carbamoyl-methyl}-2-desoxy-D-glucose,
2-Propionylamino-3-O-{[L-1-(*D*-1,3-dicarboxypropyl)-carbamoyläthyl]-carbamoylmethyl}-2-desoxy-D-glucose,
2-Benzoylamino-3-O-{[*L*-1-(*D*-1-N-carbamoylmethyl-carbamoyl-3-carboxy-propyl)-carbamoyläthyl]-carbamoylmethyl}-2-desoxy-D-glucose,
2-Acetamino-3-O-{[*L*-1-(*D*-1-carbamoyl-3-carboxy-propyl)-carbamoyl-2'-methyl-propyl]-carbamoyl-methyl}-2-desoxy-D-glucose,
2-Acetamino-3-O-{*D*-1-[*L*-1-(*D*-1-carbamoyl-3-(L-1-carboxy-äthyl)-carbamoyl-propyl)-carbamoyläthyl]-carbamoyläthyl}-2-desoxy-D-glucose,
2-Acetamino-3-O-{[L-1-(D-1,3-dicarboxypropyl)-carbamoyl-2'-methyl-propyl]-carbamoylmethyl}-2-desoxy-D-glucose,
2-Benzoylamino-3-O-{[L-1-(D-1-carbamoyl-3-carboxy-propyl-carbamoyl-2'-methyl-propyl]-carbamoyl-methyl}-2-desoxy-D-glucose,
2-Acetamino-3-O-{[L-1-(D-1-carbamoyl-3-carboxy-propyl)-carbamoyl-N,N-tetramethylen]-carbamoyl-methyl}-2-desoxy-D-glucose,
2-Acetamino-3-O-{[L-1-(D-1-carbamoyl-3-carboxy-propyl)-carbamoyläthyl]-N-methyl-carbamoyl-methyl}-2-desoxy-D-glucose,
2-Acetamino-3-O-{D-1-[*L*-1-carbamoyl-3-carboxy-propyl)-carbamoyl-2'-methyl-propyl]-carbamoyl-äthyl}-2-desoxy-D-glucose.

## Beispiel 14

In analoger Weise zu Beispiel 6 erhält man T-Lymphoblasten von CBA/J Mäusen gekuppelt mit
2-Acetamino-3-O-{*D*-1-[*L*-1-(*D*-1-carbamoyl-3-carboxy-propyl)-carbamoyl-äthyl]-carbamoyläthyl}-2-desoxy-D-glucose,
2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,
2-Benzoylamino-3-O-{[*L*-1-(*D*-1-carbamoyl-3-carboxy-propyl)-carbamoyl-äthyl]-carbamoylmethyl}-2-desoxy-D-glucose,
2-Benzoylamino-3-O-{[*L*-1-(D-1,3-dicarboxypropyl)-carbamoyläthyl)-carbamoylmethyl}-2-desoxy-D-glucose,
2-Propionylamino-3-O-{[L-1-(D-1-carbamoyl-3-carboxy-propyl)-carbamoyläthyl]-carbamoylmethyl}-2-desoxy-D-glucose,
2-Acetamino-3-O-{[L-1-(D-1-N-carbamoylmethylcarbamoyl-3-carboxy-propyl)-carbamoyläthyl]-carbamoylmethyl}-2-desoxy-D-glucose,
2-Benzoylamino-3-O-{[L-1-(D-1-carbamoyl-3-carboxy-propyl)-carbamoyl-propyl]-carbamoylmethyl}-2-desoxy-D-glucose,
2-Acetylamino-3-O-{[L-1-(D-1-carbamoyl-3-carboxy-propyl)-carbamoylpropyl]carbamoyl-methyl}-2-desoxy-D-glucose,
2-Acetamino-3-O-{[*L*-1-(D-1-carbamoyl-3-carboxypropyl)-carbamoyl-2-hydroxyäthyl]-carbamoyl-methyl}-2-desoxy-D-glucose,
2-Propionylamino-3-O-{[L-1-(*D*-1,3-dicarboxypropyl)-carbamoyläthyl]-carbamoylmethyl}-2-desoxy-D-glucose,
2-Benzoylamino-3-O-{[*L*-1-(*D*-1-N-carbamoylmethyl-carbamoyl-3-carboxy-propyl)-carbamoyläthyl]-carbamoylmethyl}-2-desoxy-D-glucose,
2-Acetamino-3-O-{[*L*-1-(*D*-1-carbamoyl-3-carboxy-propyl)-carbamoyl-2'-methyl-propyl]-carbamoyl-methyl}-2-desoxy-D-glucose,
2-Acetamino-3-O-{*D*-1-[*L*-1-(*D*-1-carbamoyl-3-(L-1-carboxy-äthyl-carbamoyl-propyl)-carbamoyläthyl]-carbamoyläthyl}-2-desoxy-D-glucose,
2-Acetamino-3-O-{[*L*-1-(D-1,3-dicarboxy-propyl)-carbamoyl-2'-methyl-propyl]-carbamoylmethyl}-2-desoxy-D-glucose,
2-Benzoylamino-3-O-{[L-1-(D-1-carbamoyl-3-carboxy-propyl)-carbamoyl-2'-methyl-propyl]-carbamoyl-methyl}-2-desoxy-D-glucose.
2-Acetamino-3-O-{[L-1-(D-1-carbamoyl-3-carboxy-propyl)-carbamoyl-N,N-tetramethylen]-carbamoyl-methyl}-2-desoxy-D-glucose,
2-Acetamino-3-O-{[L-1-(D-1-carbamoyl-3-carboxy-propyl)-carbamoyl-äthyl]-N-methyl-carbamoyl-methyl}-2-desoxy-D-glucose,
2-Acetamino-3-O-{D-1-[*L*-1-carbamoyl-3-carboxy-propyl)-carbamoyl-2'-methyl-propyl]-carbamoyläthyl}-2-desoxy-D-glucose.

Beispiel 15

1 mg Rinderserumalbumin-2-Acetamino-3-O-{[*L*-1-(*D*-1-carbamoyl-3-carboxy-propyl)-carbamoyl-äthyl]-carbamoyl-methyl}-2-desoxy-D-glucose-Konjugat wird in 0,1 ml Phosphat-gepufferter physiologischer Kochsalzlösung (PBS) gelöst; zu der Lösung werden 0,1 ml einer 5%-igen Suspension von Carboxymethylcellulose in PBS zugemischt. Das Gemisch wird in zwei gleichen Portionen intramuskulär an Meerschweinchen appliziert.

Beispiel 16

$10^8$ T-Lymphoblasten-2-acetamino-3-O-{[*L*-1-(*D*-carbamoyl-3-carboxy-propyl)-carbamoyläthyl]-carbamoyl-methyl}-2-desoxy-D-glucose-Konjugate (siehe Beispiel 6) werden in 0,5 ml Phosphat-gepufferter physiologischer Kochsalzlösung (PBS) suspendiert; zu der Suspension werden 0,5 ml einer 5%-igen Suspension von Carboxymethylcellulose in PBS zugemischt. Pro Maus werden 0,1 ml des Gemisches subcutan appliziert.

Beispiel 17

Zu 10 ml einer Suspension von Tollwut-HDC-Vaccine Behringwerke in Phosphat-gepufferter physiologischer Kochsalzlösung werden 100 mg (0,17 mMol) 2-Acetamino-3-O-{[*L*-1-(D-1-carbamoyl-3-succinimidooxy-carbonyl-propyl)-carbamoyl-äthyl]-carbamoyl-methyl}-2-desoxy-D-glucose zugegeben. Die Viruskonzentration beträgt 2 × $10^7$ $LD_{50}$/Maus in 1 ml Suspension. Die Suspension wird 4 Stunden bei 4°C geschüttelt. Danach wird das Muramyldipeptid-Tollwut-HDC-Vaccine-Konjugat sterilfiltriert, durch Dialyse gegen Wasser von niedermolekularen Reaktionsprodukten abgetrennt und gefriergetrocknet.

Beispiel 18

In analoger Weise zu Beispiel 17 erhält man Tollwut-HDC-Vaccine Behringwerke gekuppelt mit

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*[L*-1-(*D*-1-carbamoyl-3-carboxy-propyl)-carbamoyl-äthyl]-carbamoylmethyl}-2-desoxy-D-glucose,

2-Benzoylamino-3-O-{*[L*-1-(*D*-1,3-dicarboxypropyl)-carbamoyläthyl]-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-dexoxy-D-glucose,

2-Acetamino-3-O-{[L-1-(D-1-N-carbamoylmethylcarbamoyl-3-carboxy-propyl)-carbamoyl-äthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoylmethyl}-2-desoxy-D-glucose,

2-Acetamino-3-O-{[L-1-(D-1-carbamoyl-3-carboxy-propyl)-carbamoylpropyl]-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoylmethyl}-2-desoxy-D-glucose,

2-Propionylamino-3-O-{[L-1-(*D*-1,3-dicarboxypropyl)-carbamoyläthyl]-carbamoylmethyl}-2-desoxy-D-glucose,

2-Benzoylamino-3-O-{[*L*-1-(*D*-1-N-carbamoylmethyl-carbamoyl-3-carboxy-propyl)-carbamoyl-äthyl]-carbamoylmethyl}-2-desoxy-D-glucose,

2-Acetamino-3-O-{[*L*-1-(*D*-1-carbamoyl-3-carboxy-propyl)-carbamoyl-2′-methyl-propyl]-carbamoylmethyl}-2-desoxy-D-glucose,

2-Acetamino-3-O-{*D*-1-[*L*-1-(*D*-1-carbamoyl-3-(L-1-carboxy-äthyl)-carbamoyl-propyl)-carbamoyläthyl]-carbamoyläthyl}-2-desoxy-D-glucose,

2-Acetamino-3-O-{[L-1-(D-1,3-dicarboxy-propyl)-carbamoyl-2′-methyl-propyl]-carbamoylmethyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoyl-methyl}-2-desoxy-D-glucose,

2-Acetamino-3-O-{[L-1-(D-1-carbamoyl-3-carboxy-propyl)-carbamoyl-äthyl]-N-methyl-carbamoyl-methyl}-2-desoxy-D-glucose,

2-Acetamino-3-O-{D-1-[*L*-1-carbamoyl-3-carboxy-propyl)-carbamoyl-2′-methyl-propyl]-carbamoyläthyl}-2-desoxy-D-glucose.

Beispiel 19

Zu 10 ml einer Suspension von extrahierten Influenza-Virus-Antigenen vom Typ A/Victoria/3/75 in Phosphat-gepufferter physiologischer Kochsalzlösung werden 100 mg (0,17 mMol) 2-Acetamino-3-O-{[*L*-1-(D-1-carbamoyl-3-succinimidooxycarbonyl-propyl)-carbamoyl-äthyl]-carbamoyl-methyl}-2-desoxy-D-glucose zugegeben. (Die Virus-Antigene werden nach dem Tween [Polyoxyäthylensorbitanmonostearat, -palmitat oder -oleat]/Äther-Spaltverfahren der Behringwerke, Marburg, BRD — analog zur Herstellung von ®Begrivac S — gewonnen; die Konzentration beträgt 4000 I.E. in 1 ml Suspension). Die Suspension wird 4 Stunden bei 4°C geschüttelt. Danach werden die Virusantigen-Muramyl-dipeptid-Konjugate durch Dialyse gegen Phosphat-gepufferte physiologische Kochsalzlösung von niedermolekularen Reaktionsprodukten abgetrennt; die dialysierte Suspension wird eingefroren und bis zum Gebrauch bei −20°C gelagert.

Beispiel 20

In analoger Weise zu Beispiel 20 erhält man Influenza-Virus-Antigen gekuppelt an

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{*L*-1-(D-1,3-dicarboxy-propyl)-carbamoyläthyl-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyl-äthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoylmethyl}-2-desoxy-D-glucose,

2-Acetylamino-3-O-{[L-1-(D-1-carbamoyl-3-carboxy-propyl)-carbamoylpropyl]-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoyl-methyl}-2-desoxy-D-glucose,

2-Propionylamino-3-O-{[L-1-(*D*-1,3-dicarboxy-propyl)-carbamoyläthyl]-carbamoylmethyl}-2-desoxy-D-glucose,

2-Benzoylamino-3-O-{[*L*-1-(*D*-1-N-carbamoyl-methyl-carbamoyl-3-carboxy-propyl)-carbamoyl-äthyl]-carbamolymethyl}-2-desoxy-D-glucose,

2-Acetamino-3-O-{[*L*-1-(*D*-1-carbamoyl-3-carboxy-propyl)-carbamoyl-2′-methyl-propyl]-carbamoyl-methyl}-2-desoxy-D-glucose,

2-Acetamino-3-O-{*D*-1-[*L*-1-(*D*-1-carbamoyl-3-(L-1-carboxyäthyl)-carbamoyl-propyl)-carbamoyläthyl]-carbamoyläthyl}-2-desoxy-D-glucose,

2-Acetamino-3-O-{[L-1-(D-1,3-dicarboxy-propyl)-carbamoyl-2′-methyl-propyl-carbamoyl-methyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoyl-methyl}-2-desoxy-D-glucose,

2-Acetamino-3-O-{[L-1-(D-1-carbamoyl-3-carboxy-propyl)-carbamoyl-äthyl]-N-Methyl-carbamoyl-methyl}-2-desoxy-D-glucose,

2-Acetamino-3-O-{D-1-[*L*-1-carbamoyl-3-carboxy-propyl)-carbamoyl-2′-methyl-propyl]-carbamoyl-äthyl}-2-desoxy-D-glucose.

Beispiel 21

Zu 2 ml einer Lösung von Tetanustoxoid in Phosphat-gepufferter physiologischer Kochsalzlösung werden 10 mg 2-Acetamino-3-O-{[*L*-1-(*D*-1-carbamoyl-3-succinimidoxy-carbonyl-propyl)-carbamoyl-äthyl]-carbamoyl-methyl}-2-desoxy-D-glucose zugegeben. Die Toxoidkonzentration beträgt 3 mg/ml. Die Lösung wird 4 Stunden bei 4°C gerührt. Danach wird das Tetanustoxoid-Muramyldipeptid-Konjugat durch Ultrafiltration von niedermolekularen Reaktionsprodukten abgetrennt; die ultrafiltrierte Lösung wird eingefroren und bis zum Gebrauch bei −20°C gelagert. Die quantitative Bestimmung (Morgan-Elson-Reaktion) ergibt ca. 50 µg Muramyldipeptid pro 1 mg Tetanustoxoid-Muramyldipeptid-Konjugat.

Beispiel 22

In analoger Weise zu Beispiel 21 enthält man Tetanustoxoid gekuppelt an

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{[*L*-1-(D-1,3-dicarboxy-propyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyl-äthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoyl-methyl}-2-desoxy-D-glucose,

2-Acetylamino-3-O-{[L-1-(D-1-carbamoyl-3-carboxy-propyl)-carbamoylpropyl)-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoyl-methyl}-2-desoxy-D-glucose,

2-Propionylamino-3-O-{[L-1-(*D*-1,3-dicarboxy-propyl)-carbamoyläthyl]-carbamoylmethyl}-2-desoxy-D-glucose,

2-Benzoylamino-3-O-{[*L*-1-(*D*-1-N-carbamoyl-methyl-carbamoyl-3-carboxy-propyl)-carbamoyläthyl]-carbamoylmethyl}-2-desoxy-D-glucose,

2-Acetamino-3-O-{[*L*-1-(*D*-1-carbamoyl-3-carboxy-propyl)-carbamoyl-2′-methyl-propyl]-carbamoyl-methyl}-2-desoxy-D-glucose,

2-Acetamino-3-O-{*D*-1-[*L*-1-(*D*-1-carbamoyl-3-(L-1-carboxyäthyl)-carbamoyl-propyl)-carbamoyläthyl]-carbamoyläthyl}-2-desoxy-D-glucose,

2-Acetamino-3-O-{[L-1-(D-1,3-dicarboxypropyl)-carbamoyl-2′-methyl-propyl]-carbamoylmethyl}-2-desoxy-D-glucose,

2-Benzoylamino-3-O-{[L-1-(D-1-carbamoyl-3-carboxy-propyl)-carbamoyl-2′-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoyl-methyl}-2-desoxy-D-glucose,

2-Acetamino-3-O-{[L-1-(D-1-carbamoyl-3-carboxy-propyl)-carbamoyl-äthyl]-N-methyl-carbamoyl-methyl}-2-desoxy-D-glucose,

2-Acetamino-3-O-{D-1-[*L*-1-carbamoyl-3-carboxy-propyl)-carbamoyl-2′-methyl-propyl]-carbamoyl-äthyl}-2-desoxy-D-glucose.

## Beispiel 23

Zu 10 ml einer Lösung von Choleratoxid aus Vibrio cholerae in Phosphat-gepufferter physiologischer Kochsalzlösung werden 50 mg 2-Acetamido-3-O-{[*L*-1-(*D*-1-carbamoyl-3-succinimidooxycarbonyl-propyl)-carbamoyl-äthyl]-carbamoyl-methyl}-2-desoxy-D-glucose zugegeben. Die Proteinkonzentration in der Lösung beträgt 0,6 mg/ml. Die Lösung wird 4 Stunden bei 4°C gerührt. Danach wird das Choleratoxoid-Muramyldipeptid-Konjugat durch Ultrafiltration von niedermolekularen Reaktionsprodukten abgetrennt; die ultrafiltrierte Lösung wird eingefroren und bis zum Gebrauch bei −20°C gelagert. Die quantitative Bestimmung (Morgan-Elson-Reaktion) ergibt 40—50 µg Muramyldipeptid pro 1 mg Choleratoxoid-Muramyldipeptid-Konjugat.

## Beispiel 24

In analoger Weise zu Beispiel 23 erhält man Choleratoxoid gekuppelt an

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{[*L*-1-(D-1,3-dicarboxy-propyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyl-äthyl]-carbamoylmethyl}-2-desoxy-D-glucose,

2-Benzoylamino-3-O-{[L-1-(D-1-carbamoyl-3-carboxy-propyl)-carbamoyl-propyl]-carbamoyl-methyl}-2-desoxy-D-glucose,

2-Acetylamino-3-O-{[L-1-(D-1-carbamoyl-3-carboxy-propyl)-carbamoylpropyl]-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoyl-methyl}-2-desoxy-D-glucose,

2-Propionylamino-3-O-{[L-1-(*D*-1,3-dicarboxy-propyl)-carbamoyläthyl]-carbamoylmethyl}-2-desoxy-D-glucose,

2-Benzoylamino-3-O-{[*L*-1-(*D*-1-N-carbamoyl-methyl-carbamoyl-3-carboxy-propyl)-carbamoyläthyl]-carbamoylmethyl}-2-desoxy-D-glucose,

2-Acetamino-3-O-{[*L*-1-(*D*-1-carbamoyl-3-carboxy-propyl)-carbamoyl-2′-methyl-propyl]-carbamoyl-methyl}-2-desoxy-D-glucose,

2-Acetamino-3-O-{*D*-1-[*L*-1-(*D*-1-carbamoyl-3-(L-1-carboxy-äthyl)-carbamoyl-propyl)-carbamoyläthyl]-carbamoyläthyl}-2-desoxy-D-glucose,

2-Acetamino-3-O-{[L-1-(D-1,3-dicarboxypropyl)-carbamoyl-2'-methyl-propyl]-carbamoylmethyl}-2-desoxy-D-glucose,

2-Benzoylamino-3-O-{[L-1-(D-1-carbamoyl-3-carboxy-propyl)-carbamoyl-2'-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoyl-methyl}-2-desoxy-D-glucose,

2-Acetamino-3-O-{[L-1-(D-1-carbamoyl-3-carboxy-propyl)-carbamoyl-äthyl]-N-methyl-carbamoyl-methyl}-2-desoxy-D-glucose,

2-Acetamino-3-O-{D-1-[*L*-1-carbamoyl-3-carboxy-propyl)-carbamoyl-2'-methyl-propyl]-carbamoyl-äthyl}-2-desoxy-D-glucose.

Beispiel 25

20 mg eines synthetischen Eicosapeptids, welches mit der C-terminalen Sequenz des humanen Choriongonadotropins (HCG) identisch ist [Referenz: C. H. Schneider, K. Blaser, Ch.Pfeuti and E. Grunden, Febs Letters, *50*, 272 (1975)] und 30 mg 2-Acetamino-3-O-{[*L*-1-(*D*-1-carbamoyl-3-succinimidooxy-carbonyl-propyl)-carbamoyl-äthyl]-carbamoyl-methyl}-2-desoxy-D-glucose werden in 2 ml Natriumphosphat-Pufferlösung, pH 7,2, gelöst. Die Lösung wird 6 Stunden bei Raumtemperatur gerührt. Danach wird das Eicosapeptid-Muramyldipeptid-Konjugat durch Chromatographie an einer Sephadex G-25 Säule (1,4 × 40 cm) mit Wasser als Eluant isoliert und gefriergetrocknet. Die quantitative Aminosäureanalyse zeigt, dass 1-Muramyldipeptid-Molekül an 1-Eicosapeptid-Molekül gebunden ist.

Beispiel 26

In analoger Weise zu Beispiel 25 erhält man das C-terminale Eicosapeptid-Fragment des humanen Choriongonadotropins gekuppelt an

2-Acetamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{*D*-1-[*L*-1-(*D*-1-carbamoyl-3-carboxy-propyl)-carbamoyl-äthyl]-carbamoyl-äthyl}-2-desoxy-D-glucose,

2-Benzoylamino-3-O-{[*L*-1-(*D*-1-carbamoyl-3-carboxy-propyl)-carbamoyl-äthyl]-carbamoylmethyl}-2-desoxy-D-glucose,

2-Benzoylamino-3-O-{*L*-1-(D-1,3-dicarboxy-propyl)-carbamoyläthyl)-carbamoylmethyl}-2-desoxy-D-glucose,

2-Propionylamino-3-O-{[L-1-(D-1-carbamoyl-3-carboxy-propyl)-carbamoyläthyl]-carbamoylmethyl}-2-desoxy-D-glucose,

2-Acetamino-3-O-{[L-1-(D-1-N-carbamoylmethylcarbamoyl-3-carboxy-propyl)-carbamoyläthyl]-carbamoylmethyl}-2-desoxy-D-glucose,

2-Benzoylamino-3-O-{[L-1-(D-1-carbamoyl-3-carboxy-propyl)-carbamoyl-methyl}-2-desoxy-D-glucose,

2-Acetylamino-3-O-{[L-1-(D-1-carbamoyl-3-carboxy-propyl)-carbamoylpropyl]-carbamoyl-methyl}-2-desoxy-D-glucose,

2-Acetamino-3-O-{[*L*-1-(D-1-carbamoyl-3-carboxypropyl)-carbamoyl-2-hydroxyäthyl]-carbamoyl-methyl}-2-desoxy-D-glucose,

2-Propionylamino-3-O-{[L-1-(*D*-1,3-dicarboxy-propyl)-carbamoyläthyl]-carbamoylmethyl}-2-desoxy-D-glucose,

2-Benzoylamino-3-O-{[*L*-1-(*D*-1-N-carbamoyl-methyl-carbamoyl-3-carboxy-propyl)-carbamoyläthyl]-carbamoylmethyl}-2-desoxy-D-glucose,

2-Acetamino-3-O-{[*L*-1-(*D*-1-carbamoyl-3-carboxy-propyl)-carbamoyl-2'-methyl-propyl]-carbamoyl-methyl}-2-desoxy-D-glucose,

2-Acetamino-3-O-{*D*-1-[*L*-1-(*D*-1-carbamoyl-3-(L-1-carboxy-äthyl)-carbamoyl-propyl)-carbamoyläthyl]-carbamoyläthyl}-2-desoxy-D-glucose,

2-Acetamino-3-O-{[L-1-(D-1,3-dicarboxypropyl)-carbamoyl-2'-methyl-propyl]-carbamoylmethyl}-2-desoxy-D-glucose,

2-Benzoylamino-3-O-{[L-1-(D-1-carbamoyl-3-carboxy-propyl)-carbamoyl-2'-methyl-propyl]-carbamoyl-methyl}-2-desoxy-D-glucose,

2-Acetamino-3-O-{[L-1-(D-1-carbamoyl-3-carboxy-propyl)-carbamoyl-N,N-tetramethylen]-carbamoyl-methyl}-2-desoxy-D-glucose,

2-Acetamino-3-O-{[L-1-(D-1-carbamoyl-3-carboxy-propyl)-carbamoyl-äthyl]-N-methyl-carbamoyl-methyl}-2-desoxy-D-glucose,

2-Acetamino-3-O-{D-1-[*L*-1-carbamoyl-3-carboxy-propyl)-carbamoyl-2'-methyl-propyl]-carbamoyl-äthyl}-2-desoxy-D-glucose.

Beispiel 27

In analoger Weise zu Beispiel 1 erhält man Rinderserumalbumin gekuppelt mit

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyläthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilylcarboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-(D-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,
2-Glykolylamino-3-O-{[L-1-(D-1-carbamoyl-3-carboxy-propyl)-carbamoyl-äthyl-carbamoyl-methyl}-2-desoxy-D-glucose,
2-(N-Methyl-acetamino)-3-O-{[L-1-(D-1-carbamoyl-3-carboxy-propyl)-carbamoyl-äthyl]-carbamoyl-methyl}-2-desoxy-D-glucose,
2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,
2-Acetamino-3-O-{D-1-[L-1-(D-1-carbamoyl-3-carboxy-propyl)-carbamoyl-2′-phenyläthyl]-carbamoyl-äthyl}-2-desoxy-D-glucose,
2-Acetamino-3-O-{[L-1-(D-1-carbamoyl-3-carboxy-propyl)-carbamoyl-2′-(p-hydroxyphenyl)-äthyl]-carbamoyl-methyl}-2-desoxy-D-glucose,
2-Acetamino-3-O-{[L-1-(D-1-[L-1-carboxy-äthyl]-carbamoyl-3-benzylcarboxy-propyl)-carbamoyl-äthyl-carbamoylmethyl}-2-desoxy-D-glucose,
2-Acetamino-3-O-{[L-1-(D-1,3-di-carboxy-propyl)-N-methyl-carbamoyl-äthyl]-carbamoyl-methyl}-2-desoxy-D-glucose.

Beispiel 28

In analoger Weise zu Beispiel 3 erhält man Schaferythrocytenmembranen gekuppelt an
2-Acetamino-3-O-{[(D-1-carbamoyl-3-carboxy-propyl)-carbamoyl-methyl]-N-methyl-carbamoyl-methyl}-2-desoxy-D-glucose,
2-Benzoylamino-3-O-{[D-1-carbamoyl-3-carboxy-propyl)-carbamoyl-methyl]-N-methyl-carbamoyl-methyl}-2-desoxy-D-glucose,
2-Acetamino-3-O-{[L-1-(D-1-carbamoyl-3-carboxy-propyl)-carbamoyl-äthyl)-N-äthyl-carbamoyl-methyl}-2-desoxy-D-glucose,
2-Acetamino-3-O-{[L-1-(D-1-carbamoyl-3-carboxy-propyl)-carbamoyl-propyl]-N-methyl-carbamoyl-methyl}-2-desoxy-D-glucose,
2-Acetamino-3-O-{[L-1-(D-1-carbamoyl-3-carboxy-propyl)-carbamoyl-propyl]-N-äthyl-carbamoyl-methyl}-2-desoxy-D-glucose,
2-Benzamido-3-O-{[L-1-(D-1-carbamoyl-3-carboxy-propyl)-carbamoyl-propyl]-N-äthyl-carbamoyl-methyl}-2-desoxy-D-glucose,
2-Acetamino-3-O-{[L-1-(D-1-carbamoyl-3-carboxy-propyl)-carbamoyl-äthyl]-N-propyl-carbamoyl-methyl}-2-desoxy-D-glucose,
2-Acetamino-3-O-{[L-1-(D-1-carbamoyl-3-carboxy-propyl)-carbamoyl-N,N-pentamethylen]-carbamoyl-methyl}-2-desoxy-D-glucose,
2-Benzoylamino-3-O-{[L-1-(D-1-carbamoyl-3-carboxy-propyl)-carbamoyl-N,N-pentamethylen]-carbamoyl-methyl}-2-desoxy-D-glucose,
2-Acetamino-3-O-{[L-1-(D-1-carbamoyl-3-carboxy-propyl)-N-methyl-carbamoyl-äthyl]-carbamoyl-methyl}-2-desoxy-D-glucose,
2-Acetamino-3-O-{D-1-[(D-1-carbamoyl-3-carboxy-propyl)-carbamoyl-methyl]-N-methyl-carbamoyl-äthyl}-2-desoxy-D-glucose,
2-Benzoylamino-3-O-{D-1-[(D-1-carbamoyl-3-carboxy-propyl)-carbamoyl-methyl]-N-methyl-carbamoyl-äthyl}-2-desoxy-D-glucose,
2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,
2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,
2-Acetamino-3-O-{D-1-[L-1-(D-1-carbamoyl-3-carboxy-propyl)-carbamoyl-propyl-N-äthyl-carbamoyl-methyl}-2-desoxy-D-glucose,
2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,
2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,
2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,
2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,
2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,
2-Acetamino-3-O-{[L-1-(D-1-carbamoyl-3-carboxy-methyl-phenyl]-carbamoyl-methyl}-2-desoxy-D-glucose,
2-Benzoylamino-3-O-{[L-1-(D-1-carbamoyl-3-carboxy-methyl-phenyl]-carbamoyl-methyl}-2-desoxy-D-glucose,
2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyl-äthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilylcarboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1--carbamoyl-3-carboxy-propyl)-carbamoyl-2′-phenyläthyl]-carbamoyl-äthyl}-2-desoxy-D-glucose,

2-Acetamino-3-O-{[L-1-(D-1-carbamoyl-3-carboxy-propyl)-carbamoyl-2′-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose.

## Beispiel 29

In analoger Weise zu Beispiel 4 erhält man Gruppe C Polysaccharid von Neisseria meningitidis gekuppelt mit

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamid-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-[(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyl-äthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-(D-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1--carbamoyl-3-carboxy-propyl)-carbamoyl-2′-phenyläthyl]-carbamoyl-äthyl}-2-desoxy-D-glucose,

2-Acetamino-3-O-{[L-1-(D-1-carbamoyl-3-carboxy-propyl)-carbamoyl-2′-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose.

Beispiel 30

In analoger Weise zu Beispiel 5 erhält man Merozoiten des Malariaerregers Plasmodium knowlesi gekuppelt an

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-[(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyl-äthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1--carbamoyl-3-carboxy-propyl)-carbamoyl-2′-phenyläthyl]-carbamoyl-äthyl}-2-desoxy-D-glucose,

2-Acetamino-3-O-{[L-1-(D-1-carbamoyl-3-carboxy-propyl)-carbamoyl-2′-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose.

Beispiel 31

In analoger Weise zu Beispiel 6 erhält man T-Lymphoblasten von CBA/J-Mäusen gekuppelt an

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxypropyl)-carbamoyl-N,N-pentamethylen]-carbamoyl-äthyl}-2-desoxy-D-glucose,

2-Benzoylamino-3-O-{D-1-[L-1-(D-1-carbamoyl-3-carboxypropyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyl-äthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1--carbamoyl-3-carboxy-propyl)-carbamoyl-2′-phenyläthyl]-carbamoyl-äthyl}-2-desoxy-D-glucose,

2-Acetamino-3-O-{[L-1-(D-1-carbamoyl-3-carboxy-propyl)-carbamoyl-2′-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose.

Beispiel 32

In analoger Weise zu Beispiel 12 erhält man Maul- und Klauenseuche-Kultur Vakzine Beringwerke gekuppelt an

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,
2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,
2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,
2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,
2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,
2-Acetamino-3-O-{D-1-[L-1-(D-1-carbamoyl-3-carboxypropyl)-carbamoyl-N,N-pentamethylen]-carbamoyl-äthyl}-2-desoxy-D-glucose,
2-Benzoylamino-3-O-{D-1-[L-1-(D-1-carbamoyl-3-carboxypropyl)-carbamoyl-N,N-pentamethylen]-carbamoyl-äthyl}-2-desoxy-D-glucose,
2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,
2-Acetamino-3-O-{[L-1-(D-1-carbamoyl-3-carboxy-methyl-phenyl]-carbamoyl-methyl}-2-desoxy-D-glucose,
2-Benzoylamino-3-O-{[L-1-(D-1-carbamoyl-3-carboxy-methyl-phenyl]-carbamoyl-methyl}-2-desoxy-D-glucose,
2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,
2-Benzamino-3-O-{[L-1-(D-1-carbamoyl-3-carboxy-propyl)-carbamoyl-2-chloräthyl]-carbamoyl-methyl}-2-desoxy-D-glucose,
2-Acetamino-3-O-{D-1-[L-1-(D-1-carbamoyl-3,3-dicarboxy-propyl)-carbamoyl-äthyl]-carbamoyl-äthyl}-2-desoxy-D-glucose,
2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,
2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,
2-Benzamino-3-O-{[L-1-(D-1-carbamoyl-3-trimethylsilylcarboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),
2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,
2-Acetamino-3-O-{D-1-[L-1-(D-1-carbamoyl-3-carboxy-propyl)-carbamoyl-propyl]-carbamoyl-äthyl}-2-desoxy-D-glucose,
2-Acetamino-3-O-{D-1-[L-1-carbamoyl-3-carboxy-propyl)-carbamoyl-2'-hydroxy-propyl]-carbamoyl-äthyl}-2-desoxy-D-glucose,
2-Acetamino-3-O-{D-1-[L-1-(D-1-carbamoyl-3-carboxy-propyl)-carbamoyl-2'-(p-hydroxy-phenyl)-äthyl]-carbamoyl-äthyl}-2-desoxy-D-glucose,
2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,
2-Glykolylamino-3-O-{D-1-[L-1-(D-1-carbamoyl-3-carboxy-propyl)-carbamoyl-äthyl]-carbamoyl-äthyl}-2-desoxy-D-glucose,
2-Glykolylamino-3-O-{[L-1-(D-1-carbamoyl-3-carboxy-propyl)-carbamoyl-äthyl-carbamoyl-methyl}-2-desoxy-D-glucose,
2-(N-Methyl-acetamino)-3-O-{[L-1-(D-1-carbamoyl-3-carboxy-propyl)-carbamoyl-äthyl]-carbamoyl-methyl}-2-desoxy-D-glucose,
2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,
2-Acetamino-3-O-{D-1-[L-1-(D-1--carbamoyl-3-carboxy-propyl)-carbamoyl-2'-phenyläthyl]-carbamoyl-äthyl}-2-desoxy-D-glucose,
2-Acetamino-3-O-{[L-1-(D-1-carbamoyl-3-carboxy-propyl)-carbamoyl-2'-(p-hydroxyphenyl)-äthyl]-carbamoyl-methyl}-2-desoxy-D-glucose,
2-Acetamino-3-O-{[L-1-(D-1-[L-1-carboxy-äthyl]-carbamoyl-3-benzylcarboxy-propyl)-carbamoyl-äthyl-carbamoylmethyl}-2-desoxy-D-glucose,
2-Acetamino-3-O-{[L-1-(D-1,3-di-carboxy-propyl)-N-methyl-carbamoyl-äthyl]-carbamoyl-methyl}-2-desoxy-D-glucose.

## Beispiel 33

In analoger Weise zu Beispiel 17 erhält man Tollwut-HDC-Vakzine Behringwerke gekuppelt an
2-Acetamino-3-O-{[(D-1-carbamoyl-3-carboxy-propyl)-carbamoyl-methyl]-N-methyl-carbamoyl-methyl}-2-desoxy-D-glucose,
2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-[(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl)}-2-desoxy-D-glucose,

2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-propyl)-carbamoyl-2-methyl-mercapto-äthyl}-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyläthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silcarboxy-propyl)-carbamoyl-äthyl]-carbamoylmethyl}-1,4,5-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-(D-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxyphenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]carbamoyl-äthyl}-2-desoxy-D-glucose,

2-Glykolylamino-3-O-{D-1-[L-1-(D-1-carbamoyl-3-carboxy-propyl]-carbamoyl-äthyl]-carbamoyl-äthyl}-2-desoxy-D-glucose,

2-Glyklolylamino-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1-carbamoyl-3-carboxy-propyl)-carbamoyl-2'-phenyläthyl]-carbamoyl-äthyl}-2-desoxy-D-glucose,

2-Acetamino-3-O-{[L-1-(D-1-carbamoyl-3-carboxy-propyl)-carbamoyl-2'-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl]-carbamoyl-methyl}-2-desoxy-D-glucose,

2-Acetamino-3-O-{[L-1-(D-1,3-di-carboxypropyl)-N-methyl-carbamoyl-äthyl]-carbamoylmethyl}-2-desoxy-D-glucose.

Beispiel 34

In analoger Weise zu Beispiel 18 erhält man Influenza-Virus-Antigene vom Typ A/Victoria/3/75 gekuppelt an

2-Acetamino-3-O-{[D-1-carbamoyl-3-carboxypropyl)-carbamoyl-methyl]-N-methyl-carbamoyl-methyl}-2-desoxy-D-glucose,

2-Benzoylamino-3-O-{[D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-[(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyläthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-(D-1-carbamoyl-3-carboxy-propyl)-carbamoyl-2′-hydroxy-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxyphenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1-carbamoyl-3-carboxy-propyl)-carbamoyl-2′-phenyläthyl]-carbamoyl-äthyl}-2-desoxy-D-glucose,

2-Acetamino-3-O-{[L-1-(D-1-carbamoyl-3-carboxy-propyl)-carbamoyl-2′-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl] carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose,

Beispiel 35

In analoger Weise zu Beispiel 21 erhält man Tetanustoxoid gekuppelt an

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-gluocose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamino-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{L-1-[(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-D-1-carbamoyl-3-carboxy-propyl)-carbamoyl-äthyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxy-propyl)-carbamoyl-N,N,-pentamethylen]-carbamoyl-äthyl}-2-desoxy-D-glucose,

2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl]-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyl-äthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl]-carbamoyl-methyl}-2-desoxy-D-glucose,

2-(β-Carbomethoxy-succinamido)-3-O-{D-1-[L-1-(D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-(D-1-carbamoyl-3-carboxy-propyl)-carbamoyl-2'-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2'-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino]-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1-carbamoyl-3-carboxy-propyl)-carbamoyl-2'-phenyläthyl]-carbamoyl-äthyl}-2-desoxy-D-glucose,

2-Acetamino-3-O-{[L-1-(D-1-carbamoyl-3-carboxy-propyl)-carbamoyl-2'-(p-hydroxyphenyl)-äthyl)-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl]-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose,

Beispiel 36

In analoger Weise zu Beispiel 23 erhält man Choleratoxoid aus Vibriocholerae gekuppelt an

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[(D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,
2-Acetamino-3-O-{[L-1-(D-1-carbamoyl-3-carboxy-propyl)-carbamoyl-äthyl]-N-propyl-carbamoyl-methyl}-2-desoxy-D-glucose,
2-Acetamino-3-O-{[L-1-(D-1-carbamoyl-3-carboxy-propyl)-carbamoyl-N,N-pentamethylen]-carbamoyl-methyl}-2-desoxy-D-glucose,
2-Benzoylamino-3-O-{[L-1-(D-1-carbamoyl-3-carboxy-propyl)-carbamoyl-N,N-pentamethylen]-carbamoyl-methyl}-2-desoxy-D-glucose,
2-Acetamino-3-O-{[L-1-(D-1-carbamoyl-3-carboxy-propyl)-N-methyl-carbamoyl-äthyl]-carbamoyl-methyl}-2-desoxy-D-glucose,
2-Acetamino-3-O-{D-1-[(D-1-carbamoyl-3-carboxy-propyl)-carbamoyl-methyl]-N-methyl-carbamoyl-äthyl}-2-desoxy-D-glucose,
2-Benzoylamino-3-O-{D-1-[(D-1-carbamoyl-3-carboxy-propyl)-carbamoyl-methyl]-N-methylcarbamoyl-äthyl}-2-desoxy-D-glucose,
2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,
2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,
2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,
2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,
2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,
2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,
2-Benzoylamino-3-O-{D-1-[L-1-(D-1-carbamoyl-3-carboxy-propyl)-carbamoyl-N,N-pentamethylen]-carbamoyl-äthyl}-2-desoxy-D-glucose,
2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,
2-Acetamino-3-O-{[L-1-(D-1-carbamoyl-3-carboxy-methyl-phenyl]-carbamoyl-methyl}-2-desoxy-D-glucose,
2-Benzoylamino-3-O-{[L-1-(D-1-carbamoyl-3-carboxy-methyl-phenyl]-carbamoyl-methyl}-2-desoxy-D-glucose,
2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,
2-Benzamino-3-O-{[L-1-(D-1-carbamoyl-3-carboxy-propyl)-carbamoyl-2-chloräthyl]-carbamoyl-methyl}-2-desoxy-D-glucose,
2-Acetamino-3-O-{D-1-[L-1-(D-1-carbamoyl-3,3-dicarboxy-propyl)-carbamoyläthyl]-carbamoyl-äthyl}-2-desoxy-D-glucose,
2-(β-Carbomethoxy-succinamido)-3-O-{[L-1-(D-1-carbamoyl-3-carboxy-propyl)-carbamoyl-äthyl-carbamoyl-methyl}-2-desoxy-D-glucose,
2-(β-Carbomethoxy-succinamido)-3-O{D-1-[L-1-(D-1-carbamoyl-3-carboxy-propyl)-carbamoyl-äthyl]-carbamoyl-äthyl}-2-desoxy-D-glucose,
2-Benzamino-3-O-{[L-1-(D-1-carbamoyl-3-trimethylsilylcarboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),
2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,
2-Acetamino-3-O-{D-1-(L-1-(D-1-carbamoyl-3-carboxy-propyl)-carbamoyl-propyl]-carbamoyl-äthyl}-2-desoxy-D-glucose,
2-Acetamino-3-O-{D-1-[L-1-(D-1-carbamoyl-3-carboxy-propyl)-carbamoyl-2′-hydroxy-propyl-carbamoyl-äthyl}-2-desoxy-D-glucose,
2-Acetamino-3-O-{D-1-(L-1-(D-1-carbamoyl-3-carboxy-propyl)-carbamoyl-2′-(p-hydroxyphenyl)-äthyl]-carbamoyl-äthyl}-2-desoxy-D-glucose,
2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,
2-Glykolylamino-3-O-{D-1-[L-1-(D-1-carbamoyl-3-carboxy-propyl)-carbamoyl-äthyl]-carbamoyl-äthyl}-2-desoxy-D-glucose,
2-Glykolylamino-3-O-{[L-1-(D-1-carbamoyl-3-carboxy-propyl)-carbamoyl-äthyl]-carbamoyl-methyl}-2-desoxy-D-glucose,
2-(N-Methyl-acetamino)-3-O-{[L-1-(D-1-carbamoyl-3-carboxy-propyl)-carbamoyl-äthyl]-carbamoyl-methyl}-2-desoxy-D-glucose,
2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1-carbamoyl-3-carboxy-propyl)-carbamoyl-2'-phenyläthyl]-carbamoyl-äthyl}-2-desoxy-D-glucose,

2-Acetamino-3-O-{[L-1-(D-1-carbamoyl-3-carboxy-propyl)-carbamoyl-2'-(p-hydroxyphenyl)-äthyl]-carbamoyl-methyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl]-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose,

Beispiel 37

In analoger Weise zu Beispiel 25 erhält man ein synthetisches Eicosapeptid, das mit der C-terminalen Sequenz des humanen Choriongonadotropin identisch ist, gekuppelt an

2-Acetamino-3-O-{[(D-1-carbamoyl-3-carboxy-propyl)-carbamoyl-methyl]-N-methyl-carbamoyl-methyl}-2-desoxy-D-glucose,

2-Benzoylamino-3-O-{[D-1-carbamoyl-3-carboxy-propyl)-carbamoyl-methyl]-N-methyl-carbamoyl-methyl}-2-desoxy-D-glucose,

2-Acetamino-3-O-{[L-1-(D-1-carbamoyl-3-carboxy-propyl)-carbamoyl-äthyl)-N-äthyl-carbamoyl-methyl}-2-desoxy-D-glucose,

2-Acetamino-3-O-{[L-1-(D-1-carbamoyl-3-carboxy-propyl)-carbamoyl-propyl]-N-methyl-carbamoyl-methyl}-2-desoxy-D-glucose,

2-Acetamino-3-O-{[L-1-(D-1-carbamoyl-3-carboxy-propyl)-carbamoyl-propyl]-N-äthyl-carbamoyl-methyl}-2-desoxy-D-glucose,

2-Benzamido-3-O-{[L-1-(D-1-carbamoyl-3-carboxy-propyl)-carbamoyl-propyl]-N-äthyl-carbamoyl-methyl}-2-desoxy-D-glucose,

2-Acetamino-3-O-{[L-1-(D-1-carbamoyl-3-carboxy-propyl)-carbamoyl-äthyl]-N-propyl-carbamoyl-methyl}-2-desoxy-D-glucose,

2-Acetamino-3-O-{[L-1-(D-1-carbamoyl-3-carboxy-propyl)-carbamoyl-N,N-pentamethylen]-carbamoyl-methyl}-2-desoxy-D-glucose,

2-Benzoylamino-3-O-{[L-1-(D-1-carbamoyl-3-carboxy-propyl)-carbamoyl-N,N-pentamethylen]-carbamoyl-methyl}-2-desoxy-D-glucose,

2-Acetamino-3-O-{[L-1-(D-1-carbamoyl-3-carboxy-propyl)-N-methyl-carbamoyl-äthyl]-carbamoyl-methyl}-2-desoxy-D-glucose,

2-Acetamino-3-O-{D-1-[(D-1-carbamoyl-3-carboxy-propyl)-carbamoyl-methyl]-N-methyl-carbamoyl-äthyl}-2-desoxy-D-glucose,

2-Benzoylamino-3-O-{D-1-[(D-1-carbamoyl-3-carboxy-propyl)-carbamoyl-methyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-äthyl)-N-äthyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-methyl-carbamoyl-äthyl}-2-desoxy-D-glucose,

2-Acetamino-3-O-{D-1-[L-1-(D-1-carbamoyl-3-carboxy-propyl)-carbamoyl-propyl]-N-äthyl-carbamoyl-methyl}-2-desoxy-D-glucose,

2-Benzamido-3-O-{D-1-[L-1-(D-1-carbamoyl-3-carboxy-propyl)-carbamoyl-propyl]-N-äthyl-carbamoyl-äthyl}-2-desoxy-D-glucose,

2-Acetamino-3-O-{D-1-[L-1-(D-1-carbamoyl-3-carboxy-propyl)-carbamoyl-äthyl]-N-propyl-carbamoyl-äthyl}-2-desoxy-D-glucose,

2-Acetamino-3-O-{D-1-[L-1-(D-1-carbamoyl-3-carboxy-propyl)-carbamoyl-N,N-pentamethylen]-carbamoyl-äthyl}-2-desoxy-D-glucose,

2-Benzoylamino-3-O-{D-1-(L-1-(D-1-carbamoyl-3-carboxy-propyl)-carbamoyl-N,N-pentamethylen]-carbamoyl-äthyl}-2-desoxy-D-glucose,

2-Acetamino-3-O-{D-1-[L-1-(D-1-carbamoyl-3-carboxy-propyl)-N-methyl-carbamoyl-äthyl]-carbamoyl-äthyl}-2-desoxy-D-glucose,

2-Acetamino-3-O-{[L-1-(D-1-carbamoyl-3-carboxy-methyl-phenyl)-carbamoyl-methyl}-2-desoxy-D-glucose,

2-Benzoylamino-3-O-{[L-1-(D-1-carbamoyl-3-carboxy-methyl-phenyl]-carbamoyl-methyl}-2-desoxy-D-glucose,

2-Benzamino-3-O-{[L-1-(D-1-carbamoyl-3-carboxy-propyl)-carbamoyl-2-methyl-mercapto-äthyl]-carbamoyl-methyl}-2-desoxy-D-glucose,

2-Benzamino-3-O-{[L-1-(D-1-carbamoyl-3-carboxy-propyl)-carbamoyl-2-chloräthyl]-carbamoyl-methyl}-2-desoxy-D-glucose,

2-Acetamino-3-O-{D-1-[L-1-(D-1-carbamoyl-3,3-dicarboxy-propyl)-carbamoyläthyl]-carbamoyl-äthyl}-2-desoxy-D-glucose,

2-(β-Carbomethoxy-succinamido)-3-O-{[L-1-(D-1-carbamoyl-3-carboxy-propyl)-carbamoyl-äthyl-carbamoyl-methyl}-2-desoxy-D-glucose,

2-(β-Carbomethoxy-succinamido)-3-O-{D-1-[L-1-[D-1-carbamoyl-3-carboxy-propyl)-carbamoyl-äthyl]-carbamoyl-äthyl}-2-desoxy-D-glucose,

2-Benzamino-3-O-{[L-1-(D-1-carbamoyl-3-trimethylsilyl-carboxy-propyl)-carbamoyl-äthyl]-carbamoyl-methyl}-2-desoxy-1,4,6-tris-trimethylsilyl-D-glucose. (Bei Kontakt mit Wasser wird die Trimethylsilylestergruppe rasch verseift),

2-Acetamino-2-desoxy-3-O-{[L-1-(D-1-carbamoyl-3-trimethyl-silylcarboxy-propyl)-carbamoyl-äthyl]-carbamoylmethyl}-1,4,6-tris-trimethylsilyl-D-glucose,

2-Acetamino-3-O-{D-1-[L-1-(D-1-carbamoyl-3-carboxy-propyl)-carbamoyl-propyl]-carbamoyl-äthyl}-2-desoxy-D-glucose,

2-Acetamino-3-O-{D-1-[L-1-(D-1-carbamoyl-3-carboxy-propyl)-carbamoyl-2′-hydroxy-propyl]-carbamoyl-äthyl}-2-desoxy-D-glucose,

2-Acetamino-3-O-{D-1-[L-1-(D-1-carbamoyl-3-carboxy-propyl)-carbamoyl-2′-(p-hydroxy-phenyl)-äthyl]-carbamoyl-äthyl}-2-desoxy-D-glucose,

2-Acetamino-3-O-{D-1-[L-1-(D-1-carbamoyl-3-carboxy-propyl)-carbamoyl-N,N-tetramethylen]-carbamoyl-äthyl}-2-desoxy-D-glucose,

2-Glykolylamino-3-O-{D-1-[L-1-(D-1-carbamoyl-3-carboxy-propyl)-carbamoyl-äthyl]-carbamoyl-äthyl}-2-desoxy-D-glucose,

2-Glykolylamino-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{[L-1-(D-1-carbamoyl-3-carboxy-propyl)-carbamoyl-äthyl]-carbamoyl-methyl}-2-desoxy-D-glucose,

2-(N-Methyl-acetamino)-3-O-{D-1-[L-1-(D-1-carbamoyl-3-carboxy-propyl)-carbamoyl-äthyl]-carbamoyl-äthyl}-2-desoxy-D-glucose,

2-Acetamino-3-O-{D-1-[L-1-(D-1-carbamoyl-3-carboxy-propyl)-carbamoyl-2′-phenyläthyl]-carbamoyl-äthyl}-2-desoxy-D-glucose,

2-Acetamino-3-O-{[L-1-(D-1-carbamoyl-3-carboxy-propyl)-carbamoyl-2′-(p-hydroxyphenyl)-äthyl]-carbamoylmethyl}-2-desoxy-D-glucose,

2-Acetamino-3-O-{[L-1-(D-1-[L-1-carboxy-äthyl]-carbamoyl-3-benzylcarboxy-propyl)-carbamoyl-äthyl]-carbamoylmethyl}-2-desoxy-D-glucose,

2-Acetamino-3-O-{[L-1-(D-1,3-di-carboxy-propyl)-N-methyl-carbamoyl-äthyl]-carbamoyl-methyl}-2-desoxy-D-glucose,

Verschiedene der oben erwähnten Muramylpeptide bzw. ihre mit Spacern gekuppelte Formen sind neu. Sie lassen sich z.B. wie in den folgenden Beispielen beschrieben erhalten:

Beispiel 38

Eine Lösung von 3,4 g Benzyl-2-acetamino-3-O-{*D*-1-{*L*-1-[*D*-1-carbamoyl-3-(*L*-1-carboxy-äthyl-carbamoyl)-propyl]-carbamoyl-äthyl}-carbamoyl-äthyl}-2-desoxy-α-D-glucopyranosid-benzylester in 100 ml Methanol/destilliertem Wasser ⅔ wird in Gegenwart von 0,3 g 10% Paladium auf Kohle bei Normaldruck und 45°C während 24 Stunden hydriert. Man filtriert den Katalysator ab und dampft das Filtrat ein. Der Rückstand wird in 40 ml Wasser gelöst und diese Lösung 3-mal mit je 40 ml Wasser gesättigtem sec. Butanol extrahiert. Die organischen Phasen werden noch 3-mal mit je 40 ml mit sec. Butanol gesättigtem Wasser gewaschen. Die wässrigen Lösungen werden vereinigt, eingedampft, der Rückstand in wenig destilliertem Wasser gelöst und gefriergetrocknet. Man erhält so die 2-Acetamino-3-O-{*D*-1-{*L*-1-[*D*-1-carbamoyl-3-(*L*-1-carboxy-äthyl-carbamoyl)-propyl]-carbamoyl-äthyl}-carbamoyl-äthyl}-2-desoxy-D-glucose als weisses Pulver vom $[\alpha]_D^{20} = +9° \pm 1°$ (dest. Wasser, c = 1,090).

Das verwendete Ausgangsmaterial wird auf dem folgenden Weg hergestellt:

Eine Lösung von 6,1 g Benzyl-2-acetamino-3-O-{*D*-1-[*L*-1-(*D*-1-carbamoyl-3-carboxypropyl)-carbamoyl-äthyl]-carbamoyl-äthyl}-2-desoxy-α-D-glucopyranosid-monohydrat und 3,5 g L-Alaninbenzylester-p-toluolsulfonat in 30 ml N,N-Dimethyl-formamid wird mit 1,4 ml Triäthylamin, 1,1 g N-Hydroxy-succinimid und 2,3 g Dicyclohexylcarbodiimid versetzt und 48 Stunden bei Raumtemperaturgerüht. Das auskristallisierte Dicyclohexylharnsoff wird abgesaugt und mit 10 ml N,N-Dimethylformamid gewaschen und das Filtrat zur Trockne eingedampft. Der Rückstand wird in 100 ml Wasser suspendiert, 1 Stunde bei 0°C gerührt, das Unlösliche abgesaugt, mit wenig Eiswasser gewaschen und getrocknet. Das Produkt wird noch in Methanol gelöst, mit der zweifachen Menge Essigwasser gefällt, abgesaugt, mit wenig Essigester gewaschen und getrocknet: $[\alpha]_D^{20} = +72 \pm 1°$ (Methanol, c = 0,998).

In analoger Weise erhält man ausgehend von Benzyl-2-acetamino-3-O-{[*L*-1-(*D*-1-carbamoyl-3-carboxypropyl)-carbamoyl-äthyl]-carbamoyl-methyl}-2-desoxy-α-D-glucopyranosid die 2-Acetoamino-3-O-{{*L*-1-(*D*-1-carbamoyl-3-(*L*-1-carboxy-äthyl-carbamoyl)-propyl]-carbamoyl-äthyl}-carbamoyl-methyl}-2-desoxy-D-glucose.

Ausgehend von Benzyl-3-O-{*D*-1-[*L*-1-(*D*-1-carbamoyl-3-carboxy-propyl)-carbamoyl-äthyl]-carbamoyl-äthyl}-2-desoxy-2-propionamino-α-D-glucopyranosid und Glycinbenzylester-p-toluolsulfonat wird die 3-O-{*D*-1-{*L*-1-[*D*-1-Carbamoyl-3-(carboxy-methyl-carbamoyl)-propyl]-carbamoyl-äthyl}-carbamoyl-äthyl}-2-desoxy-2-propionamino-D-glucose hergestellt.

Analog werden hergestellt:

2-Acetyamino-3-O-{{*L*-1-[*D*-1-carbamoyl-3-(carboxy-methyl-carbamoyl)-propyl]-carbamoyl-äthyl}-carbamoyl-methyl}-2-desoxy-D-glucose,

2-Butyroylamino-3-O-{D-1{*L*-1-[*D*-1-carbamoyl-3-(*L*-1-carboxy-äthyl-carbamoyl)-propyl]-carbamoyl-äthyl}-carbamoyl-äthyl}-2-desoxy-D-glucose,

2-Butyroylamino-3-O-{{*L*-1-[*D*-1-carbamoyl-3-(*L*-1-carboxy-äthyl-carbamoyl)-propyl]-carbamoyl-äthyl}-carbamoyl-methyl}-2-desoxy-D-glucose,

3-O-{{*L*-1-[*D*-1-Carbamoyl-3-(carboxy-methyl-carbamoyl)-propyl]-carbamoyl-äthyl}-carbamoyl-methyl}-2-desoxy-2-propionamino-D-glucose,

2-iso-Butyroylamino-3-O-{{*L*-1-[*D*-1-carbamoyl-3-(*L*-carboxy-äthyl-carbamoyl)-propyl]-carbamoyl-propyl}-carbamoyl-methyl}-2-desoxy-D-glucose,

2-iso-Butyroylamino-3-O-{D-1-{*L*-1-[*D*-1-carbamoyl-3-(*L*-1-carboxy-äthyl-carbamoyl)-propyl]-carbamoyl-propyl}-carbamoyl-äthyl}-2-desoxy-D-glucose,

2-iso-Butyroylamino-3-O-{{*L*-1-[*D*-1-carbamoyl-3-(*L*-1-carboxy-äthyl-carbamoyl)propyl]-carbamoyl-2-methylpropyl}-carbamoyl-methyl}-2-desoxy-D-glucose,

2-iso-Butroylamino-3-O-{D-1-{*L*-1-[*D*-1-carbamoyl-3-(*L*-D-1-carboxy-äthyl-carbamoyl)-propyl]-carbamoyl-2-methylpropyl}-carbamoyl-äthyl}-2-desoxy-D-glucose,

2-iso-Butyroylamino-3-O-{{*L*-1-[*D*-1-carbamoyl-3-(carboxy-methyl-carbamoyl)-propyl]-carbamoyl-äthyl}-carbamoyl-methyl}-2-desoxy-D-glucose,

2-iso-Butyroylamino-3-O-{{*L*-1-[*D*-1-carbamoyl-3-(carboxy-methyl-carbamoyl)-propyl]-carbamoyl-äthyl}-carbamoyl-methyl}-2-desoxy-D-glucose,

2-iso-Butyroylamino-3-O-{D-1-{*L*-1-[*D*-1-carbamoyl-3-(carboxy-methyl-carbamoyl)-propyl]-carbamoyl-äthyl}-carbamoyl-äthyl}-2-desoxy-D-glucose,

2-iso-Butyroylamino-3-O-{{*L*-1-[*D*-1-carbamoyl-3-(*L*-1-carboxy-äthyl-carbamoyl]-propyl]-carbamoyl-äthyl}-carbamoyl-methyl}-2-desoxy-D-glucose,

2-iso-Butyroylamino-3-O-{*D*-1-{*L*-1-[*D*-1-carbamoyl-3-(*L*-1-carboxy-äthyl-carbamoyl)-propyl]carbamoyl-äthyl}-carbamoyl-äthyl}-2-desoxy-D-glucose,

2-iso-Butyroylamino-3-O-{{*L*-1-[*D*-1-carbamoyl-3-(*L*-1-carboxy-propyl-carbamoyl)-propyl]-carbamoyl-äthyl}-carbamoyl-methyl}-2-desoxy-D-glucose,

2-Acetamino-3-O-{{*L*-1-[*D*-1-carbamoyl-3-(carboxy-methyl-carbamoyl)-propyl]-carbamoyl-2-methylpropyl}-carbamoyl-methyl}-2-desoxy-D-glucose,

2-Acetamino-3-O-{{D-1-*L*-1-[*D*-1-carbamoyl-3-(carboxy-methyl-carbamoyl)-propyl]-carbamoyl-2-methylpropyl}-carbamoyl-äthyl}-2-desoxy-D-glucose,

2-Acetamino-3-O-{{*L*-1-[*D*-1-carbamoyl-3-(*L*-1-carboxy-äthyl-carbamoyl)-propyl]-carbamoyl-2-methylpropyl}-carbamoyl-methyl}-2-desoxy-D-glucose,

2-Acetamino-3-O-{*D*-1-{*L*-1-[*D*-1-carbamoyl-3-(*L*-1-carboxy-propyl-carbamoyl)propyl]-carbamoyl-2-methyl-propyl}-carbamoyl-methyl}-2-desoxy-D-glucose,

2-Butyroylamino-3-O-{{*L*-1-[*D*-1-carbamoyl-3-(*L*-1-carboxy-butyl-carbamoyl)-propyl)-carbamoyl-äthyl}-carbamoyl-methyl}-2-desoxy-D-glucose,

2-Acetamino-3-O-{{*D*-1-*L*-1-[*D*-1-carbamoyl-3-(*L*-1-carboxy-äthyl-carbamoyl)-propyl]-carbamoyl-propyl}-carbamoyl-äthyl}-2-desoxy-D-glucose,

2-Acetamino-3-O-{{*L*-1-[*D*-1-carbamoyl-3-(*L*-1-carboxy-äthyl-carbamoyl)-propyl]-carbamoyl-propyl}-carbamoyl-methyl}-2-desoxy-D-glucose,

2-Acetamino-3-O-{*D*-1-{*L*-1-[*D*-1-carbamoyl-3-(carboxy-methyl-carbamoyl)-propyl]-carbamoyl-propyl}-carbamoyl-äthyl}-2-desoxy-D-glucose,

2-Acetamino-3-O-{{*L*-1-[*D*-1-carbamoyl-3-(carboxy-methyl-carbamoyl)-propyl]-carbamoyl-propyl}-carbamoyl-methyl}-2-desoxy-D-glucose,

2-Acetamino-3-O-{{*L*-1-[*D*-1-carbamoyl-3-(*L*-1-carboxy-propyl-carbamoyl)-propyl]-carbamoyl-äthyl}-carbamoyl-methyl}-2-desoxy-D-glucose,

2-Acetamino-3-O-{*D*-1-{*L*-1-[*D*-1-carbamoyl-3-(*L*-1-carboxy-propyl-carbamoyl)-propyl]-carbamoyl-äthyl}-carbamoyl-äthyl}-2-desoxy-D-glucose,

2-Acetamino-3-O-{{*L*-1-[*D*-1-carbamoyl-3-(*L*-1-carboxy-2-methylpropyl-carbamoyl)-propyl]-carbamoyl-äthyl}-carbamoyl-methyl}-2-desoxy-D-glucose,

2-Acetamino-3-O-{*D*-1-{*L*-1-[*D*-1-carbamoyl-3-(*L*-1-carboxy-2-methyl-propyl-carbamoyl)-propyl]-carbamoyl-äthyl}-carbamoyl-äthyl}-2-desoxy-D-glucose,

2-Acetamino-3-O-{{*L*-1-[*D*-1-carbamoyl-3-(*L*-1-carboxy-butyl-carbamoyl)-propyl]-carbamoyl-äthyl}-carbamoyl-methyl}-2-desoxy-D-glucose,

2-Butyroylamino-3-O-{{*L*-1-[*D*-1-carbamoyl-3-(carboxy-methyl-carbamoyl)-propyl]-carbamoyl-äthyl}-carbamoyl-methyl}-2-desoxy-D-glucose,

2-Butyroylamino-3-O-{*D*-1-{*L*-1-[*D*-1-carbamoyl-3-(*L*-1-carboxy-propyl-carbamoyl)-propyl]-carbamoyl-äthyl}-carbamoyl-äthyl}-2-desoxy-D-glucose,

2-Butyroylamino-3-O-{{*L*-1-[*D*-1-carbamoyl-3-(*L*-1-carboxy-propyl-carbamoyl)-propyl]-carbamoyl-äthyl}-carbamoyl-methyl}-2-desoxy-D-glucose,

2-Butyroylamino-3-O-{*D*-1-{*L*-1-[*D*-1-carbamoyl-3-(*L*-1-carboxy-2-methyl-propyl-carbamoyl)-propyl]-carbamoyl-äthyl}-carbamoyl-äthyl}-2-desoxy-D-glucose,

2-Butyroylamino-3-O-{{*L*-1-[*D*-1-carbamoyl-3-(*L*-1-carboxy-2-methyl-propyl-carbamoyl)-propyl]-carbamoyl-äthyl}-carbamoyl-methyl}-2-desoxy-D-glucose,

2-Benzoylamino-3-O-{{*L*-1-[*D*-1-carbamoyl-3-(*L*-1-carboxy-äthyl-carbamoyl)-propyl]-carbamoyl-äthyl}-carbamoyl-methyl}-2-desoxy-D-glucose,

2-Benzoylamino-3-O-{*D*-1-{*L*-1-[*D*-1-carbamoyl-3-(carboxy-methyl-carbamoyl)-propyl]-carbamoyl-äthyl}-carbamoyl-äthyl}-2-desoxy-D-glucose,

2-Benzoylamino-3-O-{{*L*-1-[*D*-1-carbamoyl-3-(*L*-1-carboxy-äthyl-carbamoyl)-propyl]-carbamoyl-propyl}-carbamoyl-methyl}-2-desoxy-D-glucose,

2-Benzoylamino-3-O-{*D*-1-{*L*-1-[*D*-1-carbamoyl-3-(carboxy-methyl-carbamoyl)-propyl]-carbamoyl-propyl}-carbamoyl-äthyl}-2-desoxy-D-glucose,

2-Acetamino-3-O-{{*L*-1-[*D*-1-carbamoyl-3-(*L*-1-carboxy-äthyl-carbamoyl)-propyl]-carbamoyl-N,N-tetramethylen}-carbamoyl-methyl}-2-desoxy-D-glucose,

2-Acetamino-3-O-{D-1-{*L*-1-[*D*-1-carbamoyl-3-(*L*-1-carboxy-äthyl-carbamoyl)-propyl]-carbamoyl-N,N-tetramethylen}-carbamoyl-äthyl}-2-desoxy-D-glucose,

2-Acetamino-3-O-{{*L*-1-[*D*-1-carbamoyl-3-(carboxy-methyl-carbamoyl)-propyl]-carbamoyl-N,N-tetramethylen}-carbamoyl-methyl}-2-desoxy-D-glucose,

2-Acetamino-3-O-{*D*-1-{*L*-1-[*D*-1-carbamoyl-3-(carboxy-methyl-carbamoyl)-propyl]-carbamoyl-N,N-tetramethylen}-carbamoyl-äthyl}-2-desoxy-D-glucose,

2-Acetamino-3-O-{{*L*-1-[*D*-1-carbamoyl-3-(*L*-1-carboxy-äthyl-carbamoyl)-propyl]-carbamoyl-2-hydroxy-äthyl}-carbamoyl-methyl}-2-desoxy-D-glucose,

2-Acetamino-3-O-{*D*-1-{*L*-1-[*D*-1-carbamoyl-3-(*L*-1-carboxy-äthyl-carbamoyl)-propyl]-carbamoyl-2-hydroxy-äthyl}-carbamoyl-äthyl}-2-desoxy-D-glucose,

2-Benzoylamino-3-O-{{*L*-1-[*D*-1-carbamoyl-3-(*L*-carboxy-äthyl-carbamoyl)-propyl]-carbamoyl-2-hydroxy-äthyl}-carbamoyl-methyl}-2-desoxy-D-glucose,

2-Benzoylamino-3-O-{*D*-1-{*L*-1-[*D*-1-carbamoyl-3-(*L*-1-carboxy-äthyl-carbamoyl)-propyl]-carbamoyl-2-hydroxy-äthyl}-carbamoyl-äthyl}-2-desoxy-D-glucose,

2-Acetamino-3-O-{{*L*-1-[*D*-1-carbamoyl-3-(*L*-1-carboxy-methyl)-carbamoyl-propyl]-carbamoyl-methyl}-carbamoyl-methyl}-2-desoxy-D-glucose,

2-Acetamino-3-O-{*D*-1-{*L*-1-[*D*-1-carbamoyl-3-(*L*-1-carboxy-methyl-carbamoyl)-propyl]-carbamoyl-methyl}-carbamoyl-äthyl}-2-desoxy-D-glucose,

2-Acetamino-3-O-{{*L*-1-[*D*-1-carbamoyl-3-(*L*-1-carboxy-methyl-carbamoyl)-propyl]-carbamoyl-methyl}-carbamoyl-methyl}-2-desoxy-D-glucose,

2-Acetamino-3-O-{*D*-1-{*L*-1-[*D*-1-carbamoyl-3-(*L*-1-carboxy-methyl-carbamoyl)-propyl)-carbamoyl-methyl}-carbamoyl-äthyl-2-desoxy-D-glucose,

2-Acetamino-3-O-{{*L*-1-[*D*-1-carbamoyl-3-(*L*-1-carboxy-methyl-carbamoyl)-propyl)-carbamoyl-methyl}-carbamoyl-methyl}-2-desoxy-D-glucose,

## Beispiel 39

Eine Lösung von 4,2 g Benzyl-2-acetamino-3-O-{{*L*-1-[*D*-1-carbamoyl-3-(*L*-5-benzyloxycarbonylamino-5-carbamoyl-pentyl-carbamoyl)-propyl]-carbamoyl-äthyl}-carbamoyl-methyl}-2-desoxy-α-D-glucopyranosid in 100 ml Methanol/Wasser ⁴⁄₁ wird in Gegenwart von 0,5 10% Palladium/Kohle bei Normaldruck und Raumtemperatur hydriert. Dabei wird das pH des Reaktionsgemisches durch Zugabe von 1n-Salzsäure bei pH 6 gehalten. Nach beendeter Wasserstoffaufnahme filtriert man vom Katalysator ab und dampft das Filtrat zur Trockne ein. Der Rückstand wird in wenig destilliertem Wasser gelöst und gefriergetrocknet. Man erhält so die 2-Acetamino-3-O-{{*L*-1-[*D*-1-carbamoyl-3-(*L*-5-amino-5-carbamoyl-methyl}-2-desoxy-D-glucose-hydrochlorid als weisses Pulver.

Das verwendete Ausgangsmaterial kann wie folgt hergestellt werden:

Eine Lösung von 15 g α-Carbobenzoxy-ε-t-butoxy-carbonyl-L-lysin-methylester in 100 ml einer gesättigten methanolischen Ammoniaklösung wird 48 Stunden bei Raumtemperatur stehen gelassen und zur Trockne eingedampft. Das Produkt, α-Carbobenzoyl-ε-t-butoxycarbonyl-L-lysinamid wird aus Methanol/Äther umkristallisiert, Smp. 142°C $[\alpha]_D^{20} = -3° \pm 1°$ (Methanol, c = 1,018).

Eine auf 0°C gekühlte Lösung von 3 g α-Carbobenzoxy-ε-t-butyloxycarbonyl-L-lysinamid in 25 ml Trifluoressigsäure wird 1 Stunde gerührt und anschliessend zur Trockne eingedampft. Der Rückstand wird mit 20 ml gesättigter Kochsalzlösung und Eis versetzt, mit konzentrierter Ammoniaklösung alkalisch gestellt und mit Essigester extrahiert. Die organische Phase wird noch mit gesättigter Kochsalzlösung gewaschen, über Natriumsulfat getrocknet und zur Trockne eingedampft. Man erhält so das α-Carbobenzoxy-L-lysinamid als weissen Schaum.

Eine Lösung von 5,6 g Benzyl-2-acetamino-3-O-{{*L*-1-[*D*-1-carbamoyl-3-carboxy-propyl)-carbamoyl-äthyl}-carbamoyl-methyl}-2-desoxy-α-D-glucopyranosid und 2,8 g α-Carbobenzoxy-L-lysinamid in 30 ml N,N-Dimethylformamid wird mit 1,1 g N-Hydroxysuccinimid und 2,2 g Dicyclohexylcarbodiimid versetzt und 40 Stunden bei Raumtemperatur gerührt. Der auskristallisierte Dicyclohexylharnstoff wird abgesaugt und mit 10 ml N,N-Dimethylformamid gewaschen. Das Filtrat wird zur Trockne eingedampft und der Rückstand für 30 Minuten mit 100 ml destilliertem Wasser extrahiert. Das Ungelöste wird abgesaugt, mit Wasser gewaschen, getrocknet und stellt das Benzyl-2-acetamino-3-O-{{*L*-1-[*D*-1-carbamoyl-3-(*L*-5-benzyloxycarbonylamino-5-carbamoyl-pentyl-carbamoyl)-propyl]-carbamoyl-äthyl}-carbamoyl-methyl}-2-desoxy-α-D-glucopyranosid dar. Das Produkt wird aus Methanol/Essigester umkristallisiert.

Analog wird hergestellt:

2-Acetamino-3-O-{{*L*-1-[*D*-1-carbamoyl-3-(5-amino-L-1-carbamoyl-pentyl-carbamoyl)-propyl]-carbamoyl-äthyl}-carbamoyl-methyl-}2-desoxy-D-glucose-hydrochlorid.

Beispiel 40

Eine Lösung von 2,8 g Benzyl-3-O-{{*L*-1-[*D*-1-carbamoyl-3-carboxy-propyl)-carbamoyl-propyl]-carbamoyl-methyl}-2-desoxy-2-isobutyroylamino-α-D-glucopyranosid in 80 ml Methanol/destilliertem Wasser ¼wird in Gegenwart von 0,3 g 10% Palladium/Kohle bei Normaldruck und 45°C hydriert. Nach der Aufarbeitung und Gefriertrocknung des Rückstandes erhält man die 3-O-{{*L*-1-[*D*-1-carbamoyl-3-carboxy-propyl)-carbamoyl-propyl]-carbamoyl-methyl}-2-desoxy-2-isobutyroylamino-D-glucose als weisses Pulver.

Das verwendete Ausgangsmaterial wird wie folgt hergestellt:

Ein Gemisch der Lösungen von 21,0 g Benzyl-2-amino-2-desoxy-4,6-O-isopropyliden-α-D-gluocopyranosid in 150 ml destilliertem Wasser wird unter Rühren auf 0°C gekühlt und tropfenweise mit 8,5 ml iso-Buttersäurechlorid versetzt. Nach 1 Stunde Rühren bei Raumtemperatur wird die organische Phase abgetrennt, mit eiskalter 0,5 n Salzsäure, Wasser, einer gesättigten Natriumhydrogencarbonatlösung und wieder Wasser gewaschen, getrocknet und eingedampft. Das Produkt, das Benzyl-2-desoxy-2-isobutylamino-4,6-O-isopropyliden-α-D-glucopyranosid, wird aus 150 ml Äther kristallisiert, Smp. 82°C, $[\alpha]_D^{20} = +109° \pm 1°$ (Chloroform, c = 1,017).

Eine Lösung von 15,1 g Benzyl-2-desoxy-2-isobutyroylamino-4,6-O-isopropyliden-α-D-glucopyranosid in 150 ml absolutem Acetonitril wird in Stickstoffatmosphäre unter Feuchtigkeitsausschluss und Rühren mit 1,9 g Natriumhydrid (Fluka, pract.) versetzt und 1,5 Stunden bei 40°C gerührt. Anschliessend wird das Reaktionsgemisch auf −10°C gekühlt und mit 5,6 ml Bromessigsäuremethylester versetzt. Man rührt noch 15 Minuten im Eisbad und 2 Stunden bei Raumptemperatur nach. Nach der Aufarbeitung erhält man das Benzyl-2-desoxy-2-isobutylamino-4,6-O-isopropyliden-3-O-methoxycarbonyl-methyl-α-D-glucopyranosid, das aus Äther/Petroläther umkristallisiert wird, Smp. 119—120°C, $[\alpha]_D^{20} = +152 \pm 1°$ (Chloroform, c = 0,963).

Eine Lösung von 3,16 g Benzyl-2-desoxy-2-isobutyroylamino-4,6-O-isopropyliden-3-O-methoxycarbonylmethyl-α-D-glucopyranosid in 30 ml Methanol und 10 ml 1n-Natronlauge wird bei Raumtemperatur 1 Stunde stehen gelassen. Man gibt 3 ml 1n-Salzsäure zu und dampft die Lösung zur Trockne ein. Der Rückstand wird in 50 ml N,N-Dimethylformamid gelöst und mit 2,26 g L-α-Aminobutyroyl-D-isoglutamin-tert.-butylester-hydrochlorid und 1,75 EEDQ versetzt und 24 Stunden bei Raumtemperatur stehen gelassen. Man gibt noch 0,2 g EEDQ zu und lässt das Gemisch weitere 24 Stunden stehen. Das Lösungsmittel wird abdestilliert und der Rückstand in Essigester/Wasser gelöst. Die organische Phase wird abgetrennt und mit eiskalter 1n-Salzsäure, Wasser, einer gesättigten Natriumhydrogencarbonatlösung und Wasser gewaschen, über Natriumsulfat getrocknet und zur Trockne eingedampft. Man erhält so den Benzyl-3-O-{*L*-1-[*D*-1-carbamoyl-3-carboxy-propyl)-carbamoyl-propyl-carbamoyl-methyl)-2-desoxy-2-isobutylamino-4,6-O-isopropyliden-α-D-glucopyranosid-tert.-butylester als weissen Schaum.

Dieses Produkt wird in 60 ml Eisessig gelöst, mit 60 ml Wasser unter Rühren versetzt und 24 Stunden bei Raumptemperatur stehen gelassen. Diese Lösung wird im Wasserstrahlvakuum zu Trocken eingedampft und der Rückstand in Äthanol gelöst. Man filtriert mit Aktivkohle und dampft wieser zur Trockne ein. Man erhält so dem Benzyl-3-O-{{*L*-1-[*D*-1-carbamoyl-3-carboxy-propyl)-carbamoyl-propyl]-carbamoyl-methyl}-2-desoxy-2-isobutyroylamino-α-D-glucopyranosid-tert.-butylester als weisses amorphes Material vom $[\alpha]_D^{20} = +74° \pm 1°$ (Methanol, c = 0,950).

Eine auf 0°C gekühlte Lösung von 3,9 g von diesem Tert.-butylester in 40 ml 98% Trifluoressigsäure wird 1 Stunde bei 0°C gerührt und auf 500 ml absolutem Äther gegossen. Das auskristallisierte Produkt wird abgesaugt, mit Äther gewaschen und im Vakuum getrocknet. Das erhaltene Pulver wird in 40 ml Wasser/Tetrahydrofuran ¼ gelöst und 30 Minuten mit 50 ml Ionenaustauscherherz Dowex 3 (Acetat-Form) gerührt. Der Ionenaustauscher wird abfiltriert und mit 500 ml Tetrahydrofuran/1n-Essigsäure gewaschen. Das Filtrat wird zur Trockne eingedampft und das Produkt aus Methanol/Äther kristallisiert, Smp. 205—206°C.

Beispiel 41

Eine Lösung von 3,1 g Benzyl-3-{[*L*-1-(*D*-1-carbamoyl-3-carboxy-propyl)-carbamoyl-äthyl]-carbamoyl-methyl}-2-desoxy-2-isobutyroylamino-α-D-glocopyranosid in 40 ml Methanol/Wasser ¼ wird in Gegenwart von 10% Palladium/Kohle bei Normaldruck und 45°C hydriert. Nach der Aufarbeitung erhält man die 3-O-{{*L*-1-[*D*-1-carbamoyl-3-carboxy-propyl)-carbamoyl-äthyl]-carbamoyl-methyl}-2-desoxy-2-isobutyroylamino-D-glucose als weisses Pulver durch Gefriertrocknung.

Das verwendete Ausgangsmaterial wird analog wie im Beispiel 40 beschrieben, hergestellt:

Bei der Kondenation von Benzyl-3-O-caboxy-methyl-2-desoxy-2-isobutyroylamino-4,6,O-iopropyliden-α-D-glucopyranosid-natriumsalz mit L-Alanyl-D-isoglutamin-tert.butylester-hydrochlorid entsteht der Benzyl-3-O-{[*L*-1-(*D*-1-carbamoyl-3-carboxy-propyl)-carbamoyl-äthyl]-carbamoyl-methyl}-2-desoxy-2-isobutyroylamino-4,6-O-isopropyliden-α-D-glucopyranosid-tert.butylester als weisser Schaum.

Die Hydrolyse der 4,6-O-Isoproylidengruppe gibt Benzyl-3-O-{[*L*-1-(*D*-1-carbamoyl-3-carboxypropyl)-carbamoyl-äthyl]-carbamoyl-methyl}-2-desoxy-2-isobutyroylamino-α-D-glucopyranosid-tert.butylester von $[\alpha]_D^{20} = +71° \pm 1°$ (Methanol, c = 0,956).

Der tert.Butylester wird mit Trifluoroessigsäure gespalten. Man erhält so das Benzyl-3-O-{[*L*-1-(*D*-1-carbamoyl-3-carboxy-propyl)-carbamoyl-äthyl]-carbamoyl-methyl}-2-desoxy-2-isobutyroylamino-α-D-glucopyranosid als weisses amorphes Produkt.

Beispiel 42

Eine Lösung von 4,2 g Benzyl-2-acetyl-N-methylamino-3-O-{[*L*-1-(*D*-1-carbamoyl-3-carboxy-propyl)-carbamoyl-äthyl]-carbamoyl-methyl}-2-desoxy-D-glucopyranosid in 75 ml Methanol/Wasser⅟₁ wird in Gegenwart von 0,5 g 10% Palladium/Kohle bei Normaldruck und 45°C hydriert. Der Katalysator wird abfiltriert und das Filtrat zur Trockne eingedampft. Der Rückstand wird in wenig destilliertem Wasser gelöst und gefriergetrocknet. Man erhält so die 2-Acetyl-N-methylamino-3-O-{[*L*-1-(*D*-1-carbamoyl-3-carboxy-propyl)-carbmoyläthyl]-carbamoyl-methyl}-2-desoxy-D-glucose als weisses Pulver.

Das verwendete Ausgangsmaterial wird wie folgt hergestellt:

Eine Lösung von 8,5 g Benzyl-2-acetamino-2-desoxy-4,6,O-isopropyliden-3-O-methoxycarbonylmethyl-α-D-glucopyranosid in 80 ml absolutem Acetonitril wird in Stickstoffatmosphäre unter Rühren und Feuchtigkeitsausschluss mit 0,75 g Natriumhydrid (Fluka, pract.) versetzt und 1 Stunde bei 40 °C gerührt. Das Reaktionsgemisch wird nun auf Raumtemperatur gekühlt und tropfenweise während 4 Stunden mit einer Lösung von 6,0 g Methyljodid in 50 ml absolutem Acetonitril versetzt. Nach weiteren 3 Stunden wird das Reaktionsgemisch filtriert und das Filtrat zur Trockne eingedampft. Der Rückstand wird in Essigester gelöst, diese Lösung mit Wasser gewaschen, über Natriumsulfat getrocknet und zur Trockne eingedampft. Man erhält so das Benzyl-2-acetyl-N-methyl-amino-2-desoxy-4,6,O-isopropyliden-3-O-methoxy-carbonyl-methyl-α-D- gluocopyranosid als gelbes Öl vom Rf-Wert 0,45 auf Kieselgeldünnschichtplatten im System Methylenchlorid/Essigester 85/15.

Eine Lösung von 4,4 g Benzyl-2-acetyl-N-methylamino-2-desoxy-4,6-O-isopropyliden-3-O-methoxycarbonyl-methyl-α-D-glucopyranosid in 60 ml Methanol und 15 ml 1n-Natronlauge wird 1 Stunde bei Raumtemperatur stehen gelassen, mit 5 ml 1n-Salzsäure versetzt und zur Trockne eingedampft. Das erhaltene Benzyl-2-acetyl-N-methylamino-3-O-carboxymethyl-2-desoxy-4,6-O-isopropyliden-α-D-glucopyranosid-natriumalz wird in 50 ml N,N-Dimethylformamid gelöst und mit 3,2 g L-Alanyl-D-isoglutamin-tert.butylester-hydrochlorid in Gegenwart von 2,5 g EEDQ kondensiert. Die Lösung wird im Vakuum zur Trockne eingedampft und der Rückstand in Essigester gelöst. Man wäscht diese Lösung mit Wasser, eiskalten 1n-Salzsäure, Wasser einer gesättigten Natrumhydrogencarbonat-Lösung und Wasser, trocknet über Magnesiumsulfat und dampft zur Trockne ein. Man erhält so den Benzyl-2-acetyl-N-methylamino-3-O-{[*L*-1-(*D*-1-carbamoyl-3-carboxy-propyl)-carbamoyläthyl]-carbamoyl-methyl}-2-desoxy-4,6,O-isopropyliden-α-D-glucopyranosid-tert.butylester als gelblichen Schaum. Dieses Produkt wird in 45 ml auf 0°C vorgekühlter 95%iger Trifluoressigsäure gelöst und 1 Stunde bei 0°C gerührt.

Das Reaktionsgemisch wird auf 400 ml absolutem Äther gegossen, das ausgefallene Produkt abgesaugt, mit Äther gewaschen und getrocknet. Durch Behandlung dieser Substanz mit den Ionenaustauscherherz Dowex-3-Acetat-Form erhält man das trifluoressigsäurefreie Benzyl-2-acetyl-N-methyl-amino-3-O-{[*L*-1-(*D*-1-carbamoyl-3-carboxy-propyl)-carbamoyl-äthyl]-carbamoyl-methyl}-2-desoxy-α-D-glucopyranosid als weisses Pulver.

Auf analoge Weise werden hergestellt:

2-Acetyl-N-methyl-amino-3-O-{*D*-1-[*L*-1-(*D*-1-carbamoyl-3-carboxy-propyl)-carbamoyl-äthyl]-carbamoyl-äthyl}-2-desoxy-D-glucose,

2-Acetyl-N-methyl-amino-3-O-{*D*-1-[*L*-1-(*D*-1-carbamoyl-3-carboxy-propyl)-carbamoyl-propyl]-carbamoyl-äthyl}-2-desoxy-D-glucose,

2-Acetyl-N-methyl-amino-3-O-{[*L*-1-(*D*-1-carbamoyl-3-carboxy-propyl)-carbamoyl-propyl]-carbamoyl-methyl}-2-desoxy-D-glucose,

2-Acetyl-N-methyl-amino-3-O-{[*L*-1-(*D*-1-carbamoyl-3-carboxy-propyl)-carbamoyl-2-methyl-propyl]-carbamoyl-methyl}-2-desoxy-D-glucose,

2-Acetyl-N-methyl-amino-3-O-{*D*-1-[*L*-1-(*D*-1-carbamoyl-3-carboxy-propyl)-carbamoyl-2′-methyl-propyl]-carbamoyl-äthyl}-2-desoxy-D-glucose,

3-O-{[*L*-1-(*D*-1-Carbamoyl-3-carboxy-propyl)-carbamoyl-äthyl]-carbamoyl-methyl}-2-desoxy-2-propionyl-N-methyl-amino-D-glucose,

2-Butyryl-N-methyl-amino-3-O-{[*L*-1-(*D*-1-carbamoyl-3-carboxy-propyl)-carbamoyl-äthyl]-carbamoyl-methyl}-2-desoxy-D-glucose.

Beispiel 43

Die Trimethylsilyläther lassen sich z.B. wie folgt herstellen:

0,3 g 2-Benzamido-2-desoxy-3-O-{[*L*-1-(*D*-1-carbamoyl-3-carboxypropyl)-carbamoyl-äthyl]-carbamoyl-methyl}-*D*-glucose löst man in 3 ml Dimethylformamid und versetzt mit 0,4 ml Bistrimethylsilylacetamid. Nach 5 Stunden bei 35° dampft man im Vakuum zum Sirup ein, aus dem man durch Lösen in absolutem Äther oder absolutem Essigester gebildetes Acetamid abscheiden kann. Nach erneutem Eindampfen erhält man einen farblosen Sirup mit $[\alpha]_D^{20} = +15°$ (Dioxan, c = 0,8).

Analog erhält man sirupöse 2-Acetamino-2-desoxy-3-O-{[*L*-1-(*D*-1-carbamoyl-3-trimethylsilyl-carboxy-propyl)-carbamoyläthyl]-carbamoylmethyl}-1,4,6-tri-trimethylsilyl-*D*-glucose mit $[\alpha]_D^{20} = -10°$ (Dioxan, c = 0,91).

Bei Kontakt mit Wasser wird die Trimethylsilylestergruppe rasch verseift, so dass diese Derivate auch zur Kupplung geeignet sind.

Beispiel 44

Eine 5%ige Lösung von Benzyl-2-acetamido-3-O-{{*L*-1-[*D*-1-(*L*-1-carboxy-äthyl)-carbamoyl-3-carboxypropyl)-carbamoyl-äthyl}-carbamoyl-methyl}-2-desoxy-α-D-glucopyranosid in Terahydrofuran/Wasser 3/1 wird in Gegenwart von 10%igem Palladium auf Kohle bei Normaldruck und Raumtemperatur hydriert. Nachdem die theoretische Menge Wasserstoff aufgenommen ist, wird vom Katalysator abfiltriert und das Filtrat gefriergetrocknet. Man erhält so die 2-Acetamido-3-O-{{*L*-1-[*D*-1-(*L*-1-carboxy-äthyl)-carbamoyl-3-carboxypropyl]-carbamoyl-äthyl}-carbamoyl-methyl}-2-desoxy-D-glucose als weisses Pulver. Rf-Wert im Dünnschichtchromatogramm = 0,21 (Essigsäureäthylester/n-Butanol/Pyridin/Essigsäure/Wasser 42:21:21:6:10).

Auf analoge Weise erhält man das Derivat mit der echten Muraminsäure.

Das Ausgangsmaterial kann wie folgt hergestellt werden:

5,68 g N-tert.-Butoxycarbonyl-L-alanyl-*D*-γ-benzyl-glutamyl-L-alaninbenzylester werden in einem Gemisch von 5 ml Trifluoressigsäure und 5 ml 1,2-Dichloräthan gelöst und unter Feuchtigkeitausschluss 16 Stunden bei Raumtemperatur stehen gelassen. Man verdünnt diese Lösung mit 50 ml Tetrahydrofuran, kühlt im Eisbad ab und neutralisiert mit Triäthylamin. Nach der Zugabe der Lösung von 3,7 g Benzyl-2-acetamido-3-carboxymethyl-2-desoxy-α-D-glucopyranosid und 1,38 ml Triäthylamin in 100 ml Tetrahydrofuran wird das Ganze mit 2,6 g 2-Äthoxy-N-äthoxycarbonyl-1,2-dihydrochinolin versetzt und 24 Stunden bei Raumtemperatur stehen gelassen. Nach dem Abdampfen des Lösungsmittels wird der Rückstand in Chloroform/Methanol 9/1 gelöst, mit Wasser, eiskalter 2n-Salzsäure, Wasser, einer gesättigten Natriumhydrogencarbonatlösung und Wasser gewaschen, filtriert und von Lösungsmittel befreit. Man erhält so das Benzyl-2-acetamido-3-O-{{*L*-1-(*D*-1-(*L*-1-carboxy-äthyl)-carbamoyl-3-carboxy-propyl]-carbamoyläthyl}-carbamoyl-methyl}-2-desoxy-α-D-glucopyranosid als farbloses Pulver, Rf-Wert = 0,48 (im identischen System).

Das verwendete Ausgangsmaterial kann wie folgt hergestellt werden:

7,15 g N-tert.-Butoxycarbonyl-L-alanyl-*D*-glutaminsäure-γ-benzylester und 6,15 g L-Alaninbenzylester-p-tolulsulfonat werden in 100 ml wasserfreiem Dimethylformamid gelöst. Man kühlt im Eisbad ab und fügt unter Rühren nacheinander 4,03 g N-Hydroxysuccinimid, 3,61 g Dicyclohexylcarbodiimid und scliesslich 1,95 ml N-Methylmorpholin zu. Nach 6-stündigem Rühren bei 0° und 15 Stunden bei Raumtemperatur wird die Suspension abgekühlt, der Niederschlag (Dicyclohexylharnstoff und Morpholinhydrochlorid) abfiltriert und das Filtrat eingedampft. Der Rückstand wird in Essigsäureäthylester aufgenommen, mehrmals mit Wasser, 1n-Zitronensäure und 1n-Natriumhydrogencarbonatlösung und wieder Wasser gewaschen. Die Essigesterlösung wird getrocknet, eingedampft und der kristalline Rückstand aus Essigester/Petroläther 1/1 rekristallisiert, Fp. 135—136° $[\alpha]_D^{20} = -9° \pm 1°$ (Methanol, c = 1), Rf-Wert = 0,82 (im obigen System) und 0,86 (Acetonitril/Wasser 3:1).

An Stelle von Alanin kann das geschützte Dipeptid analog mit anderen natürlichen L-Aminosäuren verlängert werden.

Beispiel 45

7,5 × $10^8$ abgetötete Tripanosoma cruzi Parasiten (Erreger der Chagas-Krankheit) werden in einer Lösung von 50 mg 2-Acetamino-3-O-{[L-1-(D-carbamoyl-3-carboxypropyl)-carbamoyläthyl]-carbamoylmethyl}-2-desoxy-D-glucose-N-hydroxy-succinimidester in 6 ml physiologischer Pufferlösung suspendiert. Die Suspension wird zwei Stunden bei 37°C inkubiert. Danach werden die mit dem Muramyldipeptid konjugierten Parasiten durch Zentrifugation sedimentiert. Das Sediment wird durch Resuspension in physiologischer Pufferlösung und erneutem Zentrifugieren gewaschen. Die gewaschenen Parasiten-Muramyldipeptid-Konjugate werden in physiologischer Pufferlösung suspendiert und zur Immunisierung verwendet.

Die quantitative Bestimmung um an die Trypanosomen gekuppeltem Muramyldipeptid erfolgt wie in Beispiel 1 angegeben mit der Morgan-Elson-Reaktion und ergibt 50—70 mg Muramyldipeptid pro mg Trypanosomen.

**Patentansprüche**

1. Als Antigen wirksame Verbindungen der Formel III,

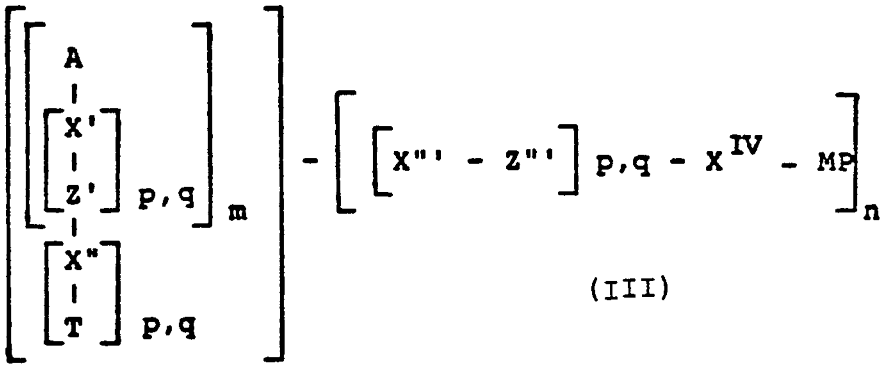

(III)

worin A den Rest eines Antigens, T den Rest eines Trägers, MP den Rest eines Muramylpeptids der Formel II,

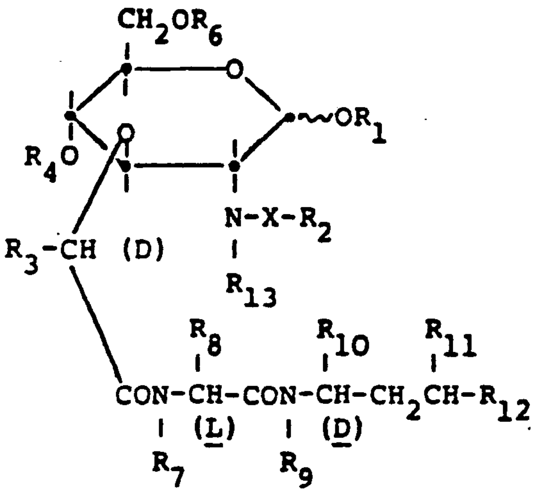

(II)

worin X eine Carbonyl-, Carbonyloxy- oder Sulfonylgruppe, $R_1$ Wasserstoff, Alkyl, gegebenenfalls substituiertes Benzyl oder Acyl, $R_2$ gegebenenfalls substituiertes Alkyl oder carbocyclisches Aryl, $R_4$ und $R_6$ unabhängig voneinander Wasserstoff, Alkyl, gegebenenfalls substituiertes Benzyl oder Acyl, $R_3$ Wasserstoff oder Alkyl, $R_7$ und $R_{13}$ Wasserstoff oder Niederalkyl, $R_8$ Wasserstoff, Niederalkyl, freies, verestertes oder veräthertes Hydroxyniederalkyl, freies, verestertes oder veräthertes Mercapto-niederalkyl, freies oder acyliertes Aminoniederalkyl, Cycloalkyl mit 5 oder 6 Kohlenstoffatomen, Cycloalkyl-niederalkyl, dessen Cycloalkylrest 5 oder 6 Kohlenstoffatome enthält, gegebenenfalls substituiertes Aryl oder Aralkyl, stickstoffhaltiges Heterocyclyl- oder Heterocyclyl-niederalkyl, $R_7$ und $R_8$ zusammen auch Alkylen mit 3 oder 4 Kohlenstoffatomen, $R_9$ Wasserstoff oder Niederalkyl, die Reste $R_{10}$, $R_{11}$ und $R_{12}$ unabhängig voneinander einen gegebenenfalls veresterten oder amidierten Carboxyrest und $R_{11}$ auch Wasserstoff bedeuten, oder $R_1$, $R_4$ und $R_6$ Triniederalkylsilyl, insbesondere Trimethylsilyl, sind, wobei das Präfix "Nieder" stets einen Rest bis und mit 7 C-Atomen kennzeichnet, Z' und Z''' bivalente Brückenglieder, X', X'', X''' und $X^{IV}$ unabhängig voneinander die kovalenten Verknüpfungselemente Carbonyloxy

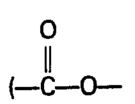 oder 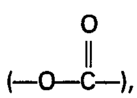, Carbonylimino 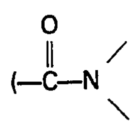 oder 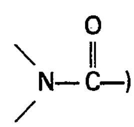

oder Carbonylthio 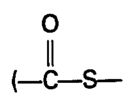 oder 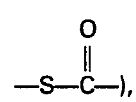,

p = 0, q = 1 und m und n ganze Zahlen grösser als 0 bedeuten, mit der Massgabe, dass in den Verbindungen der Formel III mindestens eine in der Peptidchemie übliche Verknüpfung vorhanden ist, die synthetisch hergestellt worden sein muss und dass die als Ausgangsstoffe verwendeten Komponenten

Muramylpeptid, Träger und Antigen die zur Verknüpfung miteinander oder mit dem Brückenglied geeigneten funktionellen Gruppen aufweisen.

2. Antigenderivate nach Anspruch 1, die keinen Träger enthalten.

3. Antigenderivate nach Anspruch 1 oder 2, die als Bruckenglied(er unabhängig voneinander) den bivalenten Rest eines über die Stickstoffatome gebundenen α,ω-Diamino-Niederalkans, Niederalkandicarbonyl oder bivalent gebundenes, α-, β- oder γ-Amino-Alkanocarbonyl enthalten.

4. Antigenderivate nach Anspruch 1 oder 2, worin das Antigen direkt (d.h. nicht über ein Brükkenglied) mit dem Muramylpeptid verbunden ist.

5. Antigenderivate gemäss einem der Ansprüche 1 bis 4, dadurch gekennzeichnet, dass das Antigen den wirksamen Inhaltsstoff einer Vakzine gegen Parasiten, Bakterien, Viren oder Tumorzellen, abgeschwächte oder abgetötete Formen oder Teilkomponenten von Parasietn, Bakterien, Viren oder Tumorzellen, einer Vakzine gegen körpereigene Strukturen, immunologische Erkennungsstrukturen oder einer Vakzine, die sich zur spezifischen Desensibilisierung bei Allergien eignet, darstellt.

6. Antigenderivate gemäss einem der Ansprüche 1 bis 5, die als Antigen den wirksamen Inhaltsstoff einer Vakzine gegen Malaria, Cholera, Typhus, Meningitis, rheumatisches Fieber als Folge von Streptokokkeninfekten, Influenza, Tollwut, Maul- und Klauenseuche, sowie eines Impfstoffs zur Induktion von Immunität gegen Komponenten des Fortpflanzungssystems enthalten.

7. Antigenderivate gemäss einem der Ansprüche 1 bis 4, die als Antigen Malaria-Merozoiten, typenspezifische Menigokokkenpolysaccharide A, B oder C, M-Proteine von Streptokokken, Influenzavirushaemagglutinine, autologe Tumorzellen oder autologe Tumorzellenmembranbestandteile, Partialsequenzen von humanem Chloriongonadotropin, Gräserpollenextrakte, antigenspezifische T-Zell-Lymphoblasten und deren Rezeptoren für Antigene oder antigenspezifische Immunglobuline enthalten.

8. Antigenderivate nach einem der Ansprüche 1 bis 4, die als Antigen abgeschwächte lebende, abgetötete, modifizierte oder abgebaute Chlamydien, Rickettsien, Protozoen oder metazoische Parasiten, Bestandteile von Spermatozoen, mit autologen Tumorzellen kreuzreagierende homologe bzw. heterologe Tumorzellen oder deren Tumorzellfragmente oder -membrankomponenten einschliesslich Onkornavirus-kodierter Glykoproteine enthalten.

9. Antigenderivate nach einem der Ansprüche 1 bis 5, die als Antigen den wirksamen Inhaltsstoff von Vakzinen zur Behandlung von Influenza A und B, Parainfluenza 1 bis 3, durch respiratorisches Syncital-Virus, Rhinoviren oder Andenoviren hervorgerufene respiratorische Erkrankungen, Cytomegalie, Röteln, Masern, Mumps, Pertussis, Poliomyelitis, Herpes simplex 1 und 2, Varizellen und Herpes zoster, Rotavirus-Erkrankungen, Hepatitis A und B, Trachom, Karies, Erkrankungen durch Pneumokokken, H. Influenzae, Pseudomonas und Proteus, Paratyphus, Gonorrhoe, Syphilis, Trypanosomiasen (Schlafkrankheiten und Changas-Krankheit), Leishmaniosen, Filariosen, Schistosomiasen, Ankylostomiasen oder durch Stäube oder Medikamente hervorgerufenes allergisches Asthma, allergische Rhinitis und Arzneimittelüberempfindlichkeit enthalten.

10. Antigenderivate nach einem der Ansprüche 1—4, die als Antigen ein lösliches Proteinantigen enthalten.

11. Antigenderivate nach einem der Ansprüche 1 bis 10, die den Rest eines Muramylpeptids der Formel (II) enthalten, $R_1$, $R_4$ und $R_6$ unabhängig voneinander Wasserstoff, Alkyl oder gegebenenfalls substituiertes Benzyl oder Acyl und $R_{13}$ Wasserstoff bedeuten.

12. Antigenderivate gemäss einem der Ansprüche 1 bis 11, die den Rest eines Muramylpeptids der Formel II enthalten, worin $R_1$, $R_3$, $R_4$, $R_6$ und $R_7$ Wasserstoff, X Carbonyl und $R_2$ gegebenenfalls durch Hydroxy oder Niederalkoxy substituiertes Niederalkyl oder gegebenenfalls durch Hydroxy, Niederalkoxy, Niederalkyl oder Halogen substituiertes Phenyl bedeuten.

13. Antigenderivate gemäss einem der Ansprüche 1 bis 11, die den Rest eines Muramylpeptids der Formel II enthalten, worin $R_1$, $R_4$, $R_6$ und $R_7$ Wasserstoff, X Carbonyl, $R_2$ gegebenenfalls durch Hydroxy oder Niederalkoxy substituiertes Niederalkyl oder gegebenenfalls durch Hydroxy, Niederalkoxy, Niederalkyl oder Halogen substituiertes Phenyl und $R_3$ Methyl bedeuten.

14. Antigenderivate nach einem der Ansprüche 1 bis 11, die den Rest eines Muramylpeptids der Formel II enthalten, worin $R_1$, $R_4$, $R_6$, $R_7$ und $R_9$ Wasserstoff, X Carbonyl, $R_2$ gegebenenfalls durch Hydroxy oder Methoxy substituiertes Niederalkyl oder gegebenenfalls durch Hydroxy, Methoxy, Methyl, Aethyl oder Halogen substituiertes Phenyl, $R_3$ Wasserstoff oder Methyl, $R_8$ Niederalkyl, Niederalkylmercaptoniederalkyl, Hydroxyniederalkyl, Benzyl, p-Hydroxybenzyl oder Phenyl und $R_{10}$, $R_{11}$ und $R_{12}$ Carboxyl, Niederalkoxycarbonyl oder Carbamoyl und $R_{11}$ auch Wasserstoff bedeuten.

15. Antigenderivate nach einem der Ansprüche 1 bis 11, die den Rest eines Muramylpeptids der Formel II enthalten, worin $R_1$, $R_4$ und $R_6$ Wasserstoff, X Carbonyl, $R_2$ gegebenenfalls durch Hydroxy oder Methoxy substituiertes Niederalkyl oder gegebenenfalls durch Hydroxy, Methoxy, Methyl, Aethyl oder Halogen substituiertes Phenyl, $R_3$ und $R_9$ Wasserstoff oder Methyl, $R_8$ Methyl, Aethyl, n-Propyl, i-Propyl, 2-Methylpropyl, Methylmercaptomethyl, Hydroxymethyl, Hydroxyäthyl, Phenyl, Benzyl oder p-Hydroxybenzyl und $R_{10}$, $R_{11}$ und $R_{12}$ Carboxy, Niederalkoxycarbonyl oder Carbamoyl und $R_{11}$ auch Wasserstoff bedeuten.

16. Antigenderivate nach einem der Ansprüche 1 bis 11, die den Rest eines Muramylpeptids der Formel II enthalten, worin $R_1$, $R_4$ und $R_6$ Wasserstoff, X Carbonyl und $R_7$ und $R_8$ zusammen Propylen oder Butylen bedeuten.

17. Verbindungen nach einem der Ansprüche 1 bis 11, die den Rest eines Muramylpeptids der Formel II enthalten, worin $R_1$, $R_4$ und $R_6$ Wasserstoff, X Carbonyl, $R_7$ und $R_8$ zusammen Propylen oder Butylen, $R_2$ gegebenenfalls durch Hydroxy oder Methoxy substituiertes Niederalkyl oder gegebenenfalls durch Hydroxy, Methoxy, Methyl, Aethyl oder Halogen substituiertes Phenyl, $R_3$ und $R_9$ Wasserstoff oder Methyl, $R_{10}$, $R_{11}$ und $R_{12}$ Carboxy, Niederalkoxycarbonyl oder Carbamoyl und $R_{11}$ auch Wasserstoff bedeuten.

18. Antigenderivate nach einem der Ansprüche 1—17, worin die Muramylpeptide der Formel II über den Rest des Substituenten $R_{12}$ an das Antigen gebunden sind.

19. Antigenderivate gemäss einem der Ansprüche 1 und 3 bis 17, worin das Antigen an einem hockmolekularen Träger fixiert ist.

20. Antigenderivate gemäss Anspruch 19, worin der Träger ein Polymeres der Milchsäure oder von deren Derivaten, die am Kettenende eine freie Carboxylgruppe tragen, oder Alginsäure, Polygalakturonsäure, Pektinsäure, Carboxymethylzellulose, Agarose oder eine basische, neutrale oder saure Polyaminosäure ist.

21. Antigenkonjugate gemäss einem der Ansprüche 1, 2 und 18, worin 2-Acetamido-3-O-{[*L*-1-(*D*-1-carbamoyl-3-carboxy-propyl)-carbamoyl-äthyl]-carbamoyl-methyl}-2-deoxy-*D*-glucose an Serumalbumin, Erythrocytenmembranen, Polysaccharid von Neisseria meningitidis, Merozoiten von Malariaerregern oder T-Lymphoblasten gekuppelt ist.

22. Antigenkonjugate gemäss einem der Ansprüche 1, 2 und 18, worin 2-Acetamino-3-O-{*D*-1-[*L*-(*D*-1-carbamoyl-3-(*L*-1-carboxy-äthyl)-carbamoyl-propyl)-carbamoyläthyl]-carbamoyläthyl}-2-desoxy-D-glucose an Erythrocytenmembranen, Merozoiten von Malariaerregern, Mammarcinomzellen, Maul- und Klauenseuche-Vakzine, T-Lymphoblasten, Tollwut-Vakzine oder Influenza-Virus-Antigene gekuppelt ist.

23. Antigenkonjugate gemäss einem der Ansprüche 1, 2, 4 und 18, worin 2-Acetamino-3-O-{[*L*-1-(*D*-1-carbamoyl-3-carboxy-propyl)-carbamoyl-äthyl]-N-methyl-carbamoyl-methyl}-2-desoxy-D-glucose an Tetanustoxoid, Choleratoxoid oder ein Peptid, das mit der C-terminalen Sequenz des humanen Choriongonadotropins identisch ist, gekuppelt ist.

24. Verfahren zur Herstellung neuer Antigenderivate gemäss einem der Ansprüche 1 bis 23, dadurch gekennzeichnet, dass man ein gegebenenfalls mit Brückengliedern verknüpftes Antigen mit gegebenenfalls mit Brückengliedern verknüpften Muramylpeptiden, wobei einer der beiden Teile mindestens eine freie Amino-, Hydroxy- oder Mercaptogruppe und der andere mindestens eine Carbonsäuregruppe aufweist, miteinander kondensiert, und wenn erwünscht, ie erhaltene Verbindung an einen gegebenenfalls mit Brükkengliedern verbundenen Träger ankondensiert.

25. Pharmazeutische Präparate, die ein Antigenderivat gemäss einem der Ansprüche 1 bis 23 zusammen mit einem pharmazeutisch anwendbaren Trägermaterial enthalten.

26. Pharmazeutische Präparate, gekennzeichnet, durch einen Gehalt an einer Lösung oder Suspension eines der in einem der Ansprüche 1 bis 23 genannten Antigenderivate zusammen mit Carboxymethylzellulose.

27. Verwendung eines der in den Ansprüchen 1 bis 23 genannten Antigenderivate zur Herstellung eines Arzneimittels bzw. Impfstoffes auf nicht-chemischem Wege.

28. Antigenderivat gemäss einem der Ansprüche 1 bis 23 zur Verwendung als Impfstoff.

## Claims

1. Compounds of formula III which have activity as antigens

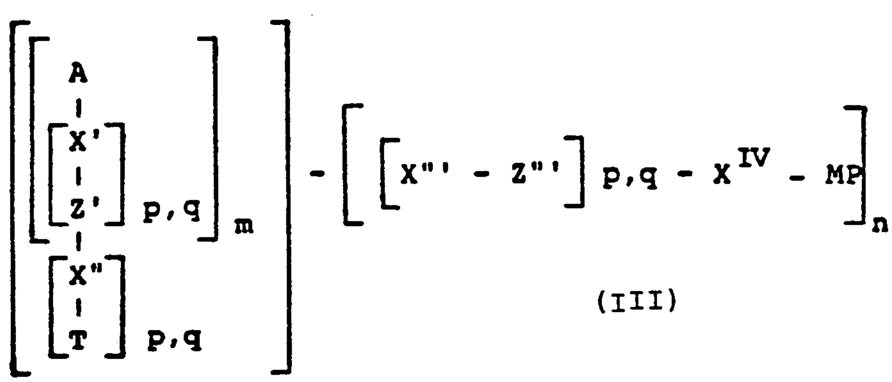

(III)

wherein A is the radical of an antigen, T is the radical of a carrier, MP is the radical of a muramyl peptide of formula II

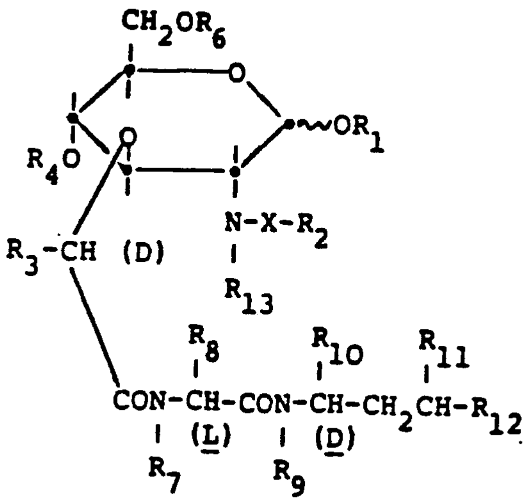

(II)

wherein X is a carbonyl, carbonyloxy or sulfonyl group, $R_1$ is hydrogen, alkyl, unsubstituted or substituted benzyl or acyl, $R_2$ is unsubstituted or substituted alkyl or carbocyclic aryl, $R_4$ and $R_6$ are each independently of the other hydrogen, alkyl, unsubstituted or substituted benzyl or acyl, $R_3$ is hydrogen or alkyl, $R_7$ and $R_{13}$ are hydrogen or lower alkyl, $R_8$ is hydrogen, lower alkyl, free, esterified or etherified hydroxy-lower alkyl, free, esterified or etherified mercapto-lower alkyl, free or acylated amino-lower alkyl, cycloalkyl containing 5 or 6 carbon atoms, cycloalkyl-lower akyl, the cycloalkyl radical of which contains 5 or 6 carbon atoms, unsubstituted or substituted aryl or aralkyl, nitrogen-containing heterocyclyl or heterocyclyl-lower alkyl, $R_7$ and $R_8$ together are also alkylene containing 3 or 4 carbon atoms, $R_9$ is hydrogen or lower alkyl, each of $R_{10}$, $R_{11}$ and $R_{12}$ independently is a free, esterified or amidated carboxyl group, and $R_{11}$ is also hydrogen, or $R_1$, $R_4$ and $R_6$ are tri-lower alkylsilyl, preferably trimethylsilyl, the prefix "lower", qualifying an alkyl radical always denoting that said radical contains up to and including 7 carbon atoms; Z' and Z''' are bivalent bridge members, and X', X'' and X''' and $X^{IV}$ are each independently the covalent linking elements carbonyloxy

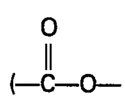 or 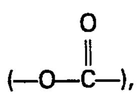, carbonylimino 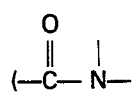 or 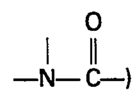

or carbonylthio 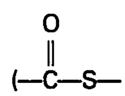 or 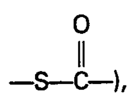,

p = O, q = I and m and n are integers greater than 0, with the proviso that in the compounds of formula III there is present at least one linkage which is customary in peptide chemistry and which has had to be produced synthetically, and that the components used as starting materials, muramyl peptide, carrier and antigen, contain functional groups suitable for a linkage with one another or with the bridge member.

2. Antigen derivatives according to claim 1 which do not contain a carrier.

3. Antigen derivatives according to claim 1 or claim 2, which contain as bridge member (or members independent of one another) the bivalent radical of an α,ω-diamino-lower-alkane bound by way of the nitrogen atoms, lower alkanecarbonyl or bivalently bonded α, β- or γ-aminoalkanecarbonyl.

4. Antigen derivatives according to either claim 1 or claim 2, wherein the antigen is linked direct (i.e. not via a bridge member) with the muramyl peptide.

5. Antigen derivatives according to any one of claims 1 to 4, wherein the antigen is the active constituent of a vaccine against parasites, bacteria, viruses or tumour cells, or attenuated or killed forms or part-components of parasites, bacteria, viruses or tumour cells; or of a vaccine against structures innate in the body, or immunological response structures; or a vaccine suitable for specific desensitisation in the case of allergies.

6. Antigen derivatives according to any one of claims 1 to 5, wherein the antigen is the active constituent of a vaccine against malaria, cholera, typhus, meningitis, rheumatic fever as a consequence of streptococcal infections, influenza, rabies or foot and mouth disease; or of a vaccine for the induction of immunity against components of the reproductive system.

7. Antigen derivatives according to any one of claims 1 to 4, wherein the antigens are malaria merozoites, type-specific meningococcal polysaccharides A, B or C, M-proteins of streptococci, influenza virus haemagglutinins, autologous tumour cells or autologous tumour cell membrane constituents, partial

sequences of human chlorinogonadotropin, grass pollen extracts, anti-specific T-cell lymphoblasts an their receptors for antigens, or antigen-specific immunoglobulins.

8. Antigen derivatives according to any one of claims 1 to 4, wherein the antigens are: attenuated living, killed, modified or degraded chlamydiae, rickettsiae, protozoa or metazoal parasites, constituents of spermatozoa, homologous or hereologous tumour cells or tumour-cell fragments or membrane components, including onkornavirus-codified glycoproteins, all cross-reacting with autologous tumour cells.

9. Antigen derivatives according to any one of claims 1 to 5, wherein the antigen is the active constitiuent of vaccines for the treatment of influenza A and B, parainfluenza 1 to 3, respiratory diseases brought about by respiratory syncital virus, rhinoviruses or adeno-viruses, cytomegaly, German measles, mumps, pertussis, polyiomyelitis, herpes simplex 1 and 2, varicell and herpes zoster, rotavirus diseases, hepatitis A and B, trachoma, caries, diseases caused by pneumococcus, H influenza, pseudomonas and proteus paratyphus, genorrhoea, syphilis, trypanosomiasis (sleeping sickness and Chagas' disease), leishmaniasis, filariasis, schistosomiasis, ankylostomiasis, or allergis asthma brought about by dust or medicaments, allergic rhinitis, and hypersensitivity to drugs.

10. Novel antigen derivatives according to any one of claims 1 to 4, which contain a soluble protein antigen as antigen.

11. Antigen derivatives according to any one of claims 1 to 10, which contain the radical of a muramyl peptide of formula (II) according to claim 19, wherein $R_1$, $R_4$ and $R_6$ are each independently of one another hydrogen, alkyl, or unsubstituted or substituted benzyl or acyl, and $R_{13}$ is hydrogen.

12. Antigen derivatives according to any one of claims 1 to 11, which contain the radical of a muramyl peptide of the formula II according to claim 10, wherein $R_1$, $R_3$, $R_4$, $R_6$ and $R_7$ are hydrogen, X is carbonyl, and $R_2$ is lower alkyl unsubstituted or substituted by hydroxyl or lower alkoxy, or phenyl unsubstituted or substituted by hydroxyl, lower alkoxy, lower alkyl or halogen.

13. Antigen derivatives according to any one of claims 1 to 11, which contain the radical of a muramyl peptide of the formula II, wherein $R_1$, $R_4$, $R_6$ and $R_7$ are hydrogen, X is carbonyl, $R_2$ is lower alkyl unsubstituted or substituted by hydroxyl or lower alkoxy, or phenyl unsubstituted or substituted by hydroxyl, lower alkoxy, lower alkyl or halogen, and $R_3$ is methyl.

14. Antigen derivatives according to any one of claims 1 to 11, which contain the radical of a muramyl peptide of the formula II, wherein $R_1$, $R_4$, $R_6$, $R_7$ and $R_9$ are hydrogen, X is carbonyl, $R_2$ is lower alkyl unsubstituted or substituted by hydroxyl or methoxy, or phenyl unsubstituted or substituted by hydroxyl, methyl, ethyl or halogen, $R_3$ is hydrogen or methyl, $R_8$ is lower alkyl, lower alkylmercapto-lower-alkyl, hydroxy-lower-alkyl, benzyl, p-hydroxybenzyl or phenyl, and $R_{10}$, $R_{11}$ and $R_{12}$ are carboxyl, lower alkoxycarbonyl or carbamoyl, and $R_{11}$ is also hydrogen.

15. Antigen derivatives according to any one of claims 1 to 11, which contain the radical of a muramyl peptide of formula II, wherein $R_1$, $R_4$ and $R_6$ are hydrogen, X is carbonyl, $R_2$ is lower alkyl unsubstituted or substituted by hydroxyl or methoxy, or phenyl unsubstituted or substituted by hydroxyl, methoxy, methyl, ethyl or halogen, $R_3$ and $R_9$ are hydrogen or methyl, $R_8$ is methyl, ethyl, n-propyl, i-propyl, 2-methylpropyl, methylmercaptomethyl, hydroxymethyl, hydroxyethyl, phenyl, benzyl or p-hydroxybenzyl, and $R_{10}$, $R_{11}$ and $R_{12}$ are carbonyl, lower alkoxycarbonyl or carbamoyl, and $R_{11}$ is also hydrogen.

16. Antigen derivatives according to any one of claims 1 to 11, which contain the radical of a muramyl peptide of the formula II, wherein $R_1$, $R_4$ and $R_6$ are hydrogen, X is carbonyl, and $R_7$ and $R_8$ together are propylene or butylene.

17. Compounds according to any one of claims 1 to 11, which contain the radical of a muramyl peptide of formula II, wherein $R_1$, $R_4$ and $R_6$ are hydrogen, X is carbonyl, $R_7$ and $R_8$ together are propylene or butylene, $R_2$ is lower alkyl unsubstituted or substituted by hydroxyl, methoxy, methyl, ethyl or halogen, $R_3$ and $R_9$ are hydrogen or methyl, $R_{10}$, $R_{11}$ and $R_{12}$ are carboxyl, lower alkoxycarbonyl or carbamoyl, and $R_{11}$ is also hydrogen.

18. Antigen derivatives according to any one of claims 1—17, wherein the muramyl peptides of formula II are bonded via the radical of the substituent $R_{12}$ to the antigen.

19. Antigen derivatives according to any one of claims 1 and 3 to 17, wherein the antigen is fixed to a high-molecular carrier.

20. Antigen derivatives according to claim 19, wherein the carrier is a polymer of lactic acid and of derivatives thereof, which carry at the end of the chain a free carboxyl group, or alginic acid, polygalacturonic acid, pectic acid, carboxymethylcellulose, agarose or a basic, neutral or acidic polyamino acid.

21. Antigen conjugates according to any one of claims 1, 2 and 18, wherein 2-acetamido-3-O-{*L*-1-(*D*-1-carbamoyl-32-carboxypropyl)carbamoylethyl]carbamoylmethyl}-2-deoxy-*D*-glucose is coupled to serum albumin, eryhthrocyte membranes, polysaccharide of Neisseria meningitidis, merozoites of maleria plasmodium or T-lymphoblasts.

22. Antigen conjugates according to any one of claims 1, 12 and 18, wherein 2-acetamino-3-O-{*D*-1-(*L*-1-(*D*-1-carbamoyl-3-(*L*-1-carboxyethyl]carbamoylpropyl)-carbamoylethyl]carbamoylethyl}-2-desoxy-D-glucose is coupled to erythrocyte membranes, merozoites of malaria plasmodium, mastocarcinoma cells, foot and mouth disease vaccines, T-lymphoblasts, rabies vaccines or influenza-virus antigens.

23. Antigens conjugates according to any one of claims 1, 2, 4 and 18, wherein 2-acetamino-3-D-{*[L*-1-

(*D*-1-carbamoyl-3-carboxypropyl)carbamoylethyl]-N-methylcarbamoylmethyl}-2-desoxy-D-glucose is coupled to tetanus toxoid, cholera toxoid or a peptide which is identical to the C-terminal sequence of the human chorionic gonadotrophin.

24. A process for the production of antigen derivatives according to any one of claims 1 to 23, wherein an antigen, optionally linked with bridge members, is subjected to a condensation reaction with muramyl peptides optionally linked with bridge members, one of the two components containing at leat one free amino-hydroxyl or mercapto group, and the other at least one carboxylic acid group; and, if desired, fusing the compound obtained to a carrier optionally linked with bridge members.

25. A pharmaceutical composition containing an antigen derivative according to any one of claims 1 to 23, together with a pharmaceutically acceptable carrier.

26. A pharmaceutical composition containing a solution or suspension of an antigen derivative according to any one of claims 1 to 23, together with carboxymethylcellulose.

27. The use of an antigen derivative according to any one of claims 1 to 23 for the production of a medicament or vaccine by a non-chemical method.

28. An antigen derivative according to any one of claims 1 to 23 for use as a vaccine.

**Revendications**

1. Composés de formule III actifs comme antigènes

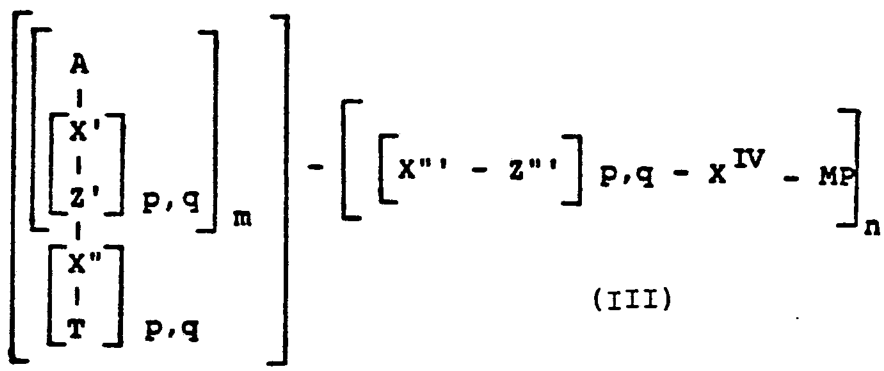

(III)

où A représente le radical d'un antigène, T le radical d'un support, MP le radical d'un muramyl-peptide de formule II

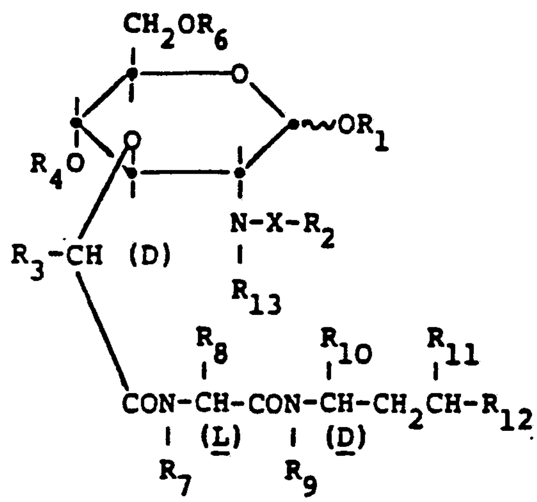

(II)

où X représente un groupe carbonyle, carbonyloxy ou sulfonyle, $R_1$ représente un hydrogène, un alcoyle, un benzyle ou un acyle éventuellement substitué, $R_2$ représente un alcoyle éventuellement substitué ou un aryle carbocyclique, $R_4$ et $R_6$ représentent indépendamment l'un de l'autre un hydrogène, un alcoyle, un benzyle ou un acyle éventuellement substitué, $R_3$ représente un hydrogène ou un alcoyle, $R_7$ et $R_{13}$ représentent un hydrogène ou un alcoyle inférieur, $R_8$ représente un hydrogène, un alcoyle inférieur, un hydroxyalcoyle inférieur libre, estérifié ou éthérifié, un mercapto-alcoyle inférieur libre, estérifié ou éthérifié, un aminoalcoyle inférieur libre ou acylé un cycloalcoyle à 5 ou 6 atomes de carbone, un cycloalcoyl-alcoyle inférieur dont le radical cycloalcoyle contient 5 ou 6 atomes de carbone, une alcoyle ou aralcoyle éventuellement substitué, un hétérocyclyle ou hétérocyclyl-alcoyle inférieur contenant de l'azote, $R_7$ et $R_8$ ensemble représentent également un alcoylène avec 3 ou 4 atomes de carbone, $R_9$ représente un

hydrogène ou un alcoyle inférieur, les radicaux $R_{10}$, $R_{11}$ et $R_{12}$ représentent indépendamment l'un de l'autre un radical carboxy éventuellement estérifié ou amidé et $R_{11}$ représente également un hydrogène, ou $R_1$, $R_4$ et $R_6$ représentent un trialcoyle inférieur-silyle, en particulier un triméthylsilyle, où le préfix "inférieur" caractérise toujours un radical ayant jusqu'à 7 atomes de carbone compris, Z' et Z''' représentent des éléments de pont bivalents, X', X'', X''' et $X^{IV}$ représentent indépendamment l'un de l'autre les éléments de liaison covalente carbonyloxy

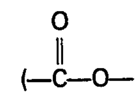 ou 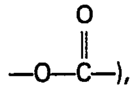, carbonylimino 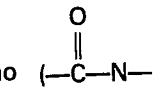 ou 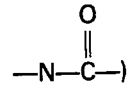

ou carbonylthio 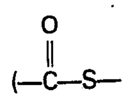 ou 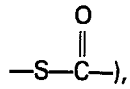,

p = O, q = I et m et n représentent des nombres entiers supérieurs à 0, avec cette précision que dans les composés de formule III se trouve au moins une liaison habituelle en chimie des peptides, qui doit être préparée par synthése, et également caractérisés en ce que les composants muramylpeptide, support et antigène utilisés comme produits de départ présentent les groupes fonctionnels appropriées à la liaison entre eux ou à l'élément de pont.

2. Dérivés d'antigène selon la revendication 1 ne contenant pas de support.

3. Dérivés d'antigène selon l'une des revendications 1 ou 2, contenant comme élément(s) de pont (indépendamment l'un de l'autre) le radical bivalent d'un α, ω-diamino-alcane inférieur lié par l'intermédiaire de l'atome d'azote, un alcane inférieur-dicarbonyle ou un α-, β- ou γ-amino-alcanecarbonyle lié de façon bivalente.

4. Dérivés d'antigène selon l'une des revendications 1 ou 2, où l'antigène est lié directement (c'est-à-dire pas par l'intermédiaire d'un élément de pont) avec le muramyl-peptide.

5. Dérivés d'antigène selon l'une des revendications 1 à 4, caractérisé en ce que l'antigène représente le contenu efficace d'un vaccin contre les parasites, les bactéries, les virus ou les cellules tumorlaes, des formes affaiblies ou tuées ou des composants partiels de parasites, de bactéries, de virus ou de cellules tumorales, d'un vaccin contre les structures propres du corps, les structures de reconnaissance immunologique ou d'un vaccin qui convient pour une désensibilisatian spécifique dans les allergies.

6. Dérivés d'antigène selon l'une des revendications 1 à 5, qui contiennent comme antigène le contenu efficace d'un vaccin contre la malaria, le choléra, le typhus, la méningite, la fièvre rhumatismale à la suite d'infections par des streptocoques, la grippe, la rage, la fièvre aphteuse, ainsi que d'un vaccin pour induire une immunité contre les composants du système de transplantation.

7. Dérivés d'antigène selon l'une des revendications 1 à 4, qui contiennent comme antigène des mérozoïtes de la malaria, des polysaccharides méningococciques spécifiques des types A, B ou C, des protéines M de streptocoque, des hémagglutinines du virus de la grippe, des cellules turmorales autologues ou des composants de membranes de cellules tumorales autologues, des séquences partielles de gonadotrophine chlorionique humain, des extraits de pollen de graminées, de lymphoblastes de cellules T antigénospécifique et leurs récepteurs pour les antigènes ou les immunoglobulines antigénospécifiques.

8. Dérivés d'antigène selon l'une des revendications 1 à 4, qui contiennent comme antigènes, vivants et affaiblis, tués, modifiés ou dégradés, des chlamydies, des rickettsies, des protozoaires ou des parasites métazoïques, des composants de spermatozoïdes, des cellules tumorales homologues ou hétérologues réagissant de façon croisée avec les cellules turmorales autologues ou leurs fragments ou composants de membranes de cellules tumorales y compris les glycoprotéines codées d'oncornavirus.

9. Dérivés d'antigène selon l'une des revendications 1 à 5, qui contiennent comme antigène le contenu efficace des vaccins pour le traitement de la grippe A et B, de la parainfluenza 1 à 3, des maladies respiratoires provoquées par le virus syncital respiratoire, les rhinovirus ou les adénovirus, de la cytomégalie, de la rubéole, de la rougeole, des oreillons, de la coqueluche, de la poliomyélite, e l'herpes simplex 1 et 2, des varicelles et du zona, des maladies à rotavirus, de lhépatite A et B, du trachome, de la carie, des maladies causées par des pneumocoques, H. influenza, Pseudomonas et Proteus, du paratyphus, de la genorrhée, de la syphilis, des trypanosomiases (maladies du sommeil et maladie de Chagas), les leishmanioses, des filarioses, des schistosomiases, des ankylostomiases ou de l'asthme allergique provoqué par la poussière ou certains médicaments, de la rhinite allergique et de l'hypersensibilité médicamenteuse.

10. Dérivés d'antigène selon l'une des revendications 1—4, qui contiennent comme antigène un antigène de protéine soluble.

11. Dérivés d'antigène selon l'une des revendications 1 à 10, contenant le radical d'un muramyl-peptide de formule II selon la revendication 10, où $R_1$, $R_4$ et $R_6$ représentent indépendamment l'un de l'autre un hydrogène, un alcoyle ou un benzyle ou une acyle éventuellement substitué et $R_{13}$ représente un hydrogène.

12. Dérivés d'antigène selon l'une des revendications 1 à 11, contenant le radical d'un muramyl-

peptide de formule II selon la revendication 10, où $R_1$, $R_3$, $R_4$, $R_6$ et $R_7$ représentent un hydrogène, X un carbonyle et $R_2$ représente un alcoyle inférieur éventuellement substitué par un hydroxy ou un alcoxy inférieur ou un phényle éventuellement substitué par un hydroxy, un alcoxy inférieur, un alcoyle inférieur ou un halogène.

13. Dérivés d'antigène selon l'une des revendications 1 à 11, contenant le radical d'un muramyl-peptide de formule II selon la revendication 10, où $R_1$, $R_4$, $R_6$ et $R_7$ représentent un hydrogène, X un carbonyle $R_2$ représente un alcoyle inférieur éventuellement substitué par un hydroxy ou un alcoxy inférieur ou un phényle éventuellement substitué par un hydroxy, un alcoxy inférieur, un alcoyle inférieur ou un halogène et $R_3$ représente unméthyle.

14. Dérivés d'antigène selon l'une des revendications 1 à 11, contenant le radical d'un muramyl-peptide de formule II selon la revendication 10, où $R_1$, $R_4$, $R_6$, $R_7$ et $R_9$ représentent un hydrogène, X un carbonyle, $R_2$ un alcoyle inférieur éventuellement substitué par un hydroxy ou un éthoxy ou un phényle éventuellement substitué per hydroxy, un méthoxy, un méthyle, un éthyle ou un halogène, $R_3$ represénte un hydrogène ou un méthyle, $R_8$ représente un alcoyle inférieur, un alcoyle inférieur-mercapto-alcoyle inférieur, un hydroxyalcoyle inférieur, un benzyle, un p-hydroxybenzyle ou un phényle et $R_{10}$, $R_{11}$ et $R_{12}$ représentent un carboxyle, un alcoxy inférieur-carbonyle ou un carbamoyle et $R_{11}$ représente également un hydrogène.

15. Dérivés d'antigène selon l'une des revendications 1 à 11, contenant le radical d'un muramyl-peptide de formule II selon la revendication 10, où $R_1$, $R_4$ et $R_6$ représentent un hydrogène, X représente un carbonyle, $R_2$ reprsente un alcoyle inférieur éventuellment substitué par un hydroxy ou un méthoxy ou un phényle éventuellement substitué par un hydroxy, un méthoxy, un méthyle, un éthyle ou un halogène, $R_3$ et $R_9$ représentent un hydrogène ou un méthyle, $R_8$ représente un méthyle, un éthyle, un n-propyle, uni-propyle, un 2-méthylpropyle, un méthylmercaptométhyle, un hydroxyméthyle, un hydroxyéthyle, un phényle, un benzyle ou un p-hydroxybenzyle et $R_{10}$, $R_{11}$ et $R_{12}$ représentent un carboxy, un alcoxy inférieur-carbonyle ou un carbamoyle et $R_{11}$ représente également un hydrogène.

16. Dérivés d'antigène selon l'une des revendications 1 à 11, contenant le radical d'un muramyl-peptide de formule II selon la revendication 10, où $R_1$, $R_4$ et $R_6$ représentent un hydrogène, X représente un carbonyle et $R_7$ et $R_8$ représentent ensemble un propylène ou un butylène.

17. Composés selon l'une des revendications 1 à 11 contenant le radical d'un muramyl-peptide de formule II selon la revendication 10, où $R_1$, $R_4$ et $R_6$ représentent un hydrogène, X représente un carbonyle, $R_7$ et $R_8$ représentent ensemble un propylène ou un butylène, $R_2$ représente un alcoyle inférieur éventuellement substitué par un hydroxy ou un méthoxy ou un phényle éventuellement substitué par un hydroxy, un méthoxy, un méthyle, un éthyle ou un halogène, $R_3$ et $R_9$ représentent un hydrogène ou un méthyle, $R_{10}$, $R_{11}$ et $R_{12}$ représentent un carboxy, un alcoxy inférieur-carbonyle ou un carbamoyle et $R_{11}$ représente également un hydrogène.

18. Dérivés d'antigène selon l'une des revendications 1—17, où les muramyl-peptides de formule II selon la revendication 10 sont liés par l'intermédiaire du radical du substituant $R_{12}$ à l'antigène.

19. Dérivés d'antigène selon l'une des revendications 1 et 3 à 17, où l'antigène est fixé à un support à haut poids moléculaire.

20. Dérivés d'antigène selon la revendication 18, où le support est un polymère de l'acide lactique et de ses dérivés, portant à l'extrémité de la chaîne un groupe carboxyle libre, ou l'acide alginique, l'acide polygalacturonique, l'acide pectinique, la carboxyméthylcellulose, l'agarose ou un acide polyaminé basique, neutre ou acide.

21. Conjuguats d'antigène selon l'une des revendications 1, 2 et 18, où le 2-acétamido-3-O-{[*L*-1-(*D*-1-carbamoyl-3-carboxypropyl)-carbamoyl-éthyl]-carbamoyl-méthyl}-2-désoxy-*D*-glucose est couplé à la sérumalbumine, à des membranes d'érythrocytes, au polysaccharide de Neisseria meningitidis, à des mérozoïtes de parasites de la malaria ou à des lymphoblastes T.

22. Conjuguats d'antigène selon l'une des revendications 1, 12 et 18, où le 2-acétamino-3-O-{*D*-1-[*L*-1-(*D*-1-carbamoyl-3-(*L*-1-carboxy-éthyl)-carbamoyl-propyl)-carbamoyléthyl]-carbamoyl-éthyl}-2-désoxy-D-glucose est couplé à des membranes d'érythrocytes, à des mérozoïtes des parasites de la malaria, à des cellules de carcinome mammaire, à des vaccins contre la fièvre aphteuse, à des lymphoblastes T, à des vaccins antirabiques ou à des antigènes de virus antigrippaux.

23. Conjugats d'antigène selon l'une des revendications 1, 2, 4 et 18, où le 2-acétamino-3-D-{[*L*-1-(*D*-carbamoyl-3-carboxypropyl)-carbamoyl-éthyl]-N-méthyl-carbamoyl-méthyl}-2-désoxy-D-glucose est couplé au toxoïde tétanique, au toxoïde cholérique ou à un peptide qui est identique à la séquence C-terminale de la gonadotrophine chlorionique humaine.

24. Procédé de préparation de nouveaux dérivés d'antigène selon l'une des revendications 1 à 23, caractérisé en ce qu'on condense un antigène éventuellement relié à des éléments de pont avec des muramylpeptides éventuellement reliés à des éléments de pont, où l'une des deux fractions présente au moins un groupe amino, hydroxy ou mércapto libre et l'autre présente au moins un groupe acide carboxylique, et, si on le désire, on condense le composé obtenu sur un support évenuellement lié à des éléments de pont.

25. Préparations pharmaceutiques caractérisées en ce qu'elles contiennent un des dérivés d'antigène mentionnés dans les revendications 1 à 23.

26. Préparations pharmaceutiques caractérisées en ce qu'elles contiennent une solution ou suspension

des dérivés d'antigène mentionnés dans les revendications 1 à 23 avec de la carboxyméthylcellulose.

27. Application d'un des dérivés d'antigène mentionnés dans les revendications 1 à 23 à la préparation d'un médicament ou d'un vaccin par un moyen non chimique.

28. Dérivé d'antigène selon l'une des revendications 1 à 23 aux fins d'application comme vaccin.